(19) Europäisches Patentamt
European Patent Office
Office européen des brevets

(11) EP 2 180 013 A1

(12) EUROPEAN PATENT APPLICATION
published in accordance with Art. 153(4) EPC

(43) Date of publication:
**28.04.2010 Bulletin 2010/17**

(21) Application number: **08791649.0**

(22) Date of filing: **25.07.2008**

(51) Int Cl.:
***C08G 61/12*** (2006.01) ***C07C 213/00*** (2006.01)
***C07C 215/68*** (2006.01) ***C07C 227/14*** (2006.01)
***C07C 229/58*** (2006.01) ***C09D 11/00*** (2006.01)
***C09K 11/06*** (2006.01) ***H01L 51/50*** (2006.01)

(86) International application number:
**PCT/JP2008/063405**

(87) International publication number:
**WO 2009/017056 (05.02.2009 Gazette 2009/06)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **31.07.2007 JP 2007198637**

(71) Applicants:
- **Sumitomo Chemical Company, Limited**
**Tokyo 104-8260 (JP)**
- **Sumation Co., Ltd.**
**Chuo-ku**
**Tokyo 104-8260 (JP)**

(72) Inventor: **KOBAYASHI, Satoshi**
**Tsukuba-shi**
**Ibaraki 305-0005 (JP)**

(74) Representative: **Duckworth, Timothy John et al**
**J.A. Kemp & Co.**
**14 South Square**
**Gray's Inn**
**London WC1R 5JJ (GB)**

(54) **COMPOUND, METHOD FOR PRODUCING THE SAME, INK COMPOSITION USING THE COMPOUND, THIN FILM, ORGANIC TRANSISTOR, AND ORGANIC ELECTROLUMINESCENT DEVICE**

(57) A compound comprises a structure represented by the following general formula (1) (including a structure of a residue obtained by removing at least one hydrogen atom from the structure represented by the following general formula (1))

[Chemical formula 1]

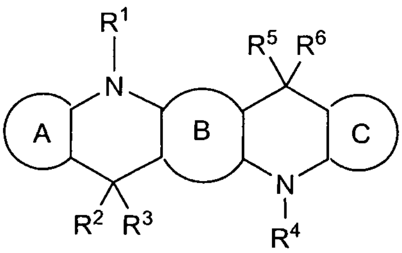

(1)

(in the formula (1), a ring A, a ring B and a ring C each independently represent any one of a monocyclic aromatic ring and fused aromatic ring; $R^1$ and $R^4$ each independently represent a monovalent group; and $R^2$, $R^3$, $R^5$ and $R^6$ each independently represent any one of a hydrogen atom, alkyl group, aryl group, arylalkyl group, alkenyl group, and alkynyl group).

EP 2 180 013 A1

## Description

## Technical Field

The present invention relates to: methods for producing novel compounds; compounds used as synthetic raw materials for these novel compounds; an ink composition comprising these novel compounds; a thin film comprising these novel compounds; an organic transistor comprising these novel compounds; an organic electroluminescence device comprising these novel compounds; and a surface light source and a display material using such a device.

## Background of the Invention

Various light-emitting materials and charge transporting materials useful for manufacturing light-emitting devices have been studied. For example, "Polymer preprints", published in 2001, vol. 42 (no. 2), p. 587 (Document 1) discloses an amine compound such as a compound having a structure of N,N'-diphenyl-N,N'-di(p-butylphenyl)-1,4-diaminobenzen e as the repeating unit.

## Disclosure of the Invention

However, the aforementioned compound has a problem that the chromaticity is not sufficent when used as a blue-light-emitting material for an organic electroluminescence device. Additionally, such a conventional amine compound has a problem that, when the compound is used in an organic electroluminescence device, the device requires a high driving voltage.

The present invention has been made in consideration of the above-described problems of the conventional technique. An object of the present invention is to provide a compound excellent in chromaticity when used as a blue-light-emitting material for an organic electroluminescence device, and to lower the driving voltage of an organic electroluminescence device, when the compound is used in the device.

The present inventors have earnestly studied in order to achieve the above object. As a result, the inventors have found out that the object is achieved, by selecting, in a compound comprising a structure of N,N'-diphenyl-N,N'-di (p-butylphenyl)-1,4-diaminobenzen e which is bridged with carbon atoms, substituents on the carbon bridge appropriately. Thus, the present inventors have completed the present invention.

Specifically, a compound of the present invention is a compound comprising a structure represented by the following general formula (1) (including a structure of a residue obtained by removing at least one hydrogen atom from the structure represented by the general formula (1)):

[Chemical formula 1]

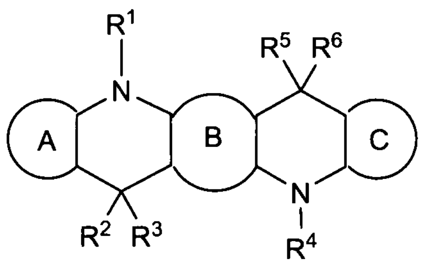

(1)

(in the formula (1), a ring A, a ring B and a ring C each independently represent any one of a monocyclic aromatic ring and fused aromatic ring; $R^1$ and $R^4$ each independently represent a monovalent group; and $R^2$, $R^3$, $R^5$ and $R^6$ each independently represent any one of a hydrogen atom, alkyl group, aryl group, arylalkyl group, alkenyl group, and alkynyl group).

Moreover, a method for producing the compound of the present invention is a method comprising a step of polymerizing a compound represented by the following general formula (7) as a raw material:

[Chemical formula 2]

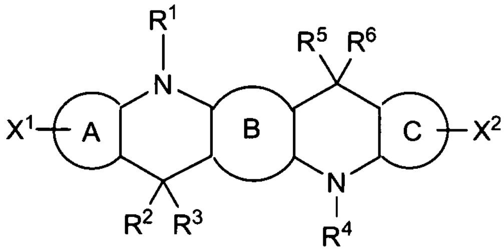

(7)

(in the formula (7), a ring A, a ring B and a ring C each independently represent any one of a monocyclic aromatic ring and fused aromatic ring; $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; and $X^1$ and $X^2$ each independently represent a substituent capable of participating in polymerization).

Furthermore, a synthetic raw material for the compound of the present invention is a synthetic raw material for obtaining the compound comprising the structure represented by the general formula (1).

Moreover, the composition of the present invention is a composition comprising the compound, and at least one material selected from the group consisting of a hole transporting material, an electron transporting material, and a light-emitting material. Furthermore, an ink composition of the present invention comprises the compound or the composition. In addition, a thin film of the present invention comprises the compound or the composition.

Furthermore, an organic transistor of the present invention comprises the thin film. Additionally, an organic electroluminescence device of the present invention comprises an organic layer being located between electrodes of an anode and a cathode, the organic layer including the compound or the composition.

Moreover, a surface light source of the present invention comprises the organic electroluminescence device. Furthermore, a display device of the present invention comprises the organic electroluminescence device.

According to the present invention, it is possible to provide a compound excellent in chromaticity when used as a blue-light-emitting material for an organic electroluminescence device. Moreover, the organic electroluminescence device using the compound of the present invention is driven by a low voltage. The compound of the present invention is generally useful as light-emitting materials and charge transporting materials. In addition, when the compound is used in an organic electroluminescence device, the luminescence wavelength is short. Moreover, when a light-emitting part for blue color comprising the compound of the present invention is set together with light-emitting parts for green, red, and other colors, the light emitting part can also serve as a light emitting part for green, red, and white colors usefully.

Accordingly, the compound of the present invention is useful in organic transistors and organic electroluminescence devices. Furthermore, the organic electroluminescence device of the present invention is useful in surface light sources and display devices (for example, display devices such as a segment display device and a dot-matrix display device, and backlights of liquid crystal display devices).

## Detailed Description of the Preferred Embodiments

Hereinafter, the present invention will be described in detail with reference to preferred embodiments thereof.

A compound of the present invention comprises a structure represented by the following general formula (1) (including a structure of a residue obtained by removing at least one hydrogen atom from the structure represented by the following general formula (1)).

[Chemical formula 3]

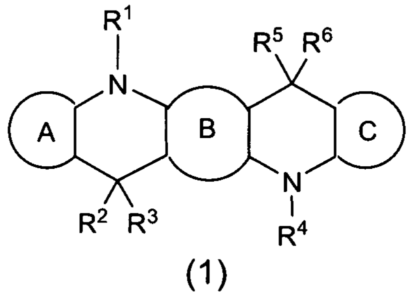

(1)

In the general formula (1), a ring A, a ring B and a ring C each independently represent any one of a monocyclic aromatic ring and fused aromatic ring. Alternatively, the ring A, the ring B and the ring C each may have a substituent.

Examples of such an aromatic ring include: aromatic hydrocarbon rings such as a benzene ring, naphthalene ring, anthracene ring, phenanthrene ring, pyrene ring, perylene ring, tetracene ring, pentacene ring, and fluorene ring; and heteroaromatic rings such as a pyridine ring, pyrimidine ring, pyridazine ring, pyrazine ring, quinoline ring, isoquinoline ring, quinoxaline ring, quinazoline ring, acridine ring, phenanthroline ring, thiophene ring, benzothiophene ring, dibenzothiophene ring, thiophene oxide ring, benzothiophene oxide ring, dibenzothiophene oxide ring, furan ring, benzofuran ring, pyrrole ring, indole ring, dibenzopyrrole ring, silole ring, benzosilole ring, dibenzosilole ring, borole ring, benzoborole ring, and dibenzoborole ring. Among these, from the viewpoints of heat resistance, fluorescence intensity, device properties, and the like, preferable are aromatic hydrocarbon rings; more preferable are a benzene ring, naphthalene ring, anthracene ring and phenanthrene ring; and particularly preferable is a benzene ring.

Meanwhile, examples of the substituent that the ring A, the ring B and the ring C may have include a halogen atom, alkyl group, alkoxy group, alkylthio group, aryl group, aryloxy group, arylthio group, arylalkyl group, arylalkoxy group, arylalkylthio group, alkenyl group, alkynyl group, disubstituted amino group, trisubstituted silyl group, acyl group, acyloxy group, imine residue, amide group, acid imide group, monovalent heterocyclic group, substituted carboxyl group, heteroaryloxy group, and heteroarylthio group.

Here, examples of the halogen atom include a fluorine atom, chlorine atom, bromine atom, and iodine atom.

The alkyl group may be linear, branched or cyclic. The carbon number of such an alkyl group is usually about 1 to 30, and perferably about 3 to 15 from the viewpoint of solubility in a solvent. Examples of such an alkyl group include a methyl group, ethyl group, propyl group, i-propyl group, butyl group, i-butyl group, t-butyl group, pentyl group, isoamyl group, hexyl group, cyclohexyl group, heptyl group, octyl group, 2-ethylhexyl group, nonyl group, decyl group, 3,7-dimethyloctyl group, lauryl group, trifluoromethyl group, pentafluoroethyl group, perfluorobutyl group, perfluorohexyl group, and perfluorooctyl group. Among these, from the viewpoint of balancing heat resistance with solubility in an organic solvent, device properties, easiness of synthesis, and the like, preferable are a pentyl group, isoamyl group, hexyl group, octyl group, 2-ethylhexyl group, decyl group, and 3,7-dimethyloctyl group.

The alkoxy group may be linear, branched or cyclic. The carbon number of such an alkoxy group is usually about 1 to 30, and preferably about 3 to 15 from the viewpoint of solubility in a solvent. Examples of such an alkoxy group include a methoxy group, ethoxy group, propyloxy group, i-propyloxy group, butoxy group, i-butoxy group, t-butoxy group, pentyloxy group, hexyloxy group, cyclohexyloxy group, heptyloxy group, octyloxy group, 2-ethylhexyloxy group, nonyloxy group, decyloxy group, 3,7-dimethyloctyloxy group, lauryloxy group, trifluoromethoxy group, pentafluoroethoxy group, perfluorobutoxy group, perfluorohexyl group, perfluorooctyl group, methoxymethyloxy group, and 2-methoxyethyloxy group. Among these, from the viewpoint of balancing heat resistance with solubility in an organic solvent, device properties, easiness of synthesis, and the like, preferable are a pentyloxy group, hexyloxy group, octyloxy group, 2-ethylhexyloxy group, decyloxy group, and 3,7-dimethyloctyloxy group.

The alkylthio group may be linear, branched or cyclic. The carbon number of such an alkylthio group is usually about 1 to 30, and preferably about 3 to 15 from the viewpoint of solubility in a solvent. Examples of such an alkylthio group include a methylthio group, ethylthio group, propylthio group, i-propylthio group, butylthio group, i-butylthio group, t-butylthio group, pentylthio group, hexylthio group, cyclohexylthio group, heptylthio group, octylthio group, 2-ethylhexylthio group, nonylthio group, decylthio group, 3,7-dimethyloctylthio group, laurylthio group, and trifluoromethylthio group. Among these, from the viewpoint of balancing heat resistance with solubility in an organic solvent, device properties, easiness of synthesis, and the like, preferable are a pentylthio group, hexylthio group, octylthio group, 2-ethylhexylthio group, decylthio group, and 3,7-dimethyloctylthio group.

The aryl group is an atomic group obtained by removing one hydrogen atom from an aromatic hydrocarbon.

The aryl group includes those having a fused ring, and those in which independent two or more benzene rings or fused rings are bonded to each other directly or via a group such as vinylene, as well. The carbon number of such an aryl group is usually about 6 to 60, and preferably about 6 to 30. Examples of such an aryl group include a phenyl group, $C_1$-$C_{12}$ alkoxyphenyl groups ($C_1$-$C_{12}$ indicates that the carbon number of an organic group immediately following $C_1$-$C_{12}$ (here, the carbon number of an alkoxy group in an alkoxyphenyl group) is 1 to 12. Hereinafter, the same), $C_1$-$C_{12}$ alkylphenyl groups, 1-naphthyl group, 2-naphthyl group, 1-anthracenyl group, 2-anthracenyl group, 9-anthracenyl group, and pentafluorophenyl group. Among these, from the viewpoint of solubility in an organic solvent, device properties, easiness of synthesis, and the like, preferable are $C_1$-$C_{12}$ alkoxyphenyl groups and $C_1$-$C_{12}$ alkylphenyl groups. Moreover, examples of the $C_1$-$C_{12}$ alkoxyphenyl groups include a methoxyphenyl group, ethoxyphenyl group, propyloxyphenyl group, i-propyloxyphenyl group, butoxyphenyl group, i-butoxyphenyl group, t-butoxyphenyl group, pentyloxyphenyl group, hexyloxyphenyl group, cyclohexyloxyphenyl group, heptyloxyphenyl group, octyloxyphenyl group, 2-ethylhexyloxyphenyl group, nonyloxyphenyl group, decyloxyphenyl group, 3,7-dimethyloctyloxyphenyl group, and lauryloxyphenyl group. Furthermore, examples of the $C_1$-$C_{12}$ alkylphenyl groups include a methylphenyl group, ethylphenyl group, dimethylphenyl group, propylphenyl group, mesityl group, methylethylphenyl group, i-propylphenyl group, butylphenyl group, i-butylphenyl group, t-butylphenyl group, pentylphenyl group, isoamylphenyl group, hexylphenyl group, heptylphenyl group, octylphenyl group, nonylphenyl group, decylphenyl group, and dodecylphenyl group.

The aryloxy group has a carbon number of usually about 6 to 60, and preferably about 6 to 30. Examples of such an aryloxy group include a phenoxy group, $C_1$-$C_{12}$ alkoxyphenoxy groups, $C_1$-$C_{12}$ alkylphenoxy groups, 1-naphthyloxy group, 2-naphthyloxy group, and pentafluorophenyloxy group. Among these, from the viewpoints of solubility in an organic solvent, device properties, easiness of synthesis, and the like, preferable are $C_1$-$C_{12}$ alkoxyphenoxy groups and $C_1$-$C_{12}$ alkylphenoxy groups. Moreover, examples of the $C_1$-$C_{12}$ alkoxyphenoxy groups include a methoxyphenoxy group, ethoxyphenoxy group, propyloxyphenoxy group, i-propyloxyphenoxy group, butoxyphenoxy group, i-butoxyphenoxy group, t-butoxyphenoxy group, pentyloxyphenoxy group, hexyloxyphenoxy group, cyclohexyloxyphenoxy group, heptyloxyphenoxy group, octyloxyphenoxy group, 2-ethylhexyloxyphenoxy group, nonyloxyphenoxy group, decyloxyphenoxy group, 3,7-dimethyloctyloxyphenoxy group, and lauryloxyphenoxy group. Furthermore, examples of the $C_1$-$C_{12}$ alkylphenoxy groups include a methylphenoxy group, ethylphenoxy group, dimethylphenoxy group, propylphenoxy group, 1,3,5-trimethylphenoxy group, methylethylphenoxy group, i-propylphenoxy group, butylphenoxy group, i-butylphenoxy group, t-butylphenoxy group, pentylphenoxy group, isoamylphenoxy group, hexylphenoxy group, heptylphenoxy group, octylphenoxy group, nonylphenoxy group, decylphenoxy group, and dodecylphenoxy group.

The arylthio group has a carbon number of usually about 6 to 60, and preferably about 6 to 30. Examples of such an arylthio group include a phenylthio group, $C_1$-$C_{12}$ alkoxyphenylthio groups, $C_1$-$C_{12}$ alkylphenylthio groups, 1-naphthylthio group, 2-naphthylthio group, and pentafluorophenylthio group. Among these, from the viewpoints of solubility in an organic solvent, device properties, easiness of synthesis, and the like, preferable are $C_1$-$C_{12}$ alkoxyphenylthio groups and $C_1$-$C_{12}$ alkylphenylthio groups.

The arylalkyl group has a carbon number of usually about 7 to 60, and preferably about 7 to 30. Examples of such an arylalkyl group include phenyl-$C_1$-$C_{12}$ alkyl groups, $C_1$-$C_{12}$ alkoxyphenyl-$C_1$-$C_{12}$ alkyl groups, $C_1$-$C_{12}$ alkylphenyl-$C_1$-$C_{12}$ alkyl groups, 1-naphthyl-$C_1$-$C_{12}$ alkyl groups, and 2-naphthyl-$C_1$-$C_{12}$ alkyl groups. Among these, from the viewpoints of solubility in an organic solvent, device properties, easiness of synthesis, and the like, preferable are $C_1$-$C_{12}$ alkoxyphenyl-$C_1$-$C_{12}$ alkyl groups and $C_1$-$C_{12}$ alkylphenyl-$C_1$-$C_{12}$ alkyl groups.

The arylalkoxy group has a carbon number of usually about 7 to 60, and preferably about 7 to 30. Examples of such an arylalkoxy group include phenyl-$C_1$-$C_{12}$ alkoxy groups such as a phenylmethoxy group, phenylethoxy group, phenylbutoxy group, phenylpentyloxy group, phenylhexyloxy group, phenylheptyloxy group and phenyloctyloxy group, $C_1$-$C_{12}$ alkoxyphenyl-$C_1$-$C_{12}$ alkoxy groups, $C_1$-$C_{12}$ alkylphenyl-$C_1$-$C_{12}$ alkoxy groups, 1-naphthyl-$C_1$-$C_{12}$ alkoxy groups, and 2-naphthyl-$C_1$-$C_{12}$ alkoxy groups. Among these, from the viewpoints of solubility in an organic solvent, device properties, easiness of synthesis, and the like, preferable are $C_1$-$C_{12}$ alkoxyphenyl-$C_1$-$C_{12}$ alkoxy groups and $C_1$-$C_{12}$ alkylphenyl-$C_1$-$C_{12}$ alkoxy groups.

The arylalkylthio group has a carbon number of usually about 7 to 60, and preferably about 7 to 30. Examples of such an arylalkylthio group include phenyl-$C_1$-$C_{12}$ alkylthio groups, $C_1$-$C_{12}$ alkoxyphenyl-$C_1$-$C_{12}$ alkylthio groups, $C_1$-$C_{12}$ alkylphenyl-$C_1$-$C_{12}$ alkylthio groups, 1-naphthyl-$C_1$-$C_{12}$ alkylthio groups, and 2-naphthyl-$C_1$-$C_{12}$ alkylthio groups. Among these, from the viewpoints of solubility in an organic solvent, device properties, easiness of synthesis, and the like, preferable are $C_1$-$C_{12}$ alkoxyphenyl-$C_1$-$C_{12}$ alkylthio groups and $C_1$-$C_{12}$ alkylphenyl-$C_1$-$C_{12}$ alkylthio groups.

The alkenyl group has a carbon number of about 2 to 30, and preferably about 2 to 15. Examples of such an alkenyl group include a vinyl group, 1-propylenyl group, 2-propylenyl group, butenyl group, pentenyl group, hexenyl group, heptenyl group, octenyl group, and cyclohexenyl group. Moreover, examples of such an alkenyl group include dienyl groups and trienyl groups such as a 1,3-butadienyl group, cyclohexa-1,3-dienyl group, and 1,3,5-hexatrienyl group.

The alkynyl group has a carbon number of about 2 to 30, and preferably about 2 to 15. Examples of such an alkynyl group include an ethynyl group, 1-propynyl group, 2-propylenyl group, butynyl group, pentynyl group, hexynyl group, heptynyl group, octynyl group, and cyclohexylethynyl group. Moreover, examples of such an alkynyl group also

include diynyl groups such as a 1,3-butadiynyl group.

Examples of the disubstituted amino group include amino groups substituted with two groups selected from the group consisting of an alkyl group, aryl group, arylalkyl group, and monovalent heterocyclic group. In such a disubstituted amino group, the alkyl group, aryl group, arylalkyl group, or monovalent heterocyclic group each may have a substituent. Meanwhile, the carbon number of such a disubstituted amino group is usually about 2 to 60, and preferably about 2 to 30, not including the carbon number of the substituent that the alkyl group or the like has. Examples of such a disubstituted amino group include a dimethylamino group, diethylamino group, dipropylamino group, diisopropylamino group, dibutylamino group, diisobutylamino group, di-t-butylamino group, dipentylamino group, dihexylamino group, dicyclohexylamino group, diheptylamino group, dioctylamino group, di-2-ethylhexylamino group, dinonylamino group, didecylamino group, di-3,7-dimethyloctylamino group, dilaurylamino group, dicyclopentylamino group, dicyclohexylamino group, pyrrolidyl group, piperidyl group, ditrifluoromethylamino group, phenylamino group, diphenylamino group, di($C_1$-$C_{12}$ alkoxyphenyl) amino groups, di($C_1$-$C_{12}$ alkylphenyl)amino groups, di-1-naphthylamino group, di-2-naphthylamino group, dipentafluorophenylamino group, dipyridylamino group, dipyridazinylamino group, dipyrimidylamino group, dipyrazylamino group, ditriazylamino group, di(phenyl-$C_1$-$C_{12}$ alkyl)amino groups, di($C_1$-$C_{12}$ alkoxyphenyl-$C_1$-$C_{12}$ alkyl) amino groups, and di($C_1$-$C_{12}$ alkylphenyl-$C_1$-$C_{12}$ alkyl)amino groups.

Examples of the trisubstituted silyl group include silyl groups substituted with three groups selected from the group consisting of an alkyl group, aryl group, arylalkyl group, and monovalent heterocyclic group. The carbon number of such a trisubstituted silyl group is usually about 3 to 90, and preferably about 3 to 45. Note that, in such a trisubstituted silyl group, the alkyl group, aryl group, arylalkyl group, or monovalent heterocyclic group may have a substituent. Examples of such a trisubstituted silyl group include a trimethylsilyl group, triethylsilyl group, tripropylsilyl group, tri-i-propylsilyl group, dimethyl-i-propylisilyl group, diethyl-i-propylsilyl group, t-butylsilyldimethylsilyl group, pentyldimethylsilyl group, hexyldimethylsilyl group, heptyldimethylsilyl group, octyldimethylsilyl group, 2-ethylhexyl-dimethylsilyl group, nonyldimethylsilyl group, decyldimethylsilyl group, 3,7-dimethyloctyl-dimethylsilyl group, lauryldimethylsilyl group, phenyl-$C_1$-$C_{12}$ alkylsilyl groups, $C_1$-$C_{12}$ alkoxyphenyl-$C_1$-$C_{12}$ alkylsilyl groups, $C_1$-$C_{12}$ alkylphenyl-$C_1$-$C_{12}$ alkylsilyl groups, 1-naphthyl-$C_1$-$C_{12}$ alkylsilyl groups, 2-naphthyl-$C_1$-$C_{12}$ alkylsilyl groups, phenyl-$C_1$-$C_{12}$ alkyldimethylsilyl groups, triphenylsilyl group, tri-p-xylylsilyl group, tribenzylsilyl group, diphenylmethylsilyl group, t-butyldiphenylsilyl group, and dimethylphenylsilyl group.

The acyl group has a carbon number of usually about 2 to 30, and preferably about 2 to 15. Examples of such an acyl group include an acetyl group, propionyl group, butyryl group, isobutyryl group, pivaloyl group, benzoyl group, trifluoroacetyl group, and pentafluorobenzoyl group.

The acyloxy group has a carbon number of usually about 2 to 30, and preferably about 2 to 15. Examples of such an acyloxy group include an acetoxy group, propionyloxy group, butyryloxy group, isobutyryloxy group, pivaloyloxy group, benzoyloxy group, trifluoroacetyloxy group, and pentafluorobenzoyloxy group.

The imine residue has a carbon number of about 2 to 30, and preferably about 2 to 15. Examples of such an imine residue include groups represented by structural formulae shown below. Note that, in the structural formulae shown below, a wavy line represents syn or anti position, and may be either syn or anti position.

[Chemical formula 4]

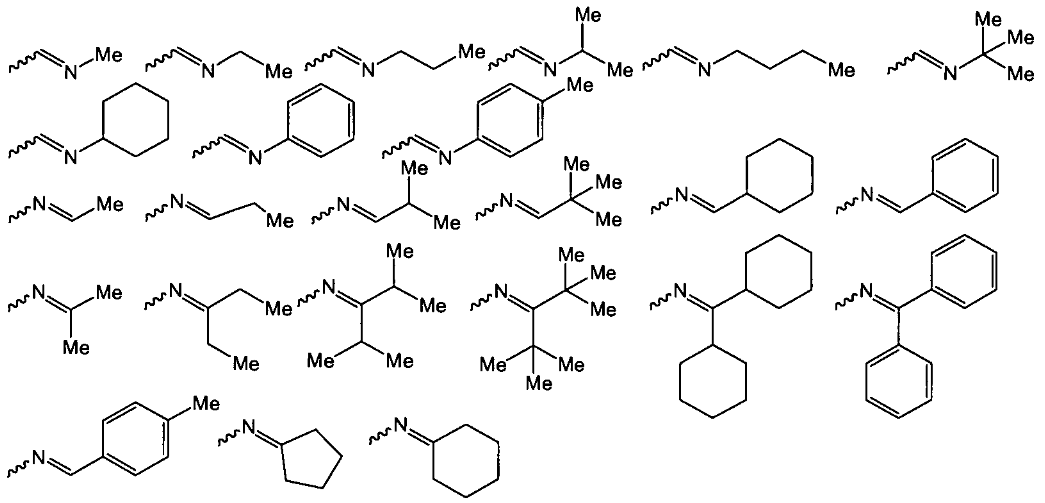

The amide group has a carbon number of usually about 2 to 30, and preferably about 2 to 15. Examples of such an amide group include a formamide group, acetamide group, propioamide group, butyroamide group, benzamide group, trifluoroacetamide group, pentafluorobenzamide group, diformamide group, diacetamide group, dipropioamide group, dibutyroamide group, dibenzamide group, ditrifluoroacetamide group, and dipentafluorobenzamide group.

The acid imide group is exemplified as a residue obtained by removing, from an acid imide, a hydrogen atom bonded to a nitrogen atom thereof. The carbon number of such an acid imide group is about 4 to 30, and preferably about 4 to 15. Examples of such an acid imide group include groups represented by structural formulae shown below.

[Chemical formula 5]

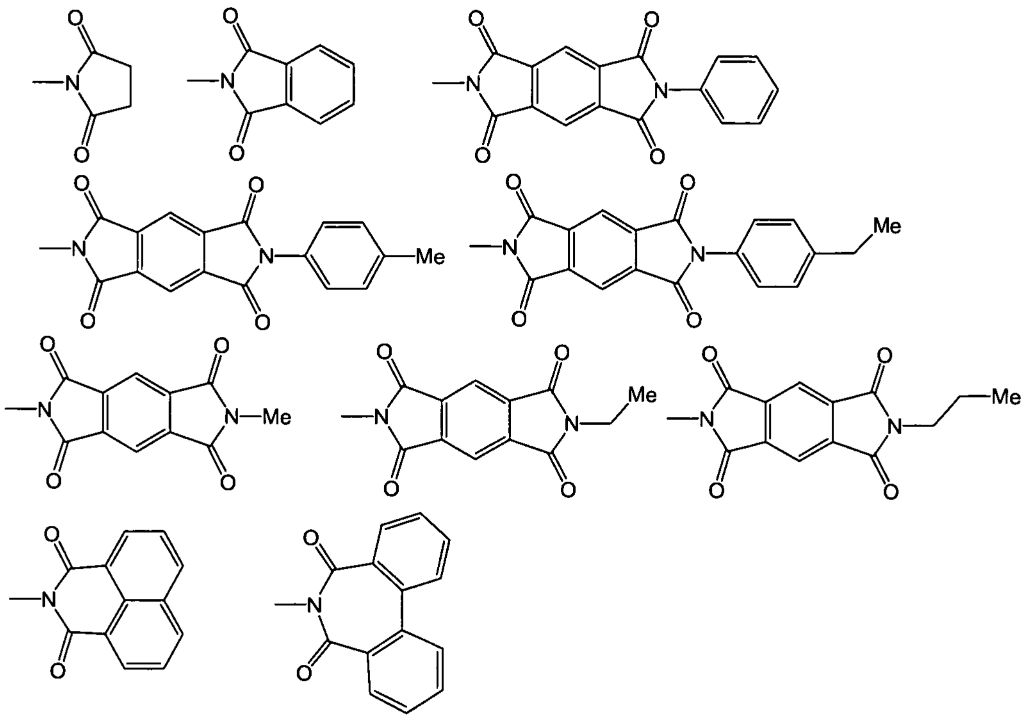

The monovalent heterocyclic group refers to an atomic group remaining after removing one hydrogen atom

from a heterocyclic compound. The carbon number of such a monovalent heterocyclic group is usually about 2 to 30, and preferably about 2 to 15. Note that, in such a monovalent heterocyclic group, a heterocyclic ring thereof may have a substituent; however, the carbon number of the substituent on the heterocyclic ring is not included in the carbon number of the monovalent heterocyclic group. Moreover, the heterocyclic compound here refers to an organic compound having a cyclic structure in which elements constituting the ring include not only a carbon atom but also a heteroatom such as oxygen, sulfur, nitrogen, phosphorus, and boron in the ring. Examples of such a monovalent heterocyclic group include a thienyl group, $C_1$-$C_{12}$ alkylthienyl groups, pyrrolyl group, furyl group, pyridyl group, $C_1$-$C_{12}$ alkylpyridyl group, piperidyl groups, quinolyl group, and isoquinolyl group. Among these, preferable are monovalent aromatic heterocyclic groups, and particularly preferable are a thienyl group, $C_1$-$C_{12}$ alkylthienyl groups, pyridyl group, and $C_1$-$C_{12}$ alkylpyridyl groups.

Examples of the substituted carboxyl group include carboxyl groups substituted with an alkyl group, aryl group, arylalkyl group or monovalent heterocyclic group. The carbon number of such a substituted carboxyl group is usually about 2 to 30, and preferably about 2 to 15. Examples of such a substituted carboxyl group include a methoxycarbonyl group, ethoxycarbonyl group, propoxycarbonyl group, i-propoxycarbonyl group, butoxycarbonyl group, i-butoxycarbonyl group, t-butoxycarbonyl group, pentyloxycarbonyl group, hexyloxycarbonyl group, cyclohexyloxycarbonyl group, heptyloxycarbonyl group, octyloxycarbonyl group, 2-ethylhexyloxycarbonyl group, nonyloxycarbonyl group, decyloxycarbonyl group, 3,7-dimethyloctyloxycarbonyl group, dodecyloxycarbonyl group, trifluoromethoxycarbonyl group, pentafluoroethoxycarbonyl group, perfluorobutoxycarbonyl group, perfluorohexyloxycarbonyl group, perfluorooctyloxycarbonyl group, phenoxycarbonyl group, naphthoxycarbonyl group, and pyridyloxycarbonyl group. Note that, in such a substituted carboxyl group, the alkyl group, aryl group, arylalkyl group, or monovalent heterocyclic group may have a substituent. Meanwhile, the carbon number of the substituent that the alkyl group or the like has is not included in the carbon number of the substituted carboxyl group.

The heteroaryloxy group (which is a group represented by $Q^1$-O-, where $Q^1$ represents a monovalent heterocyclic group) has a carbon number of usually about 2 to 30, and preferably about 2 to 15. Note that, in such a heteroaryloxy group, the monovalent heterocyclic group may have a substituent; however, the carbon number of the substituent on the monovalent heterocyclic group is not included in the carbon number of the heteroaryloxy group. Examples of such a heteroaryloxy group include a thienyloxy group, $C_1$-$C_{12}$ alkylthienyloxy groups, pyrrolyloxy group, furyloxy group, pyridyloxy group, $C_1$-$C_{12}$ alkylpyridyloxy groups, imidazolyloxy group, pyrazolyloxy group, triazolyloxy group, oxazolyloxy group, thiazoloxy group, and thiadiazoloxy group. Moreover, $Q^1$ is preferably a monovalent aromatic heterocyclic group.

The heteroarylthio group (which is a group represented by $Q^2$-S-, where $Q^2$ is a monovalent heterocyclic group) has a carbon number of usually about 2 to 30, and preferably about 2 to 15. Note that, in such a heteroarylthio group, the monovalent heterocyclic group may have a substituent; however, the carbon number of the substituent on the monovalent heterocyclic group is not included in the carbon number of the heteroarylthio group. Examples of such a heteroarylthio group include a thienylmercapto group, $C_1$-$C_{12}$ alkylthienylmercapto groups, pyrrolylmercapto group, furylmercapto group, pyridylmercapto group, $C_1$-$C_{12}$ alkylpyridylmercapto groups, imidazolylmercapto group, pyrazolylmercapto group, triazolylmercapto group, oxazolylmercapto group, thiazolemercapto group, and thiadiazolemercapto group. Moreover, $Q^2$ is preferably a monovalent aromatic heterocyclic group.

In the general formula (1), $R^1$ and $R^4$ each independently represent a monovalent group.

In the present description, examples of the monovalent group include an alkyl group, aryl group, arylalkyl group, alkenyl group, alkynyl group, trisubstituted silyl group, acyl group, monovalent heterocyclic group, and substituted carboxyl group.

Examples of these alkyl group, aryl group, arylalkyl group, alkenyl group, alkynyl group, trisubstituted silyl group, acyl group, monovalent heterocyclic group, and substituted carboxyl group include the same groups as those exemplified as the substituent that the ring A, the ring B and the ring C may have.

From the viewpoint of stability of the compound, $R^1$ and $R^4$ are preferably each independently any one of alkyl group, aryl group, arylalkyl group, and monovalent heterocyclic group, and more preferably an aryl group.

In the general formula (1), $R^2$, $R^3$, $R^5$ and $R^6$ each independently represent any one of a hydrogen atom, alkyl group, aryl group, arylalkyl group, alkenyl group, and alkynyl group.

The alkyl group represented by $R^2$, $R^3$, $R^5$ and $R^6$ may be linear, branched or cyclic, but does not have a substituent. The carbon number of such an alkyl group is usually about 1 to 30, and preferably about 3 to 15 from the viewpoint of solubility in a solvent. Examples of such an alkyl group include a methyl group, ethyl group, propyl group, i-propyl group, butylgroup, i-butyl group, t-butyl group, pentyl group, isoamyl group, hexyl group, cyclohexyl group, heptyl group, octyl group, 2-ethylhexyl group, nonyl group, decyl group, 3,7-dimethyloctyl group, and lauryl group. Among these, from the viewpoint of balancing heat resistance with solubility in an organic solvent, device properties, easiness of synthesis, and the like, preferable are a pentyl group, isoamyl group, hexyl group, octyl group, 2-ethylhexyl group, decyl group, and 3,7-dimethyloctyl group.

Examples of the aryl group, arylalkyl group, alkenyl group, and alkynyl group, which are represented by $R^2$, $R^3$, $R^5$ and $R^6$, include the same groups as those exemplified as the substituent that the ring A, the ring B and the ring C may have.

From the viewpoint of easiness of synthesis of the compound, $R^2$, $R^3$, $R^5$ and $R^6$ are preferably each independently any one of alkyl group, aryl group, and arylalkyl group.

Furthermore, from the viewpoint of easiness of synthesis of the structure represented by the general formula (1), it is preferable that the structure represented by the general formula (1) be C2 symmetry.

Moreover, from the viewpoint of enabling light emitted from the compound to have a shorter wavelength and thus having an increased purity of blue color, $R^2$, $R^3$, $R^5$ and $R^6$ in the general formula (1) are preferably each independently any one of a hydrogen atom and monovalent hydrocarbon group. Furthermore, from the viewpoint of stability in conducting a current, $R^2$, $R^3$, $R^5$ and $R^6$ are preferably each independently a monovalent hydrocarbon group. Here, the "monovalent hydrocarbon group" is a group formed of only a carbon atom and a hydrogen atom, and may be an aliphatic group or aromatic group.

Moreover, from the viewpoints of heat resistance and film formability, $R^2$, $R^3$, $R^5$ and $R^6$ in the general formula (1) are preferably each independently a group represented by the following general formula (2).

[Chemical formula 6]

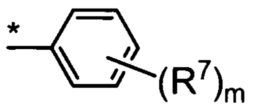

(2)

In the formula (2), * represents a bond to a carbon atom. Morever, $R^7$ represents any one of a halogen atom, alkyl group, alkoxy group, alkylthio group, aryl group, aryloxy group, arylthio group, arylalkyl group, arylalkyloxy group, arylalkylthio group, alkenyl group, alkynyl group, disubstituted amino group, and trisubstituted silyl group. Further, m represents an integer of 0 to 5. Furthermore, when m is 2 or more, $R^7$'s may be the same or different.

In this manner, examples of the halogen atom, alkyl group, alkoxy group, alkylthio group, aryl group, aryloxy group, arylthio group, arylalkyl group, arylalkyloxy group, arylalkylthio group, alkenyl group, alkynyl group, disubstituted amino group, or trisubstituted silyl group, which is represented by $R^7$, include the same groups as those exemplified as the substituent that the ring A, the ring B and the ring C may have. Among these, from the viewpoint of stability of the compound, preferable are the alkyl group, alkoxy group, aryl group, aryloxy group, and disubstituted amino group.

Moreover, from the viewpoint of solubility of the compound, $R^7$ is preferably any one of alkyl group, alkoxy group, alkylthio group, alkenyl group, alkynyl group, and disubstituted amino group, each of which has a carbon number of 3 or more. Above all, an alkyl group having a carbon number of 3 or more is preferable.

As the general formula (2), the case that the formula is represented by the following general formula (2-1) is preferable.

[Chemical formula 7]

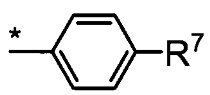

(2-1)

In the general formula (2-1), $R^7$ represents the same meaning as the above.

Furthermore, from the viewpoint of conductivity of the compound, polymerization is preferably carried out at a bond position where conjugations connected. Particularly, it is preferable that the structure represented by the general formula (1) in the compound be a repeating unit represented by the following general formula (3).

[Chemical formula 8]

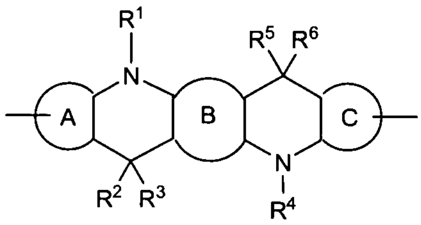

(3)

In the formula (3), a ring A, a ring B and a ring C each independently represent any one of a monocyclic aromatic ring and fused aromatic ring; $R^1$ and $R^4$ each independently represent a monovalent group; and $R^2$, $R^3$, $R^5$ and $R^6$ each independently represent any one of a hydrogen atom, alkyl group, aryl group, arylalkyl group, alkenyl group, and alkynyl group. Moreover, the ring A and the ring C each have a bond thereon. Additionally, examples of each of the ring A, the ring B, the ring C, $R^1$, $R^2$, $R^3$, $R^4$ , $R^5$ and $R^6$ include those described above.

Moreover, when the structure represented by the general formula (1) in the compound is the repeating unit represented by the general formula (3), from the viewpoint of easiness of synthesis of the compound, the ring A and the ring C in the general formula (3) are preferably benzene rings. Specifically, a case that the repeating unit is a repeating unit represented by the following general formula (4) is preferable.

[Chemical formula 9]

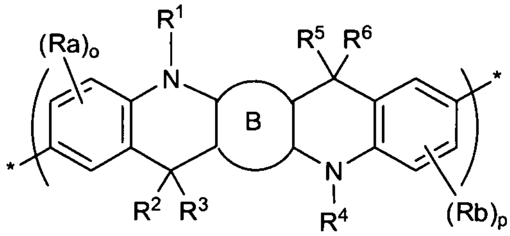

(4)

In the formula (4), * represents a bond; a ring B represents any one of a monocyclic aromatic ring and fused aromatic ring; $R^1$ and $R^4$ each independently represent a monovalent group; and $R^2$, $R^3$, $R^5$ and $R^6$ each independently represent any one of a hydrogen atom, alkyl group, aryl group, arylalkyl group, alkenyl group, and alkynyl group. Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3, a plurality of Rb's may be the same or different.

Moreover, examples of each of $R^1$, $R^2$, $R^3$, $R^4$, $R^5$ and $R^6$ in the general formula (4) include those described above. Furthermore, examples of the rings B include each of those described above. Among such rings B, from the viewpoints of heat resistance, fluorescence intensity, device properties, and the like, preferable are aromatic hydrocarbon rings; more preferable are a benzene ring, naphthalene ring, anthracene ring, and phenanthrene ring; and particularly preferable is a benzene ring. In Ra and Rb, examples of the alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group include the same groups as those exemplified as the substituent that the ring A, the ring B and the ring C may have. From the viewpoint of easiness of synthesizing the compound, it is preferable that o and p be 0.

It is further preferable that the general formula (4) be represented by the following general formula (4-1).

[Chemical formula 10]

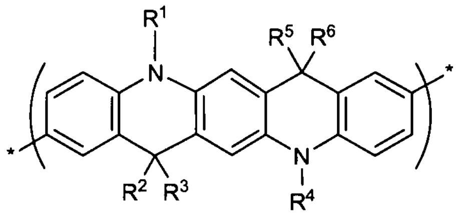

(4-1)

In the general formula (4-1), $R^1$, $R^2$, $R^3$, $R^4$, $R^5$ and $R^6$ represent the same meaning as the above.

Furthermore, from the viewpoints that the compound is excellent in device properties such as luminous efficiency and lifetime when used in an organic electroluminescence device, the compound comprising the structure represented by the general formula (1) preferably further comprises a repeating unit represented by the following general formula (5).

[Chemical formula 11]

$$-Ar^1\!\left(CR^8{=}CR^9\right)_n \qquad (5)$$

In the general formula (5), $Ar^1$ represents any one of arylene group and divalent heterocyclic group. Moreover, $R^8$ and $R^9$ each independently represent any one of a hydrogen atom, alkyl group, aryl group, monovalent heterocyclic group, and cyano group. n represents 0 or 1. Note that $Ar^1$ may be two or more species.

In $Ar^1$, the arylene group has a carbon number of usually 6 to 60, and preferably 6 to 20. In $Ar^1$, examples of such an arylene group include phenylene groups (for example, general formulae 1 to 3 shown below), naphthalenediyl groups (for example, general formulae 4 to 13 shown below), anthracenylene groups (for example, general formulae 14 to 19 shown below), biphenylene groups (for example, general formulae 20 to 25 shown below), triphenylene groups (for example, general formulae 26 to 28 shown below), and fused-ring compound groups (for example, general formula 29 to 38 shown below). Note that, in the formulae shown below, R's each independently represent any one of a hydrogen atom, halogen atom, alkyl group, alkoxy group, alkylthio group, aryl group, aryloxy group, arylthio group, arylalkyl group, arylalkyloxy group, arylalkylthio group, alkenyl group, alkynyl group, heteroaryloxy group, and heteroarylthio group. Meanwhile, the carbon number of the substituent R is not included in the carbon number of the arylene group.

[Chemical formula 12]

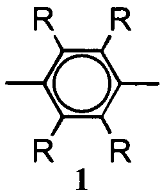
1
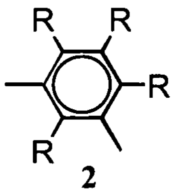
2
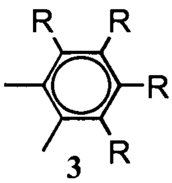
3

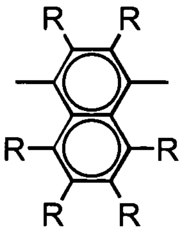
4
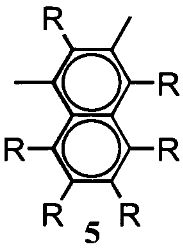
5
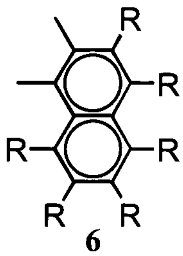
6
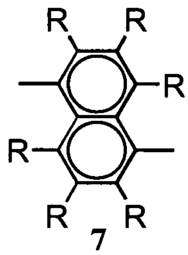
7

[Chemical formula 13]

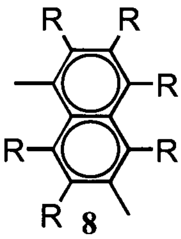
8
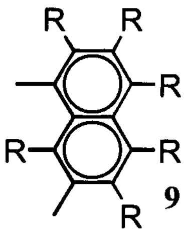
9
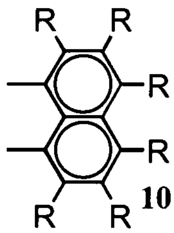
10
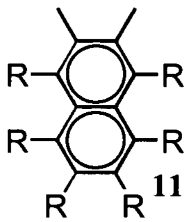
11

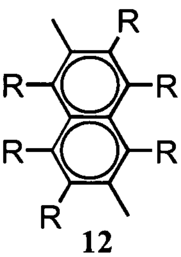
12
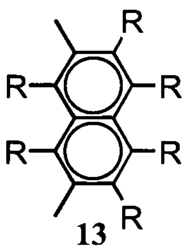
13
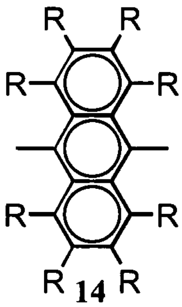
14
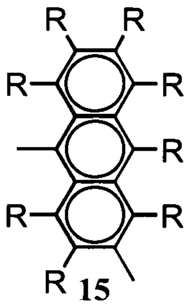
15

[Chemical formula 14]

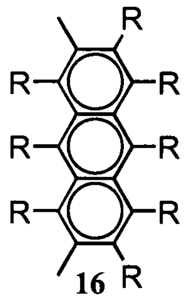
16

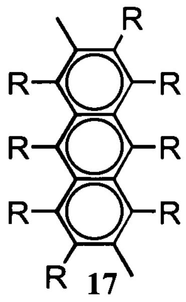
17

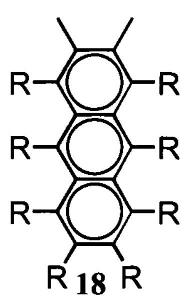
18

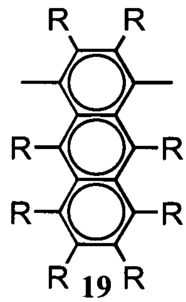
19

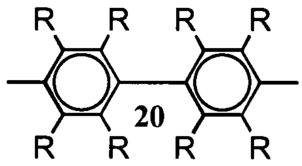
20

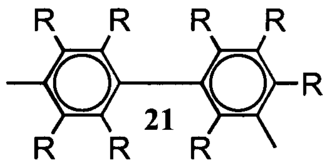
21

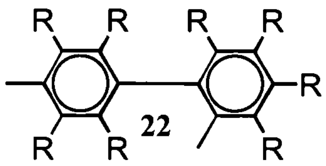
22

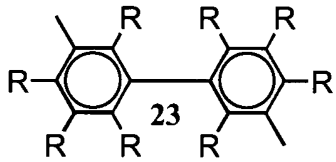
23

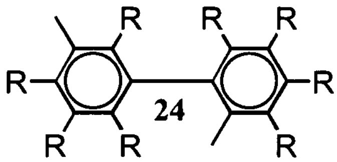
24

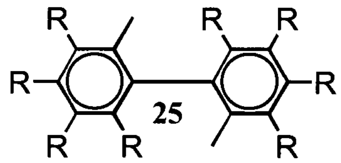
25

[Chemical formula 15]

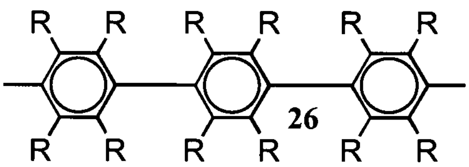
26

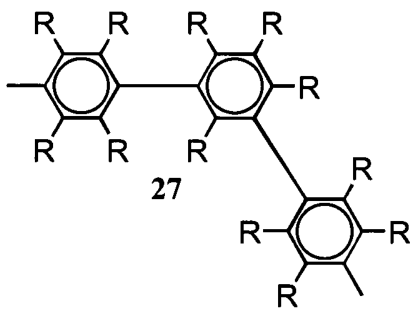
27

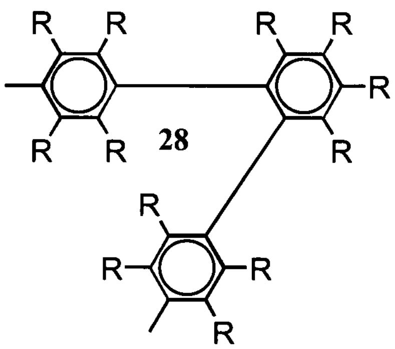
28

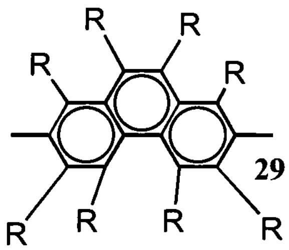
29

[Chemical formula 16]

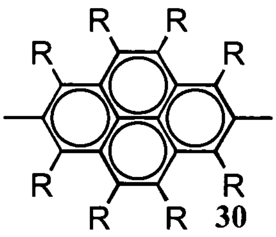
30

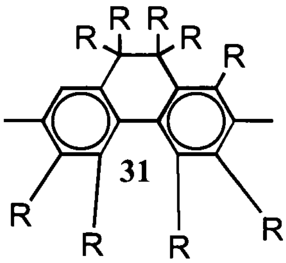
31

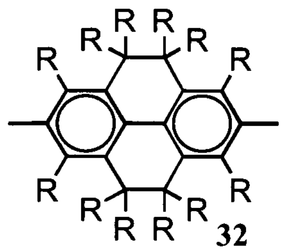
32

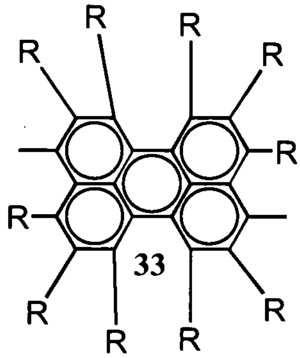
33

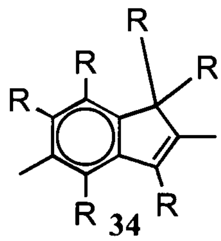
34

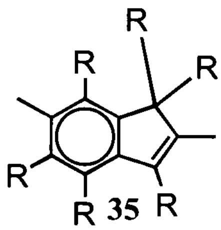
35

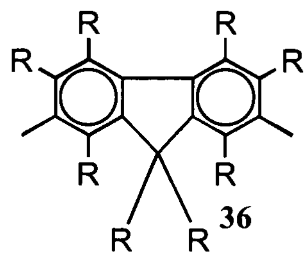
36

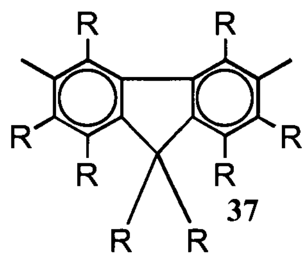
37

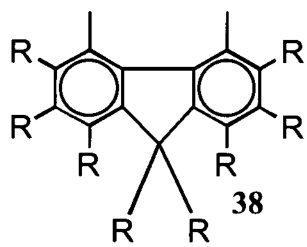
38

Moreover, in the present invention, the divalent heterocyclic group refers to an atomic group remaining after removing two hydrogen atoms from a heterocyclic compound. The carbon number of such a divalent heterocyclic group is usually 4 to 60, and preferably 4 to 20. Additionally, the heterocyclic compound here refers to an organic compound having a cyclic structure in which elements constituting the ring include not only a carbon atom but also a heteroatom such as oxygen, sulfur, nitrogen, phosphorus, and boron in the ring.

Examples of such a divalent heterocyclic group include the followings. Note that, in the formulae shown below, R's each independently represent a hydrogen atom, halogen atom, alkyl group, alkoxy group, alkylthio group, aryl group, aryloxy group, arylthio group, arylalkyl group, arylalkyloxy group, arylalkylthio group, alkenyl group, alkynyl group, heteroaryloxy group, or heteroarylthio group. Meanwhile, the carbon number of the substituent R is not included in the carbon number of the divalent heterocyclic group.

Examples of a divalent heterocyclic group including nitrogen as the heteroatom include pyridine-diyl groups (for example, general formulae 39 to 44 shown below), diazaphenylene groups (for example, general formulae 45 to 48 shown below), quinolinediyl groups (for example, general formulae 49 to 63 shown below), quinoxalinediyl groups (for example, general formulae 64 to 68 shown below), acridinediyl groups (for example, general formulae 69 to 72 shown below), bipyridyldiyl groups (for example, general formulae 73 to 75 shown below), and phenanthrolinediyl groups (for example, general formulae 76 to 78 shown below).

Examples of a group having a fluorene structure including silicon, nitrogen, sulfur, selenium, or the like as the heteroatom include groups represented by general formulae 79 to 93 shown below. Among these, desirable are groups having an aromatic amine monomer including a nitrogen atom such as a triphenylaminediyl group and carbazole represented by the formulae 82 to 84 in view of luminous efficiency.

Examples of a 5-membered ring heterocyclic group including silicon, nitrogen, sulfur, selenium, or the like as the heteroatom include groups represented by general formulae 94 to 98 shown below.

Examples of a fused 5-membered ring heterocyclic group including silicon, nitrogen, sulfur, selenium, or the

like as the heteroatom include groups represented by general formulae 99 to 109 shown below, a benzothiadiazole-4,7-diyl group, and a benzoxadiazole-4,7-diyl group.

Examples of a 5-membered ring heterocyclic group which includes silicon, nitrogen, sulfur, selenium, or the like as the heteroatom, and which is bonded at the α position of the heteroatom to form a dimer or an oligomer, include groups represented by general formulae 110 to 111 shown below.

Examples of a 5-membered ring heterocyclic group which includes silicon, nitrogen, sulfur, selenium, or the like as the heteroatom, and which is bonded to a phenyl group at the α position of the heteroatom, include groups represented by general formulae 112 to 118 shown below.

Examples of a tricyclic group in which a fused heterocyclic group including nitrogen, oxygen, sulfur, or the like as the heteroatom is bonded to benzene rings or to monocyclic heterocyclic groups include groups represented by general formulae 120 to 125 shown below.

[Chemical formula 17]

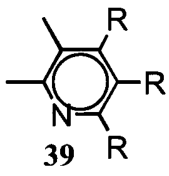
39
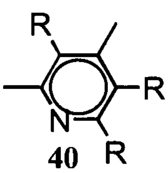
40
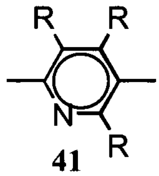
41
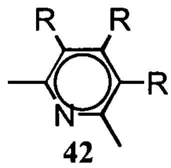
42
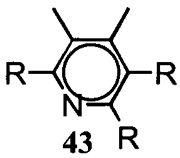
43
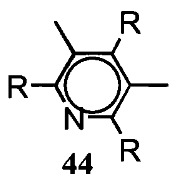
44
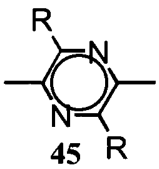
45
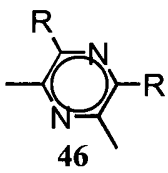
46
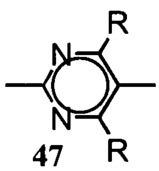
47
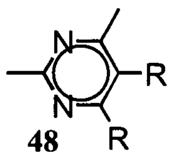
48

[Chemical formula 18]

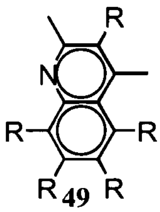
49
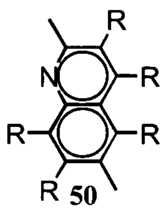
50
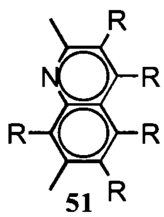
51
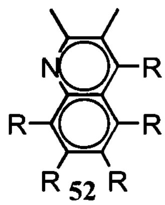
52
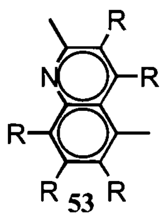
53
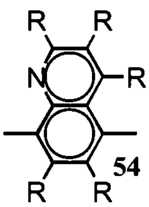
54
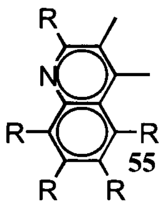
55
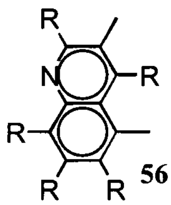
56
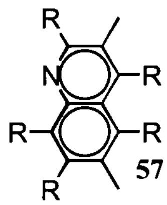
57
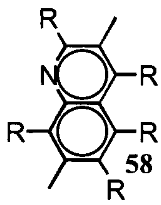
58

[Chemical formula 19]

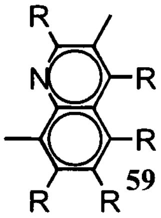 59
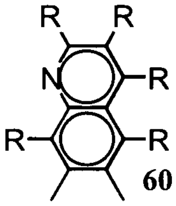 60
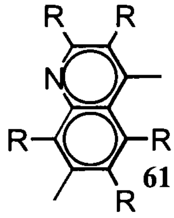 61
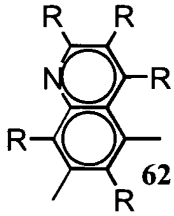 62
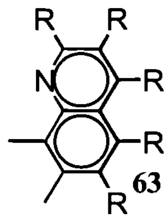 63

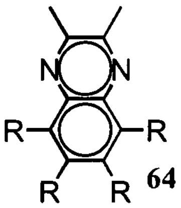 64
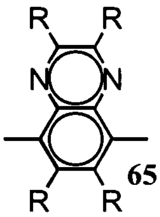 65
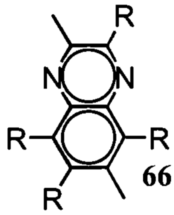 66
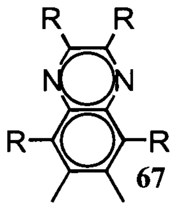 67
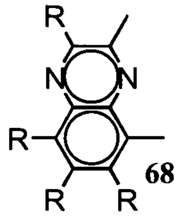 68

[Chemical formula 20]

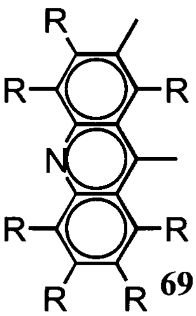 69
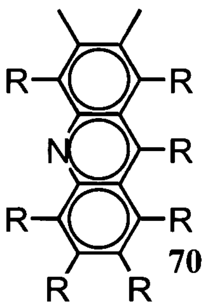 70
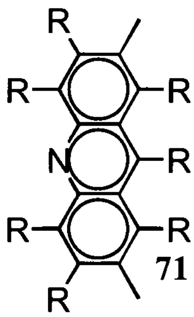 71
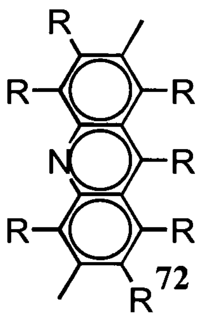 72

[Chemical formula 21]

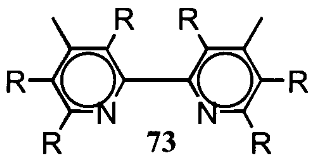
73

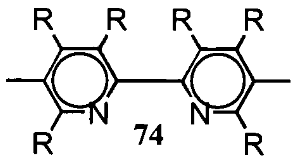
74

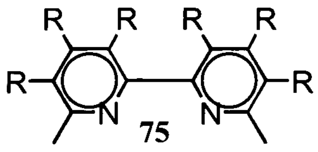
75

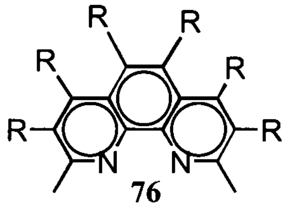
76

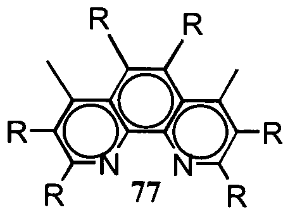
77

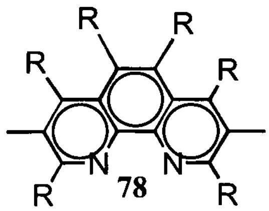
78

[Chemical formula 22]

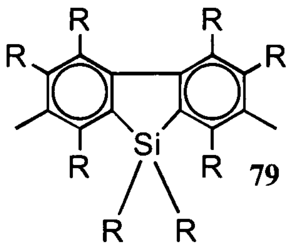
79

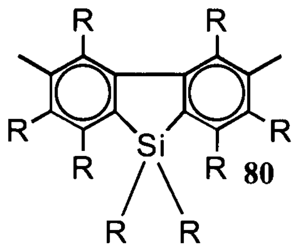
80

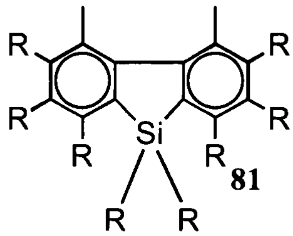
81

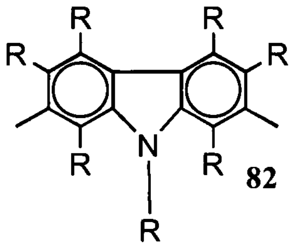
82

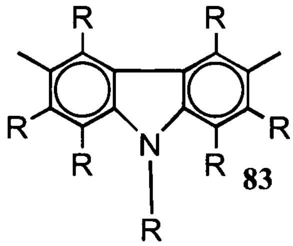
83

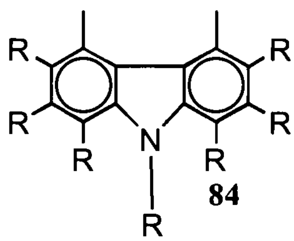
84

[Chemical formula 23]

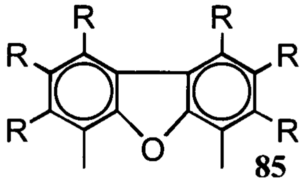
85

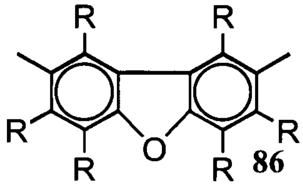
86

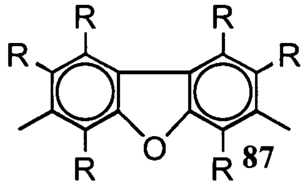
87

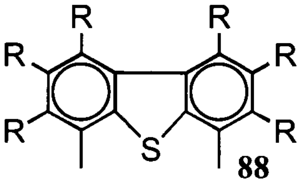
88

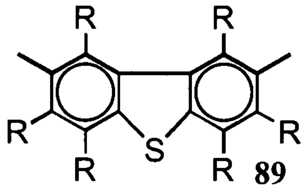
89

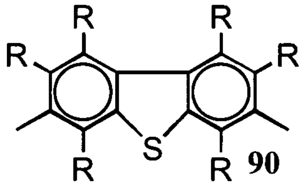
90

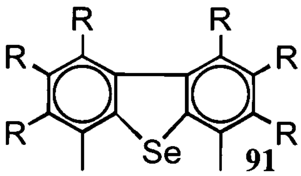
91

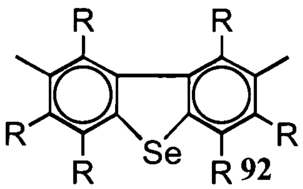
92

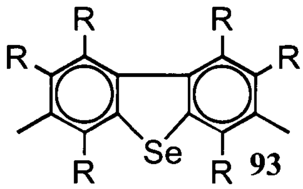
93

[Chemical formula 24]

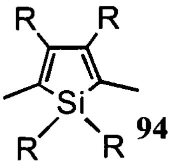 94 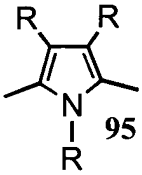 95 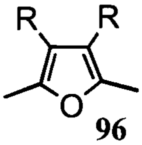 96 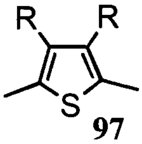 97 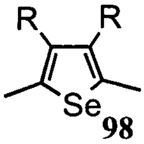 98

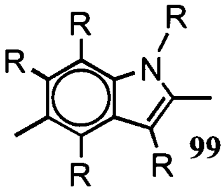 99 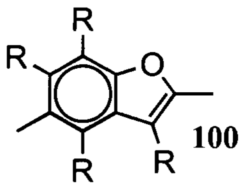 100 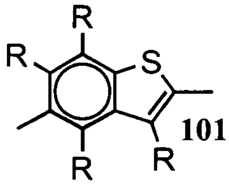 101 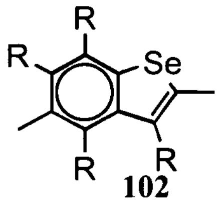 102

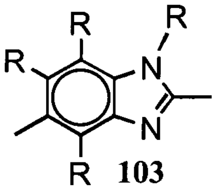 103 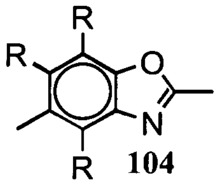 104 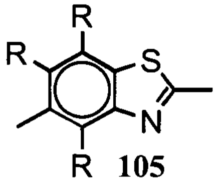 105 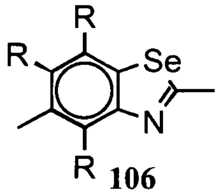 106

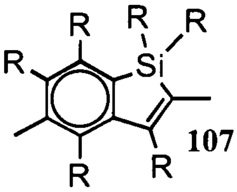 107 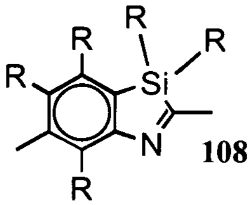 108 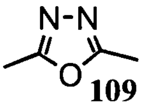 109

[Chemical formula 25]

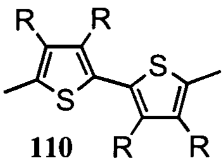
110

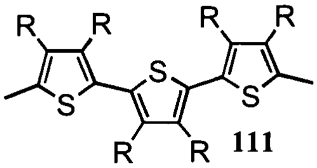
111

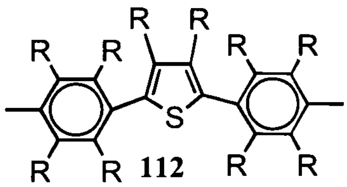
112

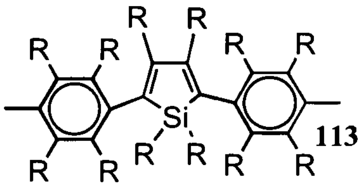
113

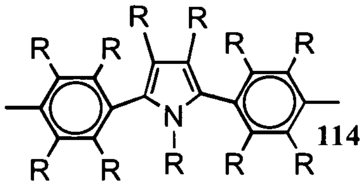
114

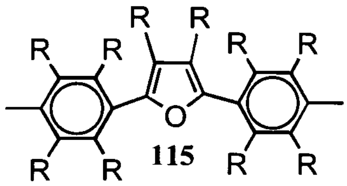
115

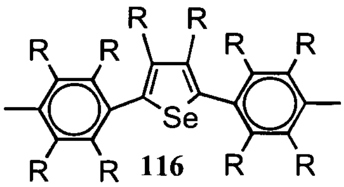
116

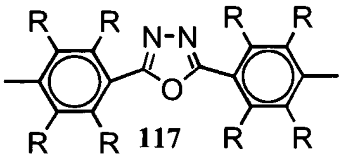
117

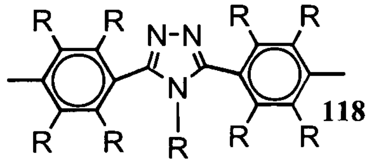
118

[Chemical formula 26]

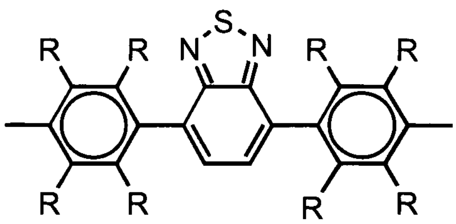
120

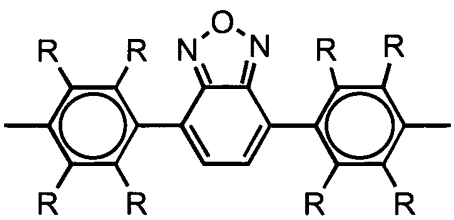
121

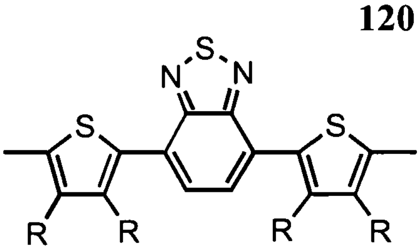
122

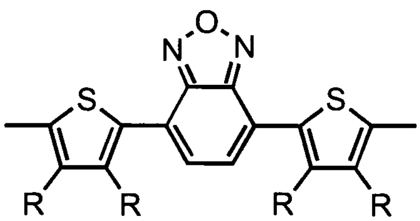
123

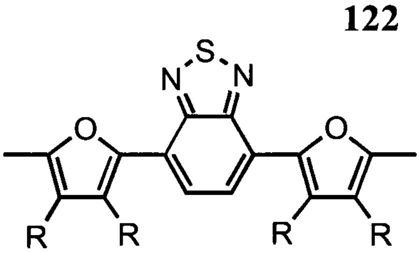
124

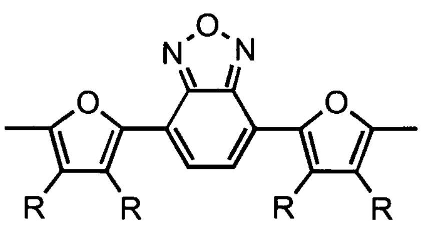
125

In the repeating unit represented by the general formula (5), it is preferable that n be 0, and more preferable that $Ar^1$ be an arylene group.

Moreover, as the repeating unit represented by the general formula (5), further preferable is a structure represented by the following general formula (5-1).

[Chemical formula 27]

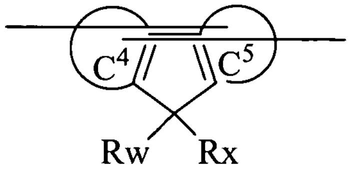

(5-1)

In the general formula (5-1), a ring $C^4$ and a ring $C^5$ each independently represent an aromatic hydrocarbon ring that may have a substituent. Two bonds are present on the ring $C^4$ or the ring $C^5$. Rw and Rx each independently represent any one of a hydrogen atom, alkyl group, alkoxy group, alkylthio group, aryl group, aryloxy group, arylthio group, arylalkyl group, arylalkoxy group, arylalkylthio group, alkenyl group, alkynyl group, disubstituted amino group, trisubstituted silyl group, acyl group, acyloxy group, imine residue, amide group, acid imide group, monovalent heterocyclic group, substituted carboxyl group, heteroaryloxy group, and heteroarylthio group. Rw and Rx may be bonded to each other to form a ring.

The aromatic hydrocarbon ring refers to a benzene ring or fused aromatic hydrocarbon ring. The carbon number of such an aromatic hydrocarbon ring is about 6 to 30, and preferably about 6 to 15. Note that the carbon number of the substituent is not included in the carbon number of the aromatic hydrocarbon group. Examples of such an aromatic hydrocarbon ring include a benzene ring, naphthalene ring, anthracene ring, phenanthrene ring, phenalene ring, naphthacene ring, triphenylene ring, pyrene ring, chrysene ring, pentacene ring, perylene ring, pentalene ring, indene ring, azulene ring, biphenylene ring, fluorene ring, and acenaphthylene ring.

In Rw and Rx, examples of the alkyl group, alkoxy group, alkylthio group, aryl group, aryloxy group, arylthio group, arylalkyl group, arylalkoxy group, arylalkylthio group, alkenyl group, alkynyl group, disubstituted amino group, trisubstituted silyl group, acyl group, acyloxy group, imine residue, amide group, acid imide group, monovalent heterocyclic group, substituted carboxyl group, heteroaryloxy group, and heteroarylthio group include the same groups as those exemplified as the substituent that the ring A, the ring B and the ring C may have.

Specific examples of the repeating unit represented by the general formula (5-1) include repeating units represented by general formulae shown below. Moreover, such repeating units may have at least one substituent selected from the group consisting of an alkyl group, alkoxy group, alkylthio group, aryl group, aryloxy group, arylthio group, arylalkyl group, arylalkoxy group, arylalkylthio group, alkenyl group, alkynyl group, disubstituted amino group, trisubstituted silyl group, acyl group, acyloxy group, imine residue, amide group, acid imide group, monovalent heterocyclic group, substituted carboxyl group, heteroaryloxy group, heteroarylthio group, halogen atom, and the like. Note that, in the general formulae shown below, it is shown that a bond can take any position.

[Chemical formula 28]

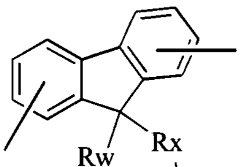
1A-0

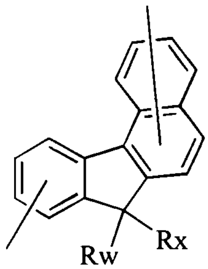
1A-1

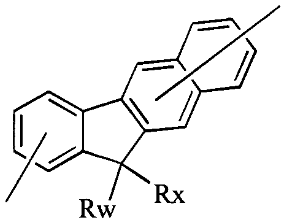
1A-2

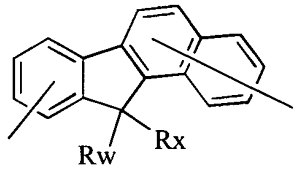
1A-3

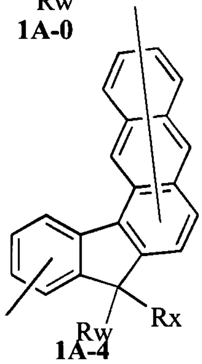
1A-4

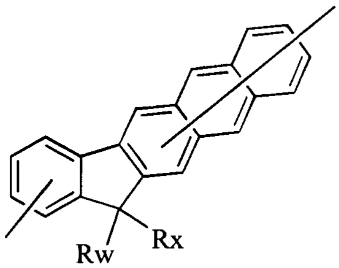
1A-5

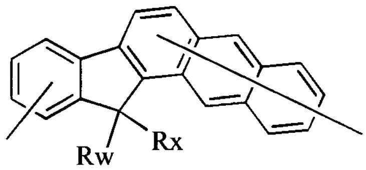
1A-6

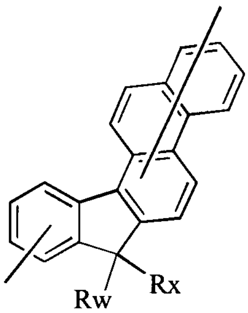
1A-7

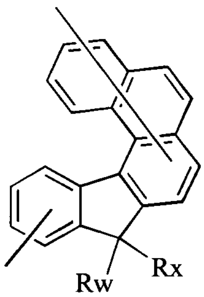
1A-8

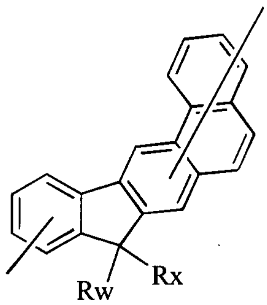
1A-9

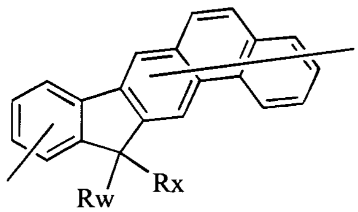
1A-10

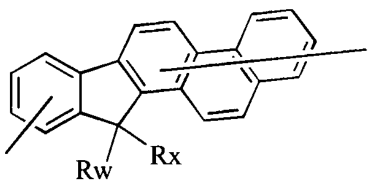
1A-11

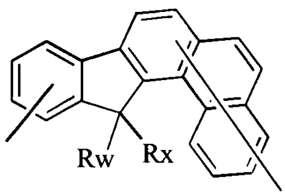
1A-12

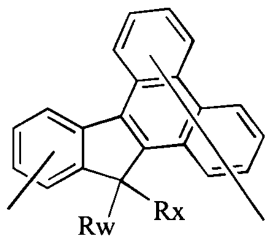
1A-13

Among these repeating units, preferable is a repeating unit represented by 1A-0, 1A-1, 1A-2 or 1A-3, and particularly preferable is a repeating unit represented by 1A-0.

Further preferable is a repeating unit represented by the following general formula (1A-0-1).

[Chemical formula 29]

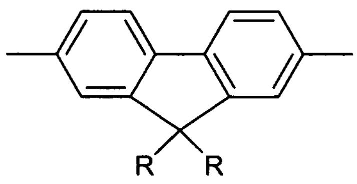

(1A-0-1)

In the general formula (1A-0-1), R is an alkyl group, aryl group, arylalkyl group, or monovalent heterocyclic compound group, and two R's may be the same as or different from each other. Moreover, R's may be bonded to each other to form a ring.

Furthermore, from the viewpoints of improvement in device properties such as improvement in heat resistance, improvement in charge transporting property, adjustment of luminous color, and increase in luminous efficiency, the compound of the present invention preferably further comprises at least one repeating unit selected from the group consisting of repeating units represented respectively by the following general formulae (6-1), (6-2) and (6-3), other than the repeating unit represented by the general formula (5). More preferably, the compound comprises two or more of the repeating units.

[Chemical formula 30]

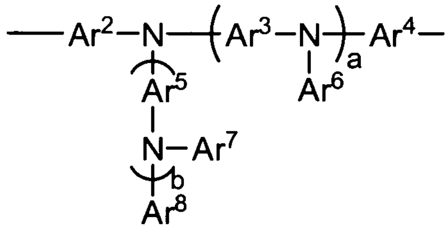

(6-1)

In the formula (6-1), $Ar^2$ , $Ar^3$, $Ar^4$, and $Ar^5$ each independently represent any one of arylene group and divalent heterocyclic group. Moreover, $Ar^6$, $Ar^7$ and $Ar^8$ each independently represent any one of aryl group and monovalent heterocyclic group. Furthermore, a and b each independently represent 0 or a positive integer. Additionally, $Ar^2$, $Ar^3$, $Ar^4$, $Ar^5$, $Ar^6$, $Ar^7$ and $Ar^8$ may have a substituent.

[Chemical formula 31]

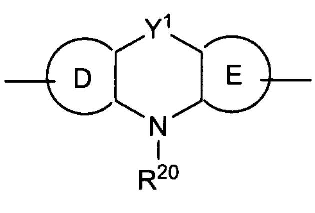

(6-2)

In the general formula (6-2), a ring D and a ring E each independently represent an aromatic ring. Moreover, $Y^1$ represents any one of -O-, -S-, and -C(=O)-. Furthermore, $R^{20}$ represents a monovalent group. Additionally, the ring D and the ring E each have a bond thereon.

[Chemical formula 32]

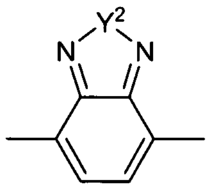

(6-3)

In the formula (6-3), $Y^2$ represents any one of -O-and -S-. Moreover, a 6-membered ring has two bonds thereon.
Specific examples of the repeating unit represented by the general formula (6-1) include ones represented by general formulae 133 to 140 shown below.

[Chemical formula 33]

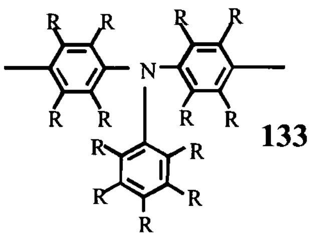

133

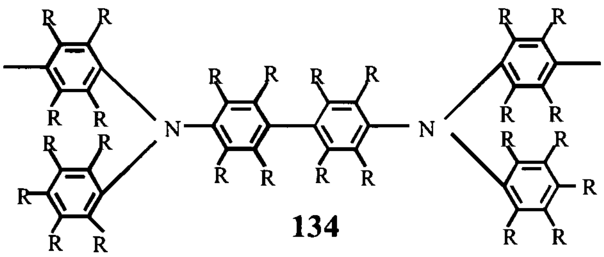

134

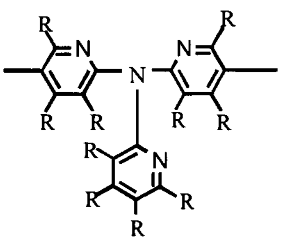

135

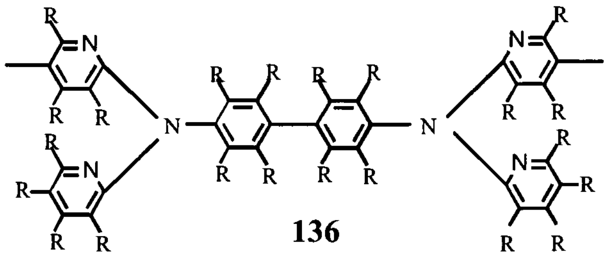

136

[Chemical formula 34]

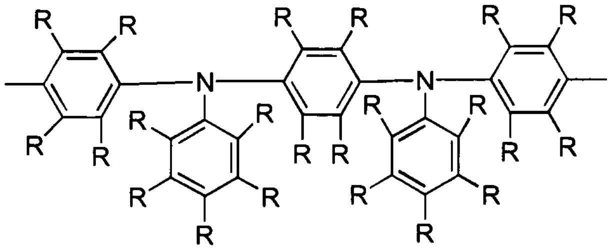

137

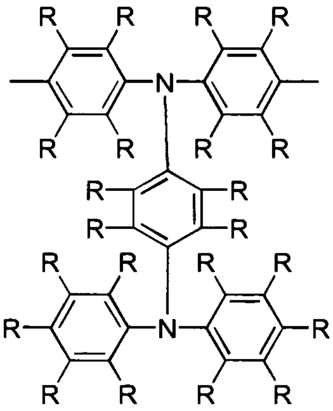

138

[Chemical formula 35]

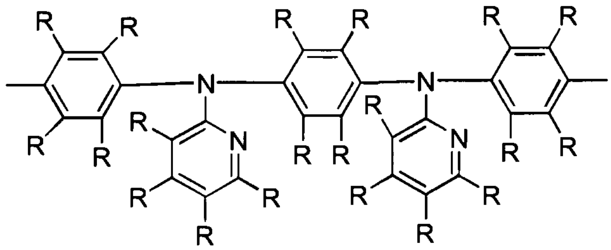

139

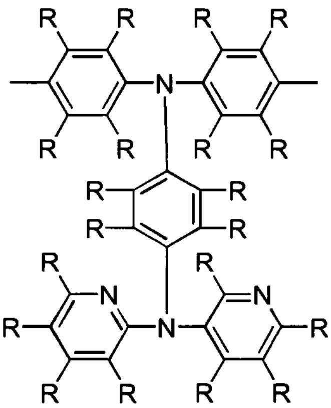

140

In the general formulae, R's each independently represent any one of a hydrogen atom, alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, monovalent heterocyclic group, carboxyl group, substituted carboxyl group, and cyano group.

Moreover, when R in the general formula is a substituent including an alkyl group, in order to increase the solubility of a polymer compound in an organic solvent, the substituent preferably includes an alkyl group having a carbon number of 3 or more. Furthermore, among the structures represented by the general formulae 133 to 140, from the viewpoint of adjusting luminescence wavelength, preferable are the structures represented by the general formulae 133, 134 and 137.

In the repeating unit represented by the general formula (6-1), from the viewpoints of device properties such as adjusting luminescence wavelength and device lifetime, $Ar^2$, $Ar^3$, $Ar^4$ and $Ar^5$ are preferably each independently an arylene group, and $Ar^6$, $Ar^7$ and $Ar^8$ are preferably each independently an aryl group. Moreover, $Ar^2$, $Ar^3$ and $Ar^4$ are preferably each independently any one of an unsubstituted phenylene group, unsubstituted biphenylene group, unsubstituted naphthylene group, and unsubstituted anthracenediyl group. Furthermore, from the viewpoints of solubility in an organic solvent, device properties, and the like, $Ar^6$ , $Ar^7$ and $Ar^8$ are preferably each independently an aryl group having one or more substituents, and more preferably an aryl group having three or more substituents. Moreover, $Ar^6$, $Ar^7$ and $Ar^8$ are more preferably each any one of a phenyl group having three or more substituents, a naphthyl group

having three or more substituents, and a anthracenyl group having three or more substituents. $Ar^6$, $Ar^7$ and $Ar^8$ are further preferably each a phenyl group having three or more substituents.

Among these repeating units, preferable is a repeating unit in which $Ar^6$, $Ar^7$ and $Ar^8$ are each independently a group represented by the following general formula (6-4) where $a+b\leq3$. It is more preferable that $a+b=1$, and particularly preferable that $a=1$, $b=0$.

[Chemical formula 36]

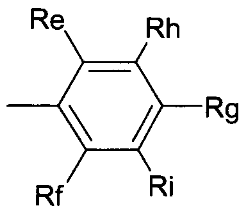

(6-4)

In the general formula (6-4), Re, Rf and Rg each independently represent any one of alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, silyloxy group, substituted silyloxy group, monovalent heterocyclic group, and halogen atom. Moreover, a hydrogen atom included in Re, Rf and Rg may be substituted with a fluorine atom. Furthermore, Rh and Ri each independently represent any one of a hydrogen atom, alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, silyloxy group, substituted silyloxy group, monovalent heterocyclic group, and halogen atom. Additionally, a hydrogen atom included in Rh and Ri may be substituted with a fluorine atom.

Moreover, in the general formula (6-4), Re and Rf are preferably each independently any one of an alkyl group having a carbon number of 3 or less, alkoxy group having a carbon number of 3 or less, and alkylthio group having a carbon number of 3 or less. In addition, Rg is preferably any one of an alkyl group having a carbon number of 1 to 30, alkoxy group having a carbon number of 1 to 30, and alkylthio group having a carbon number of 1 to 30.

In the repeating unit represented by the general formula (6-1), $Ar^3$ is preferably a group represented by the following general formula (6-5) or (6-6).

[Chemical formula 37]

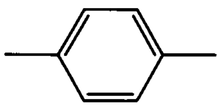

(6-5)

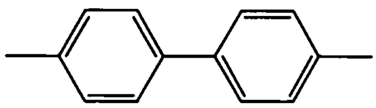

(6-6)

In the general formulae (6-5) and (6-6), benzene rings included in the structures are preferably unsubstituted, but each independently may have at least 1 but not more than 4 substituents. These substituents may be the same as or different from each other. Moreover, another aromatic hydrocarbon ring or heterocyclic ring may be fused to such benzene rings.

In addition, more preferable specific examples of the repeating unit represented by the general formula (6-1) include ones represented by general formulae 141 to 143 shown below.

[Chemical formula 38]

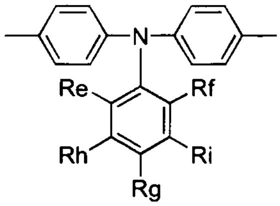

141

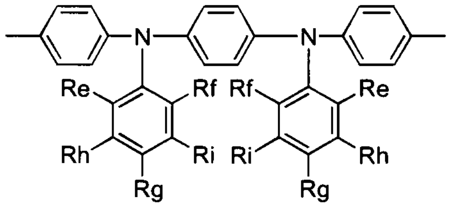

142

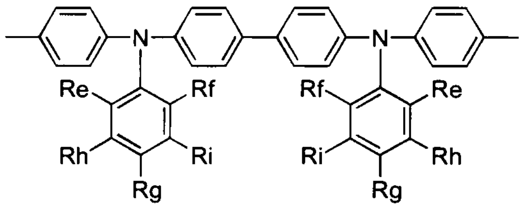

143

In the general formulae, examples of each of Re, Rf, Rg, Rh and Ri include those described above.

Furthermore, from the viewpoints of device properties such as fluorescence intensity, adjustment of luminous wavelength, and heat resistance, particularly preferable specific examples of the repeating unit represented by the general formula (6-1) include ones represented by the following general formulae (22) to (24).

[Chemical formula 39]

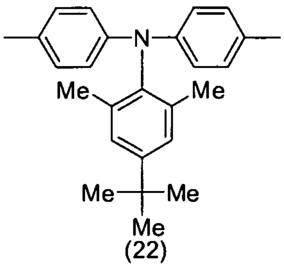

(22)

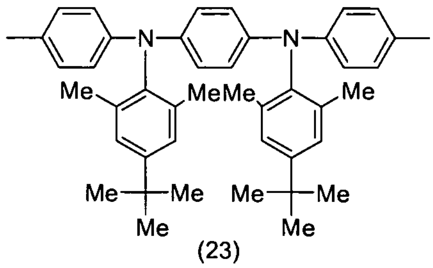

(23)

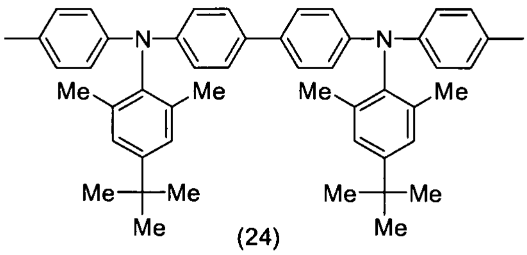

(24)

In the general formula (6-2), the ring D and the ring E each independently represent an aromatic ring. Alternatively, the ring D and the ring E each may have a substituent thereon. From the viewpoint of stability of the compound, the ring D and the ring E are preferably aromatic hydrocarbon rings, and particularly preferably benzene rings.

A preferable specific example of the repeating unit represented by the general formula (6-2) includes one represented by the following general formula (6-7).

[Chemical formula 40]

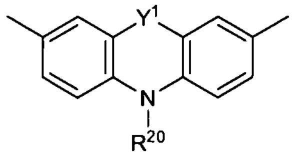

(6-7)

In the general formula (6-7), examples of each of $Y^1$ and $R^{20}$ include those described above.

Among the compounds of the present invention, from the viewpoints of charge transporting property when formed into a thin film, and device properties such as luminous efficiency and lifetime when used for an organic electroluminescence device, a conjugated polymer is preferable. Here, the conjugated polymer means a polymer in which delocalized n electron pairs exist along the main-chain skeleton of the polymer. As the delocalized electron, an unpaired electon or a lone electron pair may join to the resonance instead of a double bond.

Moreover, in the compound of the present invention, the repeating unit may be linked in non-conjugated units, or non-conjugated portions may be contained in each repeating unit, such that the luminescence property and the charge transporting property may not be deteriorated. Examples of the non-conjugated bond structure include structures represented by general formulae shown below and combinations of two or more of the structures represented by the general formulae shown below. Note that, in the formulae shown below, R's each independently represent any one of a hydrogen atom, halogen atom, alkyl group, alkoxy group, alkylthio group, aryl group, aryloxy group, arylthio group, arylalkyl group, arylalkyloxy group, arylalkylthio group, alkenyl group, alkynyl group, heteroaryloxy group, and heteroarylthio group. Moreover, Ar represents any one of an aromatic hydrocarbon ring and heterocyclic ring.

[Chemical formula 41]

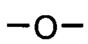 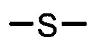 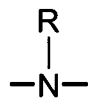 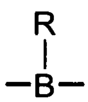 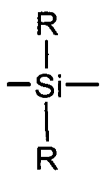 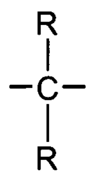

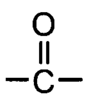 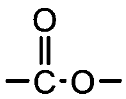 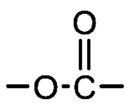 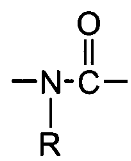 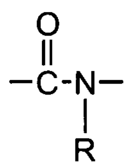

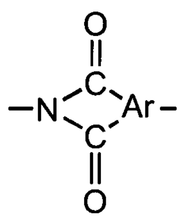 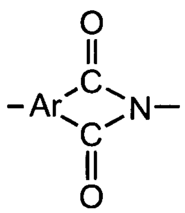 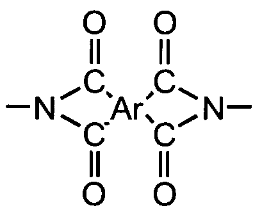

Moreover, the compound of the present invention may be an alternating, random, block or graft copolymer, or a polymer having an intermediate structure thereof, for example a random copolymer having block property. From the viewpoint of obtaining a polymeric luminous substance having a high fluorescent or phosphorescent quantum yield, a random copolymer having block property and a block or graft copolymer are preferred to a complete random copolymer. Furthermore, as the compound of the present invention, also included are a dendrimer and a compound having a branched main chain and more than three terminals.

Moreover, from the viewpoint of chromaticity, it is preferable that the compound of the present invention have a non-conjugated main chain, because the chromaticity for blue color is higher.

Furthermore, from the viewpoints of device properties such as luminous efficiency and lifetime when used in an organic electroluminescence device, the compound of the present invention preferably comprises a structural unit including the structure represented by the general formula (1) from 0. 1 mol% to 50 mol% both inclusive, and more preferably from 1 mol% to 20 mol% both inclusive, relative to the total structural unit.

Moreover, from the viewpoints of charge transporting and injecting properties, it is preferable to comprise the structural unit including the structure represented by the general formula (1) from 10 mol% to 100 mol% both inclusive, and more preferably from 30 mol% to 70 mol% both inclusive, relative to the total structural unit.

Moreover, from the viewpoints of device properties such as charge transporting property and lifetime when used in an organic electroluminescence device, the compound of the present invention preferably comprises the repeating unit represented by the general formula (5) from 1 mol% to 99 mol% both inclusive, and more preferably from 50 mol% to 97 mol% both inclusive, relative to the total structural unit.

Furthermore, from the viewpoints of device properties such as charge transporting property and adjustment of luminous color when used in an organic electroluminescence device, when the compound of the present invention comprises at least one of the repeating unit represented by the general formula (6-1), the repeating unit represented by the general formula (6-2) and the repeating unit represented by the general formula (6-3), it is preferable to comprise such a repeating unit from 0.01 mol% to 50 mol% both inclusive, and more preferably from 0.1 mol% to 30 mol% both inclusive, relative to the total structural unit.

Moreover, from the viewpoints of film formability and device properties such as luminous efficiency and lifetime when used in an organic electroluminescence device, the compound of the present invention has a polystyrene-equivalent number average molecular weight of preferably 2000 or more, more preferably $2\times10^3$ to $10^8$, and particularly preferably $1\times10^4$ to $10^6$. Note that, in the present description, a compound having a polystyrene-equivalent number average molecular weight of 2000 or more refers to a high-molecular-weight compound occasionally (hereinafter, among the compounds of the present invention, one having a polystyrene-equivalent number average molecular weight of 2000 or more may be referred to as a "polymer compound of the present invention"). On the other hand, a compound formed of a single constituent refers to a low-molecular-weight compound occasionally (generally, having a number average molecular weight of below 2000). Moreover, the compound of the present invention may comprise an intermediate structure between the low-molecular-weight compound and the high-molecular-weight compound, such as that of a dendrimer or oligomer. From the viewpoint of easiness of synthesis, when the compound of the present invention is a low-molecular-weight compound, the compound of the present invention is preferably a compound represented by the following general formula (4-2).

[Chemical formula 42]

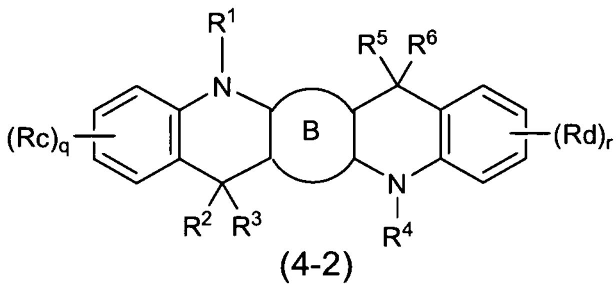

(4-2)

In the general formula (4-2), examples of each of $R^1$, $R^2$, $R^3$, $R^4$, $R^5$ and $R^6$ include those described above. Furthermore, examples of each ring B include those described above. Rc and Rd each independently represent any one of a halogen atom, alkyl group, alkoxy group, alkylthio group, aryl group, aryloxy group, arylthio group, arylalkyl group, arylalkoxy group, arylalkylthio group, alkenyl group, alkynyl group, disubstituted amino group, trisubstituted silyl group, acyl group, acyloxy group, imine residue, amide group, acid imide group, monovalent heterocyclic group, substituted carboxyl group, heteroaryloxy group, and heteroarylthio group. q and r each represent an integer of 0 to 4. When q is 2 or more, a plurality of Rc's may be the same as or different from each other. From the viewpoint of easiness of synthesizing the compound, q and r are preferably 0. Among such rings B, from the viewpoints of heat resistance, fluorescence intensity, device properties, and the like, preferable are aromatic hydrocarbon rings; more preferable are a benzene ring, naphthalene ring, anthracene ring, and phenanthrene ring; and particularly preferable is a benzene ring.

Among the compounds to be represented by the general formula (4-2), from the viewpoint of easiness of

synthesis, preferable is a compound represented by the following general formula (4-3).

[Chemical formula 43]

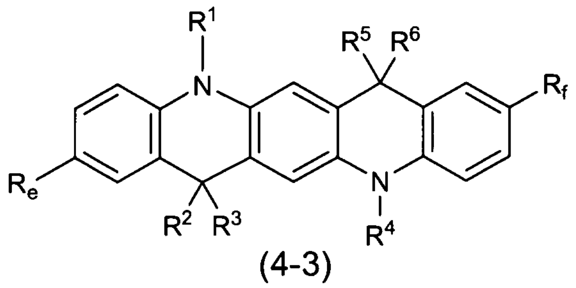

(4-3)

In the general formula (4-3), examples of each of $R^1$, $R^2$, $R^3$, $R^4$, $R^5$ and $R^6$ include those described above. Re and Rf each independently represent any one of a hydrogen atom, halogen atom, alkyl group, alkoxy group, alkylthio group, aryl group, aryloxy group, arylthio group, arylalkyl group, arylalkoxy group, arylalkylthio group, alkenyl group, alkynyl group, disubstituted amino group, trisubstituted silyl group, acyl group, acyloxy group, imine residue, amide group, acid imide group, monovalent heterocyclic group, substituted carboxyl group, heteroaryloxy group, and heteroarylthio group.

Next, a method for producing the compound of the present invention will be described.

The method for producing the compound of the present invention is a method comprising a step of: polymerizinga compound represented by the following general formula (7) as a raw material. Specifically, a compound represented by the general formula (3) can be produced by polymerizing, as a raw matrial, a compound represented by the following general formula (7).

[Chemical formula 44]

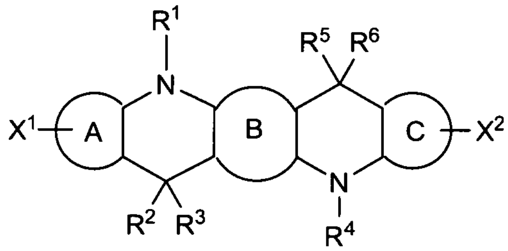

(7)

In the general formula (7), a ring A, a ring B and a ring C each independently represent any one of a monocyclic aromatic ring and fused aromatic ring; $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; and $X^1$ and $X^2$ each independently represent a substituent capable of participating in polymerization. Additionally, examples of each of the ring A, the ring B, the ring C, $R^1$, $R^2$, $R^3$, $R^4$, $R^5$ and $R^6$ include those described above.

Moreover, when the compound of the present invention (particularly, the polymer compound) comprises a repeating unit other than the repeating unit represented by the general formula (3), it should let a monomer, which is one to form the repeating unit other than the repeating unit represented by the general formula (3), coexist.

The polymer compound of the present invention can be produced through polymerization by using, as a raw material, a monomer having a substituent capable of participating in polymerization (polymerization active group). The polymerization active group used here varies depending on the polymerization method. Examples thereof include a formyl group, phosphonium group, halogen atoms such as bromine, iodine and chlorine, vinyl group, halomethyl group, acetonitrile group, alkylsulfonyloxy groups such as a trifluoromethanesulfonyloxy group, and arylsulfonyloxy groups such as a toluenesulfonyloxy group. From the viewpoints of molecular weight control, copolymer ratio control, and the like, it

is preferable that the number of the polymerization active group be 2.

As the method for producing the polymer compound of the present invention, when a main chain thereof has a vinylene group, a method described in, for example, Japanese Unexamined Patent Application Publication No. JP 05-202355 A can be used for the production with a monomer having a polymerization active group derived from a triplet light emitting complex and, if necessary, another monomer. Specifically, exemplified are [1] polymerization by the Wittig reaction of a compound having an aldehyde group with a compound having a phosphonium base, [2] polymerization by the Wittig reaction of a compound having an aldehyde group and a phosphonium base, [3] polymerization by the Heck reaction of a compound having a vinyl group with a compound having a halogen atom, [4] polymerization by the Heck reaction of a compound having a vinyl group and a halogen atom, [5] polymerization by the Horner-Wadsworth-Emmons method of a compound having an aldehyde group with a compound having an alkylphosphonate group, [6] polymerization by the Horner-Wadsworth-Emmons method of a compound having an aldehyde group and an alkylphosphonate group, [7] polycondensation by a dehydrohalogenation method of a compound having two or more halogenated methyl groups, [8] polycondensation by a sulfonium-salt decomposition method of a compound having two or more sulfonium bases, [9] polymerization by the Knoevenagel reaction of a compound having an aldehyde group with a compound having an acetonitrile group, [10] a method such as polymerization by the Knoevenagel reaction of a compound having an aldehyde group and an acetonitrile group, and [11] a method such as polymerization by the McMurry reaction of a compound having two or more aldehyde groups. The aforementioned polymerization methods [1] to [11] are shown by reaction equations below.

[Chemical formula 45]

[1]

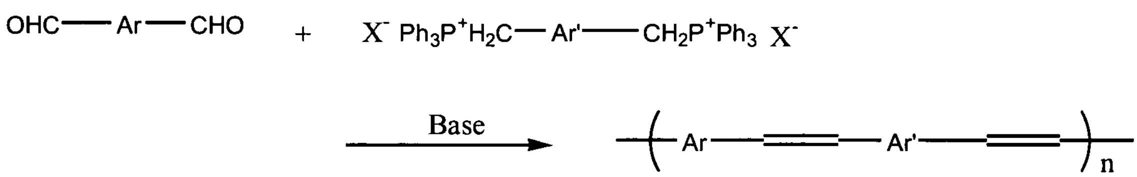


[Chemical formula 46]

[2]

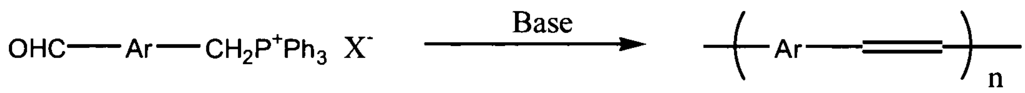


[Chemical formula 47]

[3]

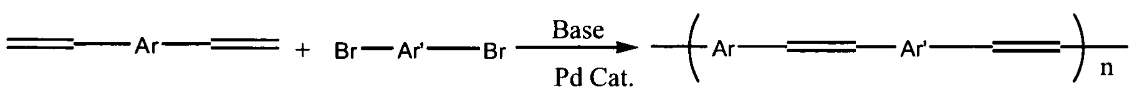

[Chemical formula 48]

〔4〕

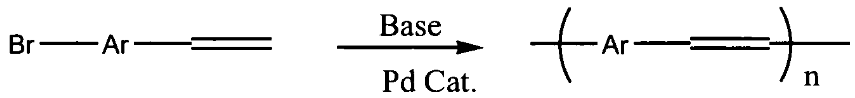


[Chemical formula 49]

〔5〕

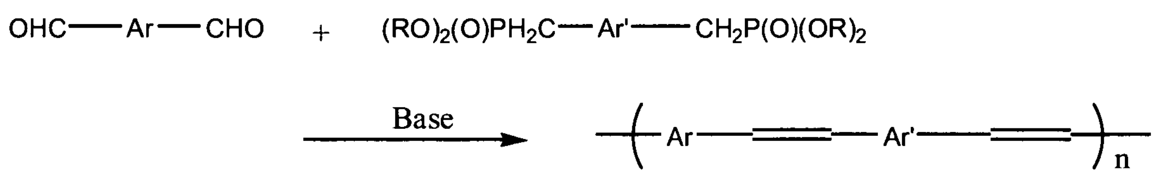


[Chemical formula 50]

〔6〕

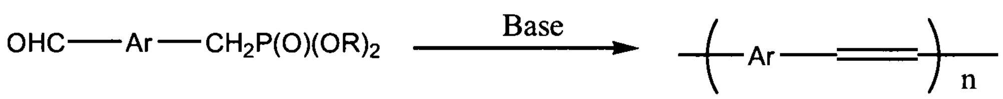


[Chemical formula 51]

〔7〕

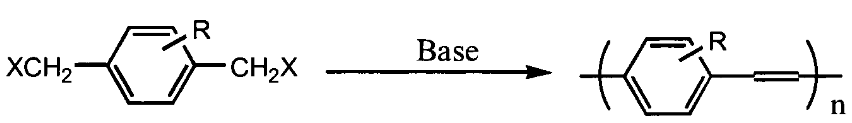

[Chemical formula 52]

〔8〕

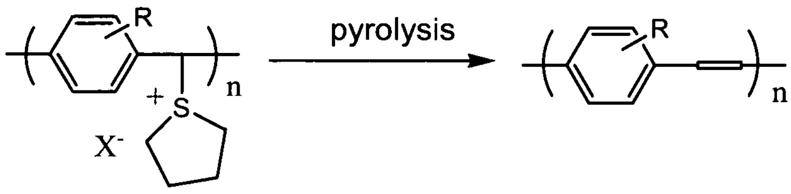


[Chemical formula 53]

〔9〕

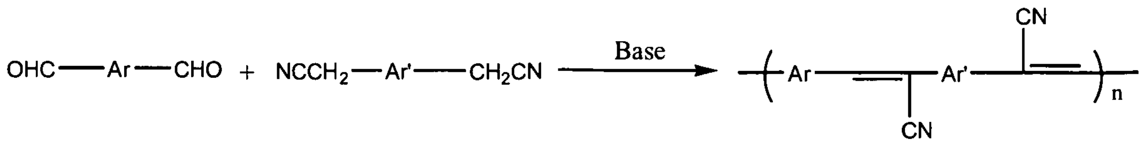


[Chemical formula 54]

〔10〕

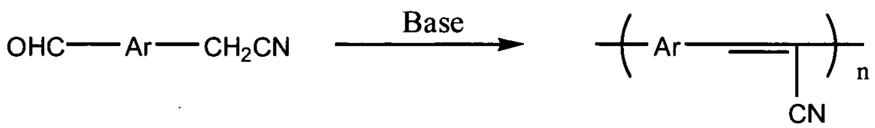


[Chemical formula 55]

〔11〕

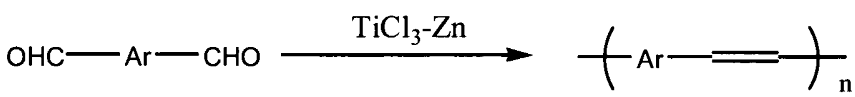


Meanwhile, when the main chain does not have a vinylene group, in the method for producing the polymer compound of the present invention, the polymer can be produced by performing polymerization using a monomer having a polymerization active group and, if necessary, another monomer. For example, exemplified is [12] a method of polymerization by the Suzuki coupling reaction, [13] a method of polymerization by the Grignard reaction, [14] a method of polymerization by the Stille coupling reaction, [15] a method of polymerization with a Ni(0) catalyst, [16] a method of

polymerization with an oxidizing agent such as $FeCl_3$/method of electrochemical oxidation polymerization, or [17] a method of decomposition of an intermediate polymer having an appropriate leaving group. The aforementioned polymerization methods [12] to [17] are shown by reaction scheme below.

[Chemical formula 56]

[12]

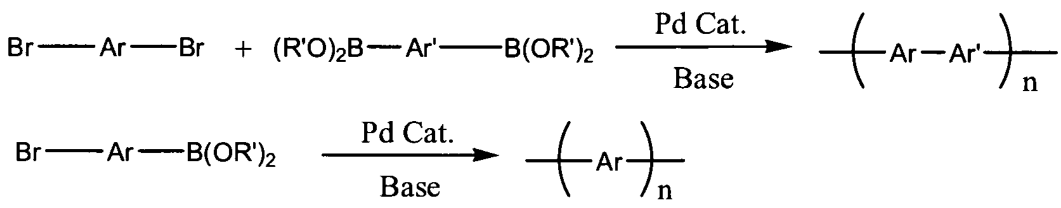


R'=H, alkyl, aryl

[Chemical formula 57]

[13]

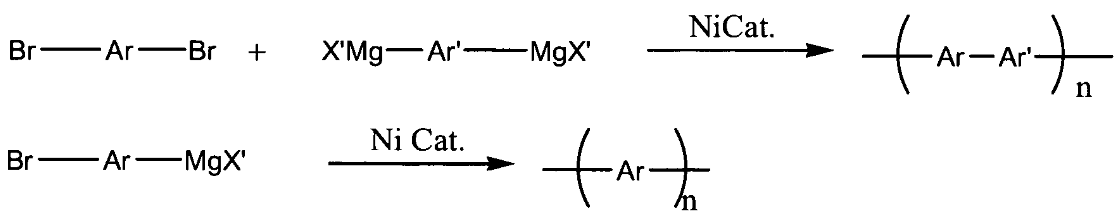


X'=Cl, Br, I

[Chemical formula 58]

[14]

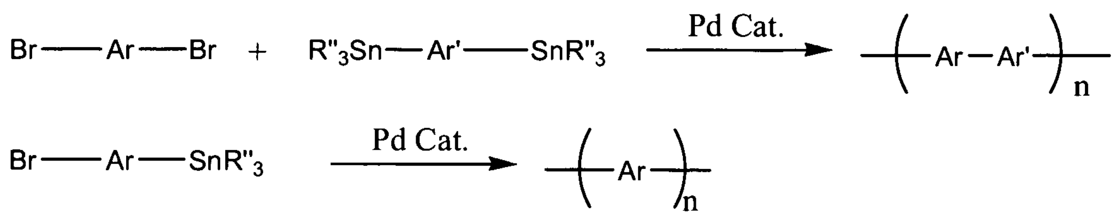


R"= alkyl, aryl, aryl alkyl

[Chemical formula 59]

〔15〕

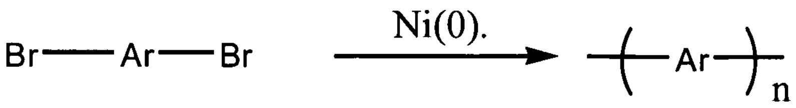


[Chemical formula 60]

〔16〕

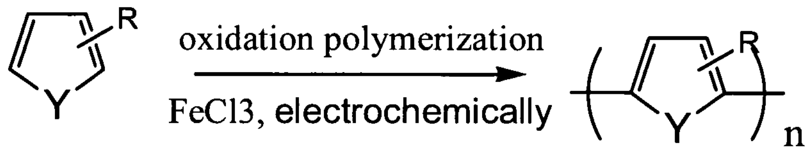


Y= S, NH

[Chemical formula 61]

〔17〕

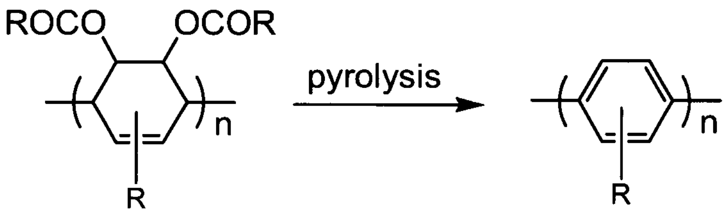


Among these polymerization methods, the polymerization by the Wittig reaction, the polymerization by the Heck reaction, the polymerization by the Horner-Wadsworth-Emmons method, the polymerization by the Knoevenagel reaction, and the method of polymerization by the Suzuki coupling reaction, the method of polymerization by the Grignard reaction, the method using the Stille coupling and the method of polymerization with a Ni(0) catalyst are preferable because of easiness of controlling the structure. Furthermore, the method of polymerization by the Suzuki coupling reaction, the method of polymerization by the Grignard reaction, and the method of polymerization with a Ni(0) catalyst are preferable because of availability of raw materials and easiness of polymerization reaction operation.

In the method for producing the polymer compound of the present invention, a monomer is dissolved in an organic solvent if necessary, and can be reacted using, for example, an alkali or suitable catalyst at a temperature from the melting point to the boiling point both inclusive of the organic solvent. As such a reaction method, it is possible to use known methods described in, for example, "Organic Reactions", vol. 14, pp. 270-490, John Wiley & Sons, Inc.) 1965, "Organic Reactions", vol. 27, pp. 345-390, John Wiley & Sons, Inc.), 1982, "Organic Syntheses", Collective Volume VI, pp. 407-411, John Wiley & Sons, Inc.), 1988, Chemical Review (Chem. Rev.), vol. 95, p. 2457 (1995), Journal of Organometallic Chemistry (J. Organomet. Chem.), vol. 576, p. 147 (1999), Journal of Practical Chemistry (J. Prakt. Chem.), vol. 336, p. 247 (1994), and Macromolecular Chemistry, Macromolecular Symposium (Makromol. Chem., Macromol. Symp.), vol. 12, p. 229 (1987).

The organic solvent varies depending on a compound and reaction to be used. However, it is preferable that

the solvent to be used be subjected to a deoxygenation treatment sufficiently and the reaction be progressed under an inert atmosphere, for generally suppressing a side reaction. Moreover, it is preferable to conduct a dehydration treatment likewise. (However, this is not the only case for a reaction in a two-phase system with water such as the Suzuki coupling reaction.)

For the reaction, an alkali or suitable catalyst is added as appropriate. These should be selected according to the reaction to be used. Here, it is preferable that the alkali or catalyst be sufficiently dissolved in the solvent used for the reaction. As a method for mixing the alkali or catalyst, exemplified is a method in which a solution of the alkali or catalyst is slowly added to a reaction solution with stiring under an inert atmosphere such as argon or nitrogen, or conversely in which a reaction solution is slowly added to a solution of the alkali or catalyst.

When the polymer compound of the present invention is used as a light-emitting material for an organic electroluminescence device, the purity thereof influences the luminescence property. Thus, a monomer before polymerization is preferably purified by a method such as distillation, sublimation purification, or recrystallization and then polymerized. Moreover, after the synthesis, a purification treatment such as re-precipitation purification or chromatographic separation is preferably conducted.

In the method for producing the polymer compound of the present invention, each monomer may be mixed together to perform the reaction or, if necessary, mixed separately.

The reaction conditions in the method for producing the polymer compound of the present invention will be described. In the cases of the Wittig reaction, Horner reaction, Knoevengel reaction, and the like, the reaction is conducted using an alkali in at least an equivalent amount, and preferably 1 to 3 equivalent amounts, to a functional group of the monomer. As the alkali, without particular limitation, it is possible to use, for example: metal alcoholates such as pottasium-t-butoxide, sodium-t-butoxide, sodium ethylate, and lithium methylate; hydride reagents such as sodium hydride; and amides such as sodium amide. As the solvent, N,N-dimethylformamide, tetrahydrofuran, dioxane, toluene, or the like is used. The reaction temperature is usually from about room temperature to 150°C, at which the reaction can be progressed. The reaction time is, for example, 5 minutes to 40 hours. However, it is only necessary to set the reaction time for the polymerization to progress sufficiently. Meanwhile, it is not necessary to leave the resultant for a long time after the reaction is complete. For this reason, the reaction time is preferably from 10 minutes to 24 hours. In the reaction, if the concentration is too low, the efficiency of the reaction is lowered, whereas, if the concentration is too high, the control of the reaction becomes difficult. Thus, the concentration should be appropriately selected in the range of about 0.01% by weight to the maximum dissolvable concentration, and usually in the range of 0.1% by weight to 20% by weight. In the case of the Heck reaction, the monomer is reacted in the presence of a base such as triethylamine using a palladium catalyst. Using a solvent having a comparatively high boiling point, such as N,N-dimethylformamide or N-methylpyrrolidone, the reaction temperature is from about 80 to 160°C, and the reaction time is from about 1 hour to 100 hours.

In the case of the Suzuki coupling reaction, as the catalyst, for example, palladium[tetrakis(triphenylphosphine)], palladium acetate, or the like is used. An inorganic base such as potassium carbonate, sodium carbonate and barium hydroxide, an organic base such as triethylamine, and inorganic salt such as cesium fluoride are added in at least an equivalent amount, and preferably 1 to 10 equivalent amounts, to the monomer for the reaction. The reaction may be conducted in a two-phase system using an inorganic salt as an aqueous solution. Examples of the solvent include N, N-dimethylformamide, toluene, dimethoxy ethane, tetrahydrofuran, and the like. Although depending on the solvent, the temperature about 50 to 160°C is preferably used. The temperature may be raised to near the boiling point of the solvent, followed by refluxing. The reaction time is from about 1 hour to 200 hours. Moreover, when a monomer having two groups selected from the group consisting of $-B(OH)_2$ and boronic acid ester is polymerized with a monomer having two groups selected from the group consisting of a halogen atom, alkyl sulfonate group, aryl sulfonate group and arylalkyl sulfonate group, the molecular weight can be increased by adding a monomer after the polymerization stops. Thus, it is easy to control the molecular weight. The molecular weight can be predicted from the following formula, and the amount of a monomer, which is added for adjusting the molecular weight to a targeted molecular weight, is determined by this formula.

$$Mn = Fw * Pn$$

Mn: number average molecular weight
Fw: average molecular weight of repeating unit
Pn: average degree of polymerization

$$Pn = 1/(1 - p + \alpha)$$

p: (number of hands reacted)/(total number of hands present before reaction)
$\alpha$: correction value based on experiment.

In the case of the Grignard reaction, the following method is exemplified. Specifically, a Grignard reagent solution is prepared by reacting a halogenated compound with metal Mg in an ether-based solvent such as tetrahydrofuran, diethyl ether, and dimethoxyethane. This solution is mixed with a separately prepared monomer solution. After a nickel catalyst or palladium catalyst is added thereto cautiously about excessive reaction, the Grignard reaction is conducted with raising the temperature under reflux. The Grignard reagent is used in at least an equivalent amount, preferably 1 to 1.5 equivalent amounts, and more preferably 1 to 1.2 equivalent amounts, to the monomer. When the polymerization is carried out by a method other than these, the reaction can also be conducted according to a known method.
Examples of a method where the reaction is conducted in the presence of the nickel catalyst include a method for polymerizing with a nickel (0) catalyst described above. Examples of the nickel catalyst include an ethylene bis (triphenylphosphine)nickel complex, tetrakis(triphenylphosphine)nickel complex, and bis(cyclooctadienyl)nickel complex.
In a case where the reaction is conducted in the presence of the palladium catalyst, an example of the method of polymerization includes the aforementioned Suzuki coupling reaction. Examples of the palladium catalyst include palladium acetate, a palladium[tetrakis(triphenylphosphine)] complex, bis(tricyclohexylphosphine)palladium complex, dichlorobis(triphenylphosphine)palladium complex, and the like.
The compound represented by the general formula (7) as has been described above is useful as a raw material for polymerizing the compound of the present invention (particularly, the polymer compound).
The present invention is to provide: compounds represented by the following formulae (8) to (10), each of which is useful as a photoelectric material for an organic transistor, organic electroluminescence device, and the like, or useful an intermediate of the photoelectric material therefor; and methods for synthesizing the compounds.
Among such compounds represented by the general formula (7), from the viewpoint of easiness of synthesis, a compound represented by the following general formula (8) is preferable.

[Chemical formula 62]

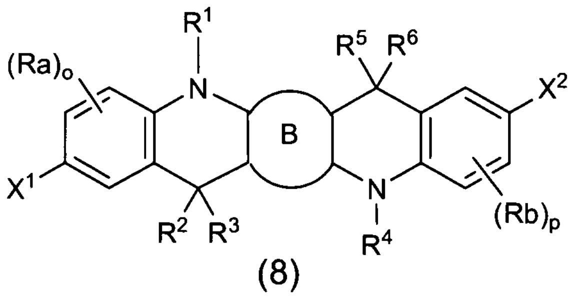

(8)

In the general formula (8), a ring B represents any one of a monocyclic aromatic ring and fused aromatic ring; $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; and $X^1$ and $X^2$ each independently represent a substituent capable of participating in polymerization. Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3 , a plurality of Rb' s may be the same or different. Morover, examples of each of the ring B, $R^1$, $R^2$, $R^3$, $R^4$, $R^5$, $R^6$, $X^1$ and $X^2$ include those described above. In Ra and Rb, examples of the alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group include the same groups as those exemplified as the substituent that the ring A, the ring B and the ring C may have. Additionally, from the viewpoint of easiness of synthesis of the compound, it is preferable that o and p be 0.
A compound represented by the following general formula (8-1) is further preferable.

[Chemical formula 63]

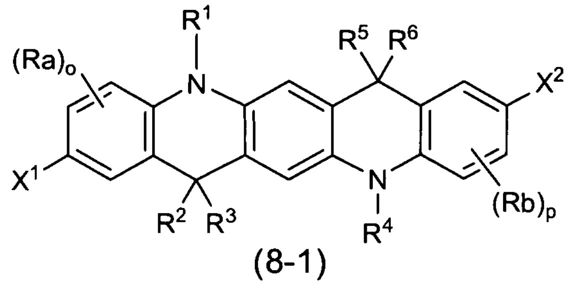

(8-1)

In the general formula (8-1), $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; and $X^1$ and $X^2$ each independently represent a substituent capable of participating in polymerization. Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3, a plurality of Rb's may be the same or different. Moreover, examples of each of $R^1$, $R^2$, $R^3$, $R^4$, $R^5$, $R^6$, Ra, Rb, $X^1$, $X^2$ o and p include those described above.

Above all, a preferable method is: (i) a method adopting the Suzuki coupling with a boronic acid residue or boronic acid ester residue and halogen atom, alkyl sulfonate group, aryl sulfonate group, or arylalkyl sulfonate group in the presence of a palladium catalyst and base; (ii) a method adopting the Yamamoto polymerization for coupling a halogen atom, alkyl sulfonate group, aryl sulfonate group, or arylalkyl sulfonate group in the presence of a nickel (0) catalyst; (iii) a method adopting the Stille coupling with a stannyl group and a halogen atom, alkyl sulfonate group, aryl sulfonate group, or arylalkyl sulfonate group in the presence of a palladium catalyst; or (iv) the Grignard coupling method for coupling halogenated magnesium to a halogen atom, alkyl sulfonate group, aryl sulfonate group, or arylalkyl sulfonate group in the presence of a nickel catalyst. This is because such methods are high in reaction yield, making it easy to obtain a polymer compound having a high molecular weight. In addition, when copolymerization is to be carried out, a copolymer can be obtained in accordance with the monomer feed ratio. Thus, the reaction is easily controllable. Among these methods, from the viewpoint of safety of a reagent, more preferable are the Suzuki polymerization method and the Yamamoto polymerization method. Moreover, from the viewpoint of reactivity, among the halogen atoms, alkyl sulfonate group, aryl sulfonate group, and arylalkyl sulfonate group, which serve as the substituent capable of participating in polymerization (polymerization active group), preferable are a chlorine atom, bromine atom, and iodine atom, and particularly preferable is a bromine atom.

The compound represented by the general formula (8) can be synthesized by performing a functional group transformation of $X^3$ and $X^4$ in a compound represented by the following general formula (9).

[Chemical formula 64]

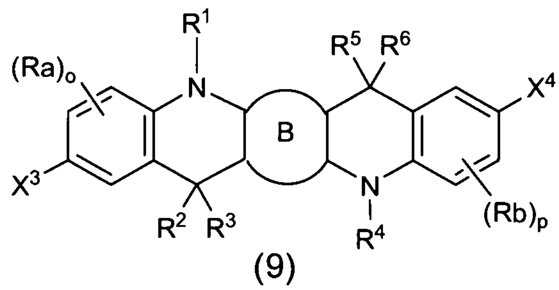

(9)

In the general formula (9), a ring B represents any one of a monocyclic aromatic ring and fused aromatic ring; $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; and $X^3$ and $X^4$ each

independently represent any one of a chlorine atom, bromine atom, and iodine atom. Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3, a plurality of Rb' s may be the same or different. Moreover, examples of each of the ring B, $R^1$, $R^2$, $R^3$, $R^4$, $R^5$, $R^6$, Ra and Rb include those described above. Furthermore, from the viewpoint of easiness of synthesis of the compound, it is preferable that o and p be 0.

A compound represented by the following general formula (9-1) is further preferable.

[Chemical formula 65]

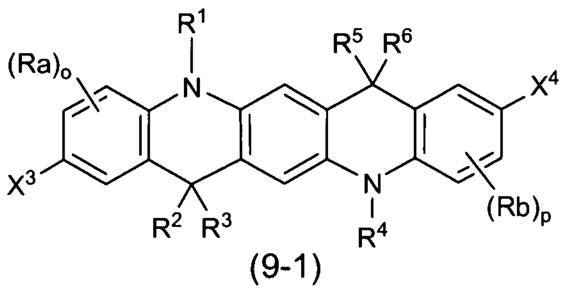

(9-1)

In the general formula (9-1), $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; and $X^3$ and $X^4$ each independently represent any one of a chlorine atom, bromine atom, and iodine atom. Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3, a plurality of Rb's may be the same or different. Moreover, examples of each of $R^1$, $R^2$, $R^3$, $R^4$, $R^5$, $R^6$, Ra, Rb, $X^3$, $X^4$, o and p include those described above.

When $X^1$ and/or $X^2$ in the general formula (8) are a boronic acid ester residue, the boronic acid ester residue can be synthesized by replacing $X^3$ and/or $X^4$ with a Grignard reagent or lithium, and then reacting those with a boronic acid ester. Examples of such a boronic acid ester include trimethyl borate, triisopropyl borate, 2-isopropoxy-4,4,5,5-tetramethyl-1,3,2-dioxaborolane, and the like. Alternatively, the boronic acid ester residue can be synthesized by reaction with diborate in the presence of a palladium catalyst and base, as described in J. Org. Chem., 60 (23), 7508 (1995). Examples of the diborate include bis(pinacolate)diboron, bis(catecholate)diboron, bis(neopentyl glycolate)diborane, bis (trimethylene glycolate)diboron, and the like.

Moreover, when $X^1$ and/or $X^2$ in the general formula (8) are $-B(OH)_2$, $-B(OH)_2$ can be synthesized by a method for hydrolyzing the aforementioned boronic acid ester in the presence of an acid or base.

Furthermore, when $X^1$ and/or $X^2$ in the general formula (8) are halogenated magnesium, the halogenated magnesium can be synthezised in the presence of magnesium.

Alternatively, when $X^1$ and/or $X^2$ in the general formula (8) are halogenated magnesium, the halogenated magnesium can be synthesized by replacing $X^3$ and/or $X^4$ with a Grignard reagent or lithium, and then reacting those with a trialkyltin chloride. Examples of the trialkyltin chloride include trimethyltin chloride, tri-n-butyltin chloride, and the like.

Furthermore, when $X^1$ and/or $X^2$ in the general formula (8) are any one of alkyl sulfonate group, aryl sulfonate group, and arylalkyl sulfonate group, such a group can be synthesized by transforming $X^3$ and/or $X^4$ into a hydroxyl group, and then reacting those with corresponding sulfonic anhydride or sulfonyl chloride in the presence of a base. Examples of the sulfonic anhydride include methanesulfonic anhydride, trifluoromethanesulfonic anhydride, benzenesulfonic anhydride, and the like. Moreover, examples of the sulfonyl chloride include methanesulfonyl chloride, trifluoromethanesulfonyl chloride, benzenesulfonyl chloride, and the like.

Meanwhile, a method for transforming $X^3$ and/or $X^4$ in the general formula (9) into a hydroxyl group is a method in which the compound can be synthesized by using peroxide, and by oxidizing a compound which is obtained as described above and in which $X^1$ and/or $X^2$ are $-B(OH)_2$. Examples of the peroxide include hydrogen peroxide, m-chlorobenzene perbenzoic acid, t-butylhydroperoxide, and the like.

The compound represented by the general formula (9) can be synthesized by halogenating a compound represented by the following general formula (10) in presence of a halogenating agent. By selecting suitable reaction conditions, the compound represented by the formula (9) can be synethsized with a very good selectively.

[Chemical formula 66]

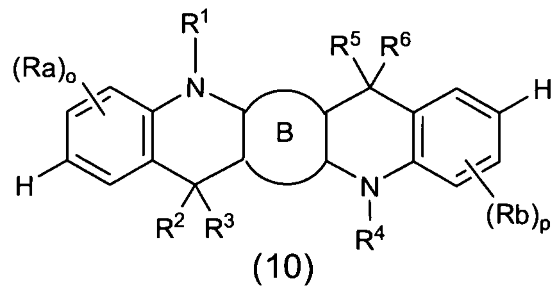

(10)

In the general formula (10), a ring B represents any one of a monocyclic aromatic ring and fused aromatic ring; $R^1$ and $R^4$ each independently represent a monovalent group; and $R^2$, $R^3$, $R^5$ and $R^6$ each independently represent any one of a hydrogen atom, alkyl group, aryl group, arylalkyl group, alkenyl group, and alkynyl group. Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3 , a plurality of Rb' s may be the same or different. Moreover, examples of each of the ring B, $R^1$, $R^2$, $R^3$, $R^4$, $R^5$, $R^6$, Ra and Rb include those described above. Furthermore, from the viewpoint of easiness of synthesis of the compound, it is preferable that o and p be 0.

A compound represented by the following general formula (10-1) is further preferable.

[Chemical formula 67]

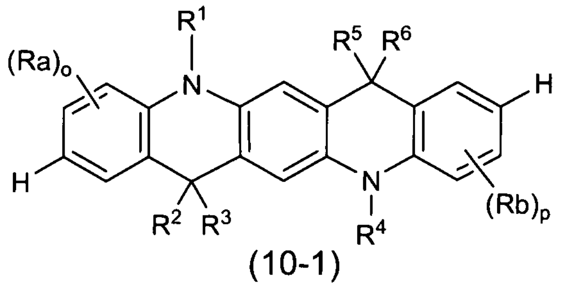

(10-1)

In the general formula (10-1), $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; and Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3, a plurality of Rb's may be the same or different. Moreover, examples of each of $R^1$, $R^2$, $R^3$, $R^4$, $R^5$, $R^6$, Ra, Rb, o and p include those described above.

Here, examples of the halogenating agent include: N-halogeno compounds such as N-chlorosuccinimide, N-chlorophthalimide, N-chlorodiethylamine, N-chlorodibutylamine, N-chlorocyclohexylamine, N-bromosuccinimide, N-bromophthalimide, N-bromoditrifluoromethylamine, N-iodosuccinimide, and N-iodophthalimide; halogen elements such as fluorine, chlorine, and bromine; and benzyltrimethylammonium tribromide. Among these, N-halogeno compounds are preferable.

Examples of the solvent to be used for the reaction include: saturated hydrocarbons such as pentane, hexane, heptane, octane, and cyclohexane; unsaturated hydrocarbons such as benzene, toluene, ethylbenzene, and xylene;

saturated halogenated hydrocarbons such as carbon tetrachloride, chloroform, dichloromethane, chlorobutane, bromobutane, chloropentane, bromopentane, chlorohexane, bromohexane, chlorocyclohexane, and bromocyclohexane; unsaturated halogenated hydrocarbons such as chlorobenzene, dichlorobenzene, and trichlorobenzene; alcohols such as methanol, ethanol, propanol, isopropanol, butanol, and t-butyl alcohol; carboxylic acids such as formic acid, acetic acid, and propionic acid; ethers such as dimethyl ether, diethyl ether, methyl-t-butyl ether, tetrahydrofuran, tetrahydropyran, and dioxane; amines such as trimethylamine, triethylamine, N,N,N',N'-tetramethylethylenediamine, and pyridine; and amides such as N,N-dimethylformamide, N,N-dimethylacetamide, N,N-diethylacetamide, N-methylmorpholine oxide, and N-methyl-2-pyrrolidone. These solvents can be used alone or in combination of two or more kinds.

The reaction temperature is from about -100°C to the boiling point of the solvent, and preferably from -20°C to 50°C.

The compound represented by the general formula (10) can be produced by performing a substitution reaction on a nitrogen atom in a compound represented by the following general formula (11) in presence of a base. For example, when $R^1$ and/or $R^4$ are an alkyl group, the compound can be produced by performing a nucleophilic substitution reaction to a halogenated alkyl in the presence of a base. Meanwhile, when $R^1$ and/or $R^4$ are an aromatic group (i.e., an aryl group or monovalent aromatic heterocyclic group, hereinafter the same), the compound can be produced in the Ullmann coupling condition for reacting an aromatic iodide in the presence of a copper catalyst and base. Alternatively, the compound can also be produced by reacting a palladium catalyst and a base with a halogenated aromatic compound, as described in Angewandte Chemie, International Edition in English, (1995), 34(12), 1348.

[Chemical formula 68]

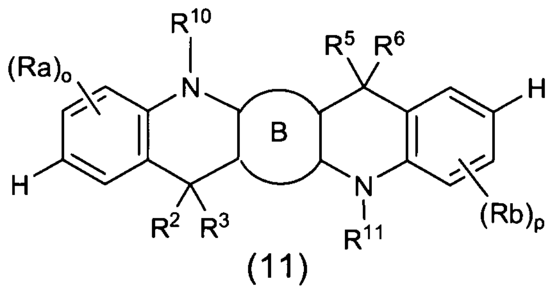

(11)

In the general formula (11), a ring B represents any one of a monocyclic aromatic ring and fused aromatic ring; and $R^2$, $R^3$, $R^5$ and $R^6$ each independently represent any one of a hydrogen atom, alkyl group, aryl group, arylalkyl group, alkenyl group, and alkynyl group; and $R^{10}$ and $R^{11}$ each independently represent any one of a hydrogen atom and monovalent group. Moreover, at least one of $R^{10}$ and $R^{11}$ is a hydrogen atom. Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3, a plurality of Rb's may be the same or different. Furthermore, examples of each of $R^2$, $R^3$, $R^5$, $R^6$, Ra and Rb include those described above. Additionally, from the viewpoint of easiness of synthesis of the compound, it is preferable that o and p be 0.

A compound represented by the following general formula (11-1) is further preferable.

[Chemical formula 69]

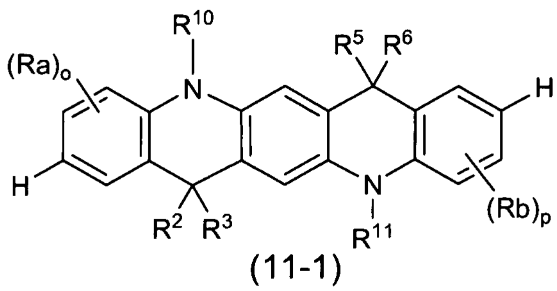

(11-1)

In the general formula (11-1), $R^2$, $R^3$, $R^5$ and $R^6$ each independently represent any one of a hydrogen atom, alkyl group, aryl group, arylalkyl group, alkenyl group, and alkynyl group; and $R^{10}$ and $R^{11}$ each independently represent any one of a hydrogen atom and monovalent group. Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3, a plurality of Rb' s may be the same or different. Moreover, examples of each of $R^2$, $R^3$, $R^5$, $R^6$, $R^{10}$, $R^{11}$, Ra, Rb, o and p include those described above.

The compound represented by the general formula (10) or (11) can be produced by cyclizing a compound represented by the following general formula (12) in presence of an acid.

[Chemical formula 70]

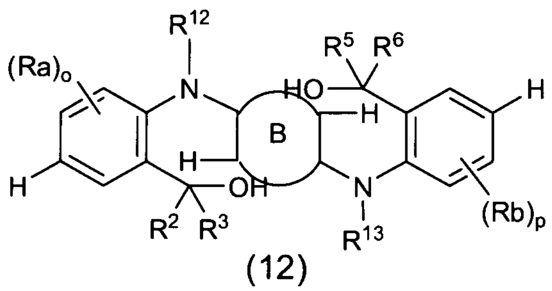

(12)

In the general formula (12), a ring B represents any one of a monocyclic aromatic ring and fused aromatic ring; $R^2$, $R^3$, $R^5$ and $R^6$ each independently represent any one of a hydrogen atom, alkyl group, aryl group, arylalkyl group, alkenyl group, and alkynyl group; and $R^{12}$ and $R^{13}$ each independently represent any one of a hydrogen atom and monovalent group. Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3, a plurality of Rb's may be the same or different. Moreover, examples of each of the ring B, $R^2$, $R^3$, $R^5$, $R^6$, Ra and Rb include those described above. Furthermore, from the viewpoint of easiness of synthesis of the compound, it is preferable that o and p be 0.

Such an acid may be a proton acid or may be a Lewis acid. Examples of the proton acid include: sulfonic acids such as methanesulfonic acid, trifluoromethanesulfonic acid, and p-toluenesulfonic acid; carboxylic acids such as formic acid, acetic acid, trifluoroacetic acid, and propionic acid; and inorganic acid such as sulfuric acid, hydrochloric acid, nitric acid, and phosphoric acid. Among these proton acids, preferable are strong inorganic acids such as hydrochloric acid, sulfuric acid, and nitric acid. Menawhile, examples of the Lewis acid include: boron halide such as boron tribromide, boron trichloride, and a boron trifluoride ether complex; and halogenated metals such as aluminium chloride, titanium chloride, manganese chloride, iron chloride, cobalt chloride, copper chloride, zinc chloride, aluminium bromide, titanium bromide, manganese bromide, iron bromide, cobalt bromide, copper bromide, and zinc bromide. Among these Lewis acids, preferable is triphenylmethyl tetrafluoroborate. These Lewis acids can be used alone or in combination of two or more kinds.

A medium for the reaction may be the aforementioned acid, but other solvents may be used. Examples of the solvent to be used include: saturated hydrocarbons such as pentane, hexane, heptane, octane, and cyclohexane; saturated halogenated hydrocarbons such as carbon tetrachloride, chloroform, dichloromethane, chlorobutane, bromobutane, chloropentane, bromopentane, chlorohexane, bromohexane, chlorocyclohexane, and bromocyclohexane; unsaturated halogenated hydrocarbons such as chlorobenzene, dichlorobenzene, and trichlorobenzene; and nitrated compounds such as nitromethane and nitrobenzene. These solvents can be used alone or in combination of two or more kinds.

The reaction temperature is from about -50°C to the boiling point of the solvent, and preferably from 0 to 100°C.

Note that, when at least one of $R^{12}$ and $R^{13}$ in the general formula (10) is an aromatic group, the yield is lowered due to a side reaction. For this reason, the following method is preferably used. Specifically, a compound in which $R^{12}$ and/or $R^{13}$ are a hydrogen atom is used to produce a compound represented by the general formula (11), and then an aromatic group is transformed onto a nitrogen atom.

Among the compounds represented by the formula (12), from the viewpoint of easiness of synthesis, a compound

represented by the following general formula (12-1) is preferable.

[Chemical formula 71]

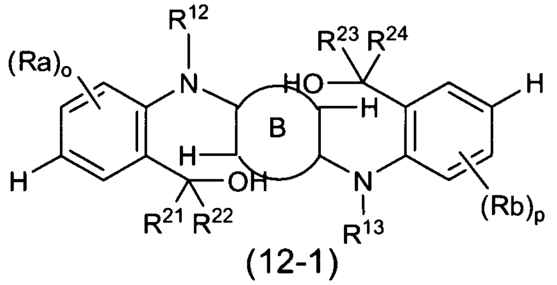

(12-1)

In the general formula (12-1), a ring B represents any one of a monocyclic aromatic ring and fused aromatic ring; and $R^{12}$ and $R^{13}$ each independently represent any one of a hydrogen atom and monovalent group. $R^{21}$, $R^{22}$, $R^{23}$ and $R^{24}$ each independently represent any one of a hydrogen atom, alkyl group, aryl group, arylalkyl group, alkenyl group, and alkynyl group; and at least one of $R^{21}$, $R^{22}$, $R^{23}$ and $R^{24}$ represents an aryl group. Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3, a plurality of Rb's may be the same or different. Moreover, examples of each of the ring B, $R^{12}$, $R^{13}$, Ra, Rb, o and p include those described above. In $R^{21}$, $R^{22}$, $R^{23}$ and $R^{24}$, examples of the alkyl group, aryl group, arylalkyl group, alkenyl group, or alkynyl group include the same groups as those exemplified for $R^2$, $R^3$ , $R^5$ and $R^6$.

The compound represented by the general formula (12-1) can be produced by performing a nucleophilic reaction on a compound represented by the following general formula (13) with a compound represented by a general formula: $R^{14}$-M (in the formula, $R^{14}$ represents any one of alkyl group, aryl group, arylalkyl group, alkenyl group, and alkynyl group; and M represents any one of lithium and halogenated magnesium).

[Chemical formula 72]

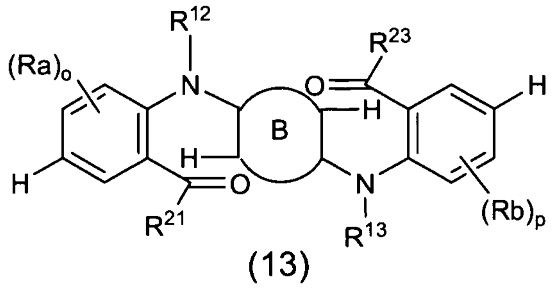

(13)

In the general formula (13), a ring B represents any one of a monocyclic aromatic ring and fused aromatic ring; $R^{21}$ and $R^{23}$ each independently represent any one of a hydrogen atom, alkyl group, aryl group, arylalkyl group, alkenyl group, and alkynyl group; and $R^{12}$ and $R^{13}$ each independently represent any one of a hydrogen atom and monovalent group. Ra and Rb each independently represent any one of alkyl group, an alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. At least one of $R^{21}$, $R^{23}$ and $R^{14}$ represents an aryl group. When o is 2 or 3, a plurality of Ra's may be the same or different. When p is 2 or 3, a plurality of Rb's may be the same or different. Moreover, examples of each of the ring B, $R^{12}$, $R^{13}$, Ra and Rb include those described above. Furthermore, examples of the alkyl group represented by $R^{14}$ include the same groups as those exemplified as the groups represented by $R^2$, $R^3$, $R^5$ and $R^6$. Examples of the

aryl group, arylalkyl group, alkenyl group, or alkynyl group, which is represented by $R^{14}$, include the same groups as those exemplified as the substituent that the ring A, the ring B and the ring C may have. Additionally, from the viewpoint of easiness of synthesis of the compound, it is preferable that o and p be 0.

The equivalent amount of the compound represented by the general formula: $R^{14}$-M, which is to be used, is preferably 4 or more equivalent amounts, when both of $R^{12}$ and $R^{13}$ in the general formula (13) are a hydrogen atom. Meanwhile, when any one of $R^{12}$ and $R^{13}$ is a hydrogen atom, 3 or more equivalent amounts are preferable. Furthermore, when both of $R^{12}$ and $R^{13}$ are not a hydrogen atom, 2 or more equivalent amounts are preferable.

Alternatively, the compound represented by the general formula (12-1) can be produced by performing a nucleophilic reaction on a compound represented by the following general formula (14) with a compound represented by a general formula: $R^{15}$-M (in the formula, $R^{15}$ represents any one of alkyl group, aryl group, arylalkyl group, alkenyl group, and alkynyl group; and M represents any one of lithium and halogenated magnesium).

[Chemical formula 73]

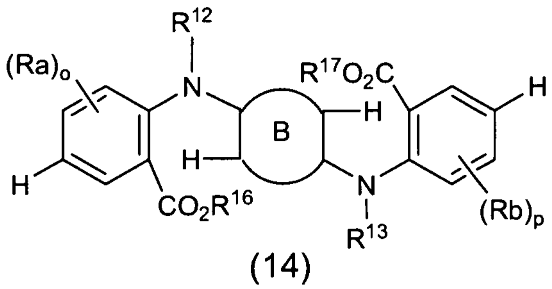

(14)

In the general formula (14), a ring B represents any one of a monocyclic aromatic ring and fused aromatic ring; $R^{12}$ and $R^{13}$ each independently represent any one of a hydrogen atom and monovalent group; and $R^{16}$ and $R^{17}$ each independently represent any one of an alkyl group, aryl group, and arylalkyl group. Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group. o and p each independently represent an integer of 0 to 3. When o is 2 or 3, a plurality of Ra's may be the same or different. When p is 2 or 3, a plurality of Rb's may be the same or different. Moreover, examples of each of the ring B, $R^{12}$, $R^{13}$, Ra and Rb include those described above. Furthermore, examples of the alkyl group, aryl group, or arylalkyl group, which is represented by $R^{16}$ and $R^{17}$, include the same groups as those exemplified as the substituent that the ring A, ring B and the ring C may have. Additionally, from the viewpoint of easiness of synthesis of the compound, it is preferable that o and p be 0.

The equivalent amount of the compound represented by the general formula: $R^{15}$-M, which is to be used, is preferably 6 or more equivalent amounts, when both $R^{12}$ and $R^{13}$ are a hydrogen atom. Meanwhile, when any one of $R^{12}$ and $R^{13}$ is a hydrogen atom, 5 or more equivalent amounts are preferable. When both $R^{12}$ and $R^{13}$ are not a hydrogen atom, 4 or more equivalent amounts are preferable.

Both of the reaction for producing the compound represented by the general formula (12-1) from the compound represented by the general formula (13) and the reaction for producing the compound represented by the general formula (12-1) from the compound represented by the general formula (14) are preferably conducted under an atmosphere of an inert gas such as argon or nitrogen.

Examples of the solvent used for the reaction include: saturated hydrocarbons such as pentane, hexane, heptane, octane, and cyclohexane; unsaturated hydrocarbons such as benzene, toluene, ethylbenzene, and xylene; and ethers such as dimethyl ether, diethyl ether, methyl-t-butyl ether, tetrahydrofuran, tetrahydropyran, and dioxane. These solvents can be used alone or in combination of two or more kinds.

The reaction temperature is from about -100°C to the boiling point of the solvent, and preferably from -80°C to room temperature.

Next, description will be given of a method for producing a compound represented by the following general formula (14-1), which can be favorably used from the viewpoint of easiness of synthesis of the compound, among the compounds represented by the general formula (14).

[Chemical formula 74]

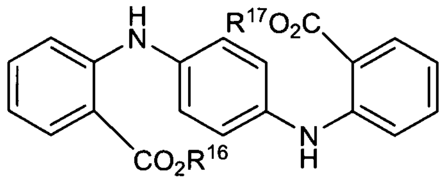

(14-1)

In the general formula (14-1), $R^{16}$ and $R^{17}$ each independently represent any one of alkyl group, aryl group, and arylalkyl group. The compound represented by the general formula (14-1) can be produced by performing a condensation reaction on a compound represented by the following general formula (15), a compound represented by the following general formula (16), and a compound represented by the following general formula (17), in presence of a catalyst including at least one metal selected from the group consisting of palladium, nickel, and copper.

[Chemical formula 75]

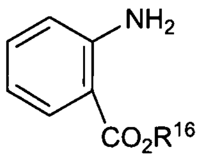

(15)

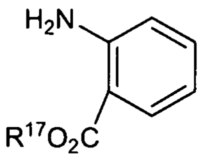

(16)

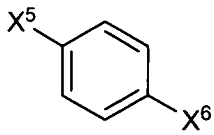

(17)

In the general formulae (15) to (17), $R^{16}$ and $R^{17}$ each independently represent any one of alkyl group, aryl group, and arylalkyl group; and $X^5$ and $X^6$ each independently represent any one of a chlorine atom, bromine atom, iodine atom, alkyl sulfonate group, aryl sulfonate group, and arylalkyl sulfonate group. Moreover, examples of each of $R^{16}$ and $R^{17}$ include those described above.

As the reaction condition, the compound can be produced in the Ullmann coupling condition for reacting an aromatic iodide in the presence of a copper catalyst and base. Alternatively, the compound can also be produced by reacting a palladium catalyst and base with a halogenated aromatic compound, as described in Angewandte Chemie, International Edition in English, (1995), 34(12), 1348.

From the viewpoint of easiness of synthesis, it is preferable that the compound represented by the general formula (15) and the compound represented by the general formula (16) be identical.

Alternatively, the compound represented by the general formula (14-1) can also be produced by performing a condensation reaction on a compound represented by the following general formula (18), a compound represented by the following general formula (19), and a compound represented by the following general formula (20) in presence of a catalyst including at least one metal selected from the group consisting of palladium, nickel, and copper.

[Chemical formula 76]

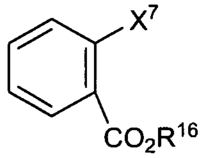

(18)

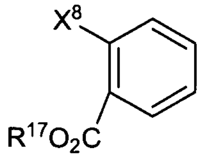

(19)

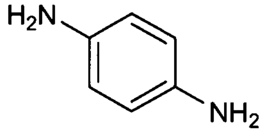

(20)

In the general formulae (18) to (20), $R^{16}$ and $R^{17}$ each independently represent any one of alkyl group, aryl group, and arylalkyl group; and $X^7$ and $X^8$ each independently represent any one of a chlorine atom, bromine atom, iodine atom, alkyl sulfonate group, aryl sulfonate group, and arylalkyl sulfonate group. Moreover, examples of each of $R^{16}$ and $R^{17}$ include those described above.

As the reaction condition, the compound can be produced in the Ullmann coupling condition for reacting an aromatic iodide in the presence of a copper catalyst and base. Alternatively, the compound can also be produced by reacting a palladium catalyst and base with a halogenated aromatic compound, as described in Angewandte Chemie, International Edition in English, (1995), 34(12), 1348.

From the viewpoint of easiness of synthesis, it is preferable that the compound represented by the general formula (18) and the compound represented by the general formula (19) be identical.

Next, a composition comprising the compound of the present invention will be described. The composition comprises: at least one material selected from the group consisting of a hole transporting material, an electron transporting material, and a light-emitting material; and the compound of the present invention. The composition can be used as a light-emitting material and a charge transporting material.

In the composition of the present invention, the content ratio of at least one material selected from the group consisting of a hole transporting material, an electron transporting material, and a light-emitting material to the compound of the present invention should be determined according to usage. Meanwhile, two or more kinds of the compound of the present invention can be mixed and used as the composition.

Moreover, using the composition of the present invention, a light-emitting layer of an organic electroluminescence device can be formed. The optimum value of the film thickness of such a light-emitting layer varies depending on the material to be used. The film thickness should be selected so as to give the driving voltage and luminous efficiency the optimum values. However, the film thickness is, for example, from 1 nm to 1 $\mu$m, preferably 2 nm to 500 nm, and further preferably 5 nm to 200 nm.

As a method for forming such a light-emitting layer, exemplified is a method in which a film is formed from a solution. As the film formation method from a solution, it is possible to use application methods such as a spin coating method, casting method, micro gravure coating method, gravure coating method, bar coating method, roll coating method, wire bar coating method, dip coating method, spray coating method, screen printing method, flexo printing method, offset printing method, and inkjet printing method. In views of easiness of pattern formation and multicolor separate application, printing methods such as a screen printing method, flexo printing method, offset printing method and inkjet printing method are preferable.

An ink composition (solution) used in a printing method or the like may only comprise at least one kind of the compound of the present invention, or may comprise a hole transporting material, an electron transporting material, a light-emitting material, a solvent, and an additive such as a stabilizer in addition to the compound of the present invention.

The ratio of the compound of the present invention in such an ink composition is usually from 20% by weight to 100% by weight, and preferably from 40% by weight to 100% by weight, based on the total weight of the composition excepting the solvent. Meanwhile, when the solvent is included in such an ink composition, the ratio of the solvent is from 1% by weight to 99.9% by weight, preferably 60% by weight to 99.5% by weight, and further preferably 80% by weight to 99.0% by weight, based on the total weight of the composition. Note that, although the viscosity of the ink composition varies depending on the printing method, the viscosity at 25°C is preferably in the range of 1 to 20 mPa·s for preventing clogging and flight bending during ejection in a case of adopting the inkjet printing method or the like in which the ink composition passes through an ejecting apparatus.

The ink composition (solution) of the present invention may comprise an additive for adjusting the viscosity and/or surface tension, in addition to the compound of the present invention. As such an additive, a polymer compound (thickening agent) having a high molecular weight for increasing the viscosity, a poor solvent, a compound having a low molecular weight for lowering the viscosity, a surfactant for lowering the surface tension, and the like may be used in combination as appropriate.

Such a polymer compound having a high molecular weight for increasing the viscosity should be soluble to the same solvent as the compound of the present invention, and should not disturb light emission and charge transportation. For example, high-molecular-weight polystyrene, polymethyl methacrylate, a compound of the present invention having a high molecular weight, or the like can be used. Moreover, the polystyrene-equivalent weight average molecular weight is preferably 500,000 or more, and more preferably 1,000,000 or more.

Moreover, the poor solvent can also be used as a thickening agent. Specifically, by adding a small amount of the poor solvent for a solid content in the solution, the viscosity can be increased. When such a poor solvent is added for this purpose, the kinds and amount of the solvent to be added should be selected such that the solid content in the solution may not be deposited. When the stability during storage is also taken into consideration, the amount of the poor solvent is preferably 50% by weight or less, and more preferably 30% by weight or less, based on the whole solution.

Moreover, the ink composition (solution) of the present invention may comprise, in addition to the compound of the present invention, an antioxidant for improving the storage stability. The antioxidant should be soluble to the same

solvent as the compound of the present invention, and should not disturb light emission and charge transportation. Examples thereof include phenol-based antioxidants, phosphorus-based antioxidants, and the like.

Furthermore, when such a solution is used as an ink composition, a solvent to be used is not particularly limited, but preferable is one that can dissolve or uniformly disperse a material other than the solvent constituting the ink composition. Examples of such a solvent include: chlorine-based solvents such as chloroform, methylene chloride, 1,2-dichloroethane, 1,1,2-trichloroethane, chlorobenzene, and o-dichlorobenzene; ether-based solvents such as tetrahydrofuran, dioxane, and anisole; aromatic hydrocarbon-based solvents such as toluene and xylene; aliphatic hydrocarbon-based solvents such as cyclohexane, methylcyclohexane, n-pentane, n-hexane, n-heptane, n-octane, n-nonane, and n-decane; ketone-based solvents such as acetone, methyl ethyl ketone, cyclohexanone, benzophenone, and acetophenone; ester-based solvents such as ethyl acetate, butyl acetate, ethyl cellosolve acetate, methyl benzoate, and phenyl acetate; polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, and 1,2-hexanediol, and derivatives thereof; alcohol-based solvents such as methanol, ethanol, propanol, isopropanol, and cyclohexanol; sulfoxide-based solvent such as dimethyl sulfoxide; and amide-based solvents such as N-methyl-2-pyrrolidone and N,N-dimethylformamide. Meanwhile, these solvents can be used alone or in combination of two or more kinds. Among these solvents, from the viewpoints of solubility of the polymer compound and the like, uniformity during film formation, viscosity property, etc, preferable are aromatic hydrocarbon-based solvents, aliphatic hydrocarbon-based solvents, ester-based solvents, and ketone-based solvent; and more preferable are toluene, xylene, ethylbenzene, diethylbenzene, trimethylbenzene, n-propylbenzene, i-propylbenzene, n-butylbenzene, i-butylbenzene, s-butylbenzene, anisole, ethoxybenzene, 1-methylnaphthalene, cyclohexane, cyclohexanone, cyclohexylbenzene, bicyclohexyl, cyclohexenyl cyclohexanone, n-heptylcyclohexane, n-hexylcyclohexane, 2-propylcyclohexanone, 2-heptanone, 3-heptanone, 4-heptanone, 2-octanone, 2-nonanone, 2-decanone, dicyclohexyl ketone, acetophenone, and benzophenone. Furthermore, from the viewpoint of film formability and from the viewpoionts of device properties, and the like, the number of kinds of the solvent in the solution is preferably two or more, more preferably 2 to 3, and particularly preferably 2.

Moreover, when two kinds of the solvent are contained in the solution, at least one kind of the solvent may be in a solid state at 25°C. From the viewpoint of film formability, one kind of the solvent is preferably a solvent having a boiling point of 180°C or higher, and more preferably a solvent having a boiling point of 200°C or higher. Meanwhile, from the viewpoint of viscosity, 1% by weight or more of the compound of the present invention is preferably dissolved in both of the two kinds of the solvent at 60°C, and 1% by weight or more of the compound of the present invention is preferably dissolved in one kind of the solvent among the two kinds of the solvent at 25°C.

Furthermore, from the viewpoints of viscosity and film formability, when two or more kinds of the solvent are contained in the solution, the content of a solvent having the highest boiling point is preferably 40 to 90% by weight, more preferably 50 to 90% by weight, and particularly preferably 65 to 85% by weight, based on the weight of all the solvents in the solution.

One kind or two or more kinds of the compound of the present invention may be contained in such a solution. A compound other than the compound of the present invention may be contained such that the device properties and the like may not be deteriorated.

The ink composition (solution) of the present invention may comprise water, a metal and a salt thereof in the range of 1 to 1000 ppm (weight basis). Examples of the metal include lithium, sodium, calcium, pottasium, iron, copper, nickel, aluminium, zinc, chromium, manganese, cobalt, platinum, and iridium. Moreover, the ink composition (solution) of the present invention may comprise silicon, phosphorus, fluorine, chlorine, and bromine in the range of 1 to 1000 ppm (weight basis).

Using the ink composition (solution) of the present invention, a thin film of the present invention can be formed by application methods such as a spin coating method, casting method, micro gravure coating method, gravure coating method, bar coating method, roll coating method, wire bar coating method, dip coating method, spray coating method, screen printing method, flexo printing method, offset printing method, and inkjet printing method. Among these application methods, a screen printing method, flexo printing method, offset printing method, and inkjet printing method are preferably used to form the film with the ink composition (solution) of the present invention, and an inkjet method is more preferably used to form the film.

The thin film comprising the compound of the present invention can be formed by using the ink composition (solution) of the present invention as described above. Examples of such a thin film include a light-emitting thin film, electric conductive thin film, and organic semiconductor thin film.

The electric conductive thin film of the present invention preferably has a surface resistance of 1 KΩ/□ or lower. Moreover, the electric conductivity can be increased by doping such a thin film with a Lewis acid, ionic compound, or the like. Furthermore, the surface resistance is more preferably 100 Ω/□ or lower, and particularly preferably 10 Ω/□.

In the organic semiconductor thin film of the present invention, the larger of the electron mobility and the hole mobility is preferably $10^{-5}$ $cm^2$/V/second or more, more preferably $10^{-3}$ $cm^2$/V/second or more, and particularly preferably

$10^{-1}$ $cm^2$/V/second or more.

Moreover, an organic transistor can be obtained by forming the organic semiconductor thin film of the present invention on a Si substrate having an insulating film such as $SiO_2$ and a gate electrode formed thereon, and then forming a source electrode and a drain electrode with Au and the like.

Meanwhile, examples of the organic electroluminescence device of the present invention include: an organic electroluminescence device having an electron transporting layer being located between a cathode and a light-emitting layer; an organic electroluminescence device having a hole transporting layer being located between an anode and a light-emitting layer; an organic electroluminescence device having an electron transporting layer being located between a cathode and a light-emitting layer, and a hole transporting layer between an anode and the light-emitting layer; organic electrolytic light-emitting devices that are the devices each further having an interlayer being located between the anode and the light-emitting layer; and the like.

As the structure of such an organic electroluminescence device, the following structures a) to d) are exemplified.

a) anode/light-emitting layer/cathode
b) anode/hole transporting layer/light-emitting layer/cathode
c) anode/light-emitting layer/electron transporting layer/cathode
d) anode/hole transporting layer/light-emitting layer/electron transporting layer/cathode

(here, / indicates that adjacent lamination of layers. Hereinafter the same.)
Moreover, these structures are also exemplified as structures each having an interlayer being located between a light-emitting layer and an anode and being adjacent to the light-emitting layer. Specifically,

a') anode/interlayer/light-emitting layer/cathode
b') anode/hole transporting layer/interlayer /light-emitting layer/cathode
c') anode/interlayer/light-emitting layer/electron transporting layer/cathode
d') anode/hole transporting layer/interlayer /light-emitting layer/electron transporting layer/cathode.

The interlayer may have at least one function of hole injection, hole transportation and electron block.

The compound of the present invention can be used singly or as a mixture component in all or part of the layers of the organic electroluminescence device. When the compound of the present invention is used in part of the layers, and when the compound is used as a mixture component, generally-available materials as described below can be used.

Examples of the light-emitting layer of the organic electroluminescence device of the present invention include ones formed using, as a polymeric material, a conjugated-type polymer compound such as polyfluorene derivatives, polyparaphenylene vinylene derivatives, polyphenylene derivatives, polyparaphenylene derivative, polythiophene derivatives, polydialkylfluorene, polyfluorenebenzothiadiazole, polyalkylthiophene, or polymer compound of the present invention.

Moreover, the light-emitting layers formed using these polymeric materials may contain: a high-molecular-weight pigment compound such as a perylene-based pigment, coumarin-based pigment, and rhodamine-based pigment; and a low-molecular-weight pigment compound such as rubrene, perylene, 9,10-diphenylanthracene, tetraphenylbutadiene, Nile red, coumarin 6, and quinacridone. Moreover, the light-emitting layers may contain naphthalene derivatives, anthracene or derivatives thereof, perylene or derivatives thereof, polymethine-based, xanthene-based, coumarin-based, cyanine-based, or other pigments, metal complexes of 8-hydroxyquinoline or derivatives thereof, aromatic amine, tetraphenylcyclopentadiene or derivatives thereof, tetraphenylbutadiene or derivatives thereof, or metal complexes that emit phosphorescent light, such as tris(2-phenylpyridine)iridium.

Meanwhile, the light-emitting layer that the light-emitting device of the present invention comprises may be formed of a mixture composition of a non-conjugated-type polymer compound [for example, polyvinyl carbazole, polyvinyl chloride, polycarbonate, polystyrene, polymethyl methacrylate, polybutyl methacrylate, polyester, polysulfone, polyphenylene oxide, polybutadiene, poly(N-vinylcarbazole), hydrocarbon resin, ketone resin, phenoxy resin, polyamide, ethyl cellulose, vinyl acetate, ABS resin, polyurethane, melamine resin, unsaturated polyester resin, alkyd resin, epoxy resin, silicone resin, carbazole derivatives, triazole derivatives, oxazole derivatives, oxadiazole derivatives, imidazole derivatives, polyarylalkane derivatives, pyrazoline derivatives, pyrazolone derivatives, phenylenediamine derivatives, arylamine derivatives, amino-substituted chalcone derivatives, styrylanthracene derivatives, fluorenone derivatives, hydrazone derivatives, stilbene derivatives, silazane derivatives, aromatic tertiary amine compounds, styrylamine compounds, aromatic dimethylidyne-based compounds, porphyrin-based compounds, polysilane-based compounds, poly (N-vinylcarbazole) derivatives, and polymer including organic silane derivatives] with a light-emitting organic compound such as the organic pigment and a metal complex.

As specific examples of such a polymer compound, exemplified are polyfluorene, derivatives and copolymers thereof, polyarylene, derivatives and copolymers thereof, polyarylenevinylene, derivatives and copolymers thereof, and

(co)polymers of aromatic amine and derivatives thereof, which are disclosed in International Publications Nos. WO 97/09394, WO 98/27136, WO 99/54385, WO 00/22027, WO 01/19834, GB 2340304 A, GB 2348316, US 573636, US 5741921, US 5777070, EP 0707020, Japanese Unexamined Patent Application Publications Nos. JP 09-111233 A, JP 10-324870 A, 2000-80167 A, 2001-123156 A, 2004-168999 A, 2007-162009 A, Development of Organic EL Device & Their Materials (published by CMC Publishing Co., Ltd. in 2006), and so on.

Meanwhile, as specific examples of the low-molecular-weight compound, exemplified are compounds descriebd in, for example, Japanese Unexamined Patent Application Publication No. JP 57-51781 A, Data book on work function of organic thin films [2nd ed.] (published by CMC Publishing Co., Ltd. in 2006), Development of Organic EL Device & Their Materials (published by CMC Publishing Co., Ltd. in 2006), and so on.

The materials may be a single component or a composition formed of multiple components. Moreover, the light-emitting layer may have a single-layer structure formed of one kind or two or more kinds of the materials, or may have a multi-layer structure formed of a single composition or different compositions.

The film formation method of the light-emitting layer is not limited, but examples thereof include the same film formation methods as for the hole injecting layer. Examples of the film formation method from a solution include the application methods and printing methods such as a spin coating method, casting method, bar coating method, slit coating method, spray coating method, nozzle coating method, gravure printing method, screen printing method, flexo printing method, and inkjet printing method. Examples when a material made of a sublimation compound is used include a vacuum vapor-deposition method, transfer method, and the like. Meanwhile, examples of a solvent used in the film formation method from a solution include the solvents listed for the film formation method of the hole injecting layer.

When an organic compound layer such as the electron transporting layer is to be formed by the application method after the light-emitting layer, if the lower layer is dissolved in a solvent contained in a solution of the layer to be then applied, the lower layer can be made insoluble in the solvent by the same method as that exemplified for the film formation method of the hole injecting layer.

The optimum value of the film thickness of the light-emitting layer varies depending on the material to be used. The film thickness should be selected so as to give the driving voltage and luminous efficiency appropriate values. Nevertheless, a certain thickness is needed so as not to cause a pin hole at least. Meanwhile, if the thickness is too large, the driving voltage of the device is unpreferably increased. Thus, the film thickness of the light-emitting layer is, for example, from 5 nm to 1 μm, preferably 10 nm to 500 nm, and further preferably 30 nm to 200 nm.

When the organic electroluminescence device of the present invention comprises the hole transporting layer, examples of a hole transporting material to be used include polyvinyl carbazole and derivatives thereof, polysilane and derivatives thereof, polysiloxane derivatives having an aromatic amine on a side chain or main chain, pyrazoline derivatives, arylamine derivatives, stilbene derivatives, triphenyldiamine derivatives, polyaniline and derivatives thereof, polythiophene and derivatives thereof, polypyrrole and derivatives thereof, poly(p-phenylene vinylene) and derivatives thereof, and poly(2,5-thienylene vinylene) and derivatives thereof.

Moreover, examples of such a hole transporting material include those described in, for example, Japanese Unexamined Patent Application Publications Nos. JP 63-70257 A, JP 63-175860 A, JP 02-135359 A, JP 02-135361 A, JP 02-209988 A, JP 03-37992 A, and JP 03-152184 A.

Among these, preferable as the hole transporting material used in the hole transporting layer are polymeric hole transporting materials such as polyvinyl carbazole and derivatives thereof, polysilane and derivatives thereof, polysiloxane derivatives having an aromatic amine compound group on a side chain or main chain, polyaniline and derivatives thereof, polythiophene and derivatives thereof, poly(p-phenylene vinylene) and derivatives thereof, and poly (2,5-thienylene vinylene) and derivatives thereof. More preferable are polyvinyl carbazole and derivatives thereof, polysilane and derivatives thereof, and polysiloxane derivatives having an aromatic amine on a side chain or main chain.

Menawhile, as the hole transporting material being made of low-molecular-weight compound, exemplified are pyrazoline derivatives, arylamine derivatives, stilbene derivatives, and triphenyldiamine derivatives. Note that, in a case where the hole transporting material is made of a low-molecular-weight compound, the compound is preferably dispersed in a polymer binder when used.

The polymer binder to be mixed with the low-molecular-weight compound as described above preferably does not disturb the charge transportation extremely, and one that does not strongly absorb visible light is favorably used. Examples of such a polymer binder include poly(N-vinylcarbazole), polyaniline and derivatives thereof, polythiophene and derivatives thereof, poly(p-phenylene vinylene) and derivatives thereof, poly(2,5-thienylene vinylene) and derivatives thereof, polycarbonate, polyacrylate, polymethylacrylate, polymethyl methacrylate, polystyrene, polyvinyl chloride, and polysiloxane.

Polyvinyl carbazole and derivatives thereof are obtained, for example, from a vinyl monomer by cation polymerization or radical polymerization.

Moreover, as polysilane and derivatives thereof, exemplified are compounds described in Chemical Review (Chem. Rev.), vol. 89, p. 1359 (1989), the description of GB Patent No. 2300196, and so on. As the synthesis method, methods described in these can be used, and particularly, the Kipping method is favorably used.

Furthermore, polysiloxane and derivatives thereof show little hole transporting property in the siloxane skeleton structures, and thus ones having a structure of the low-molecular-weight hole transporting material on a side chain or main chain are favorably used. Particularly, exemplified is one that has an aromatic amine showing hole transporting property on a side chain or main chain.

The film formation method of the hole transporting layer is not limited, but exemplified in the case of the low-molecular-weight hole transporting material, is a film formation method from a mixed solution with a polymer binder. Meanwhile, in the case of a high-molecular-weight hole transporting material, a film formation method from a solution is exemplified.

A solvent used in the film formation from a solution as described above is preferably one that can dissolve or uniformly disperse the hole transporting material. Examples of such a solvent include: chlorine-based solvents such as chloroform, methylene chloride, 1,2-dichloroethane, 1,1,2-trichloroethane, chlorobenzene, and o-dichlorobenzene; ether-based solvents such as tetrahydrofuran, and dioxane; aromatic hydrocarbon-based solvents such as toluene and xylene; aliphatic hydrocarbon-based solvents such as cyclohexane, methylcyclohexane, n-pentane, n-hexane, n-heptane, n-octane, n-nonane, and n-decane; ketone-based solvents such as acetone, methyl ethyl ketone, and cyclohexanone; ester-based solvents such as ethyl acetate, butyl acetate, and ethyl cellosolve acetate; polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, and 1,2-hexanediol, and derivatives thereof; alcohol-based solvents such as methanol, ethanol, propanol, isopropanol, and cyclohexanol; sulfoxide-based solvent such as dimethyl sulfoxide; and amide-based solvents such as N-methyl-2-pyrrolidone and N, N-dimethylformamide. Meanwhile, these solvents can be used alone or in combination of two or more kinds.

As the film formation method from a solution, it is possible to use application methods from a solution, such as a spin coating method, casting method, micro gravure coating method, gravure coating method, bar coating method, roll coating method, wire bar coating method, dip coating method, spray coating method, screen printing method, flexo printing method, offset printing method, and inkjet printing method.

The optimum value of the film thickness of the hole transporting layer varies depending on the material to be used. The film thickness should be selected so as to give the driving voltage and luminous efficiency appropriate values. Nevertheless, a certain thickness is needed so as not to cause a pin hole at least. Meanwhile, if the thickness is too large, the driving voltage of the device is unpreferably increased. Thus, the film thickness of such a hole transporting layer is, for example, from 1 nm to 1 μm, preferably 2 nm to 500 nm, and further preferably 5 nm to 200 nm.

When the organic electroluminescence device of the present invention comprises the electron transporting layer, a known material can be used as an electron transporting material to be used. Examples thereof include oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, metal complexes of 8-hydroxyquinoline and derivatives thereof, polyquinoline and derivatives thereof, polyquinoxaline and derivatives thereof, and polyfluorene and derivatives thereof.

Moreover, examples of such an electron transporting material include those described in, for example, JP 63-70257 A, JP 63-175860 A, JP 02-135359 A, JP02-135361 A, JP 02-209988 A, JP 03-37992 A, and JP 03-152184 A.

Among these, preferable are oxadiazole derivatives, benzoquinone and derivatives thereof, anthraquinone and derivatives thereof, metal complexes of 8-hydroxyquinoline and derivatives thereof, polyquinoline and derivatives thereof, polyquinoxaline and derivatives thereof, polyfluorene and derivatives thereof. More preferable are 2-(4-biphenylyl)-5-(4-t-butylphenyl)-1,3,4-oxadiazole, benzoquinone, anthraquinone, tris(8-quinolinol)aluminium, and polyquinoline.

The film formation method of the electron transporting layer is not particularly limited, but exemplified in the case of a low-molecular-weight electron transporting material is a vacuum vapor-deposition method from powder or a film formation method from a solution or melted condition. Meanwhile, in the case of a high-molecular-weight electron transporting material, a film formation method from a solution or melted condition is exemplified. When a film is formed from a solution or melted condition, the above-described polymer binder may be used together.

A solvent used in the film formation from a solution as described above is preferably one that can dissolve or uniformly disperse the electron transporting material and/or polymer binder. Examples of such a solvent include: chlorine-based solvents such as chloroform, methylene chloride, 1,2-dichloroethane, 1,1,2-trichloroethane, chlorobenzene, and o-dichlorobenzene; ether-based solvents such as tetrahydrofuran and dioxane; aromatic hydrocarbon-based solvents such as toluene and xylene; aliphatic hydrocarbon-based solvents such as cyclohexane, methylcyclohexane, n-pentane, n-hexane, n-heptane, n-octane, n-nonane, and n-decane; ketone-based solvents such as acetone, methyl ethyl ketone, and cyclohexanone; ester-based solvents such as ethyl acetate, butyl acetate, and ethyl cellosolve acetate; polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, and 1,2-hexanediol, and derivatives thereof; alcohol-based solvents such as methanol, ethanol, propanol, isopropanol, and cyclohexanol; sulfoxide-based solvent such as dimethyl sulfoxide; and amide-based solvents such as N-methyl-2-pyrrolidone and N,N-dimethylformamide. Meanwhile, these solvents can be used alone or in combination of two or more kinds.

As the film formation method from a solution or melted condition, it is possible to use application methods such as a spin coating method, casting method, micro gravure coating method, gravure coating method, bar coating method, roll coating method, wire bar coating method, dip coating method, spray coating method, screen printing method, flexo printing method, offset printing method, and inkjet printing method.

The optimum value of the film thickness of the electron transporting layer varies depending on the material to be used. The film thickness should be selected so as to give the driving voltage and luminous efficiency appropriate values. Nevertheless, a certain thickness is needed so as not to cause a pin hole at least. Meanwhile, if the thickness is too large, the driving voltage of the device is unpreferably increased. Thus, the film thickness of such an electron transporting layer is, for example, from 1 nm to 1 μm, preferably 2 nm to 500 nm, and further preferably 5 nm to 200 nm.

As a material used in the interlayer, exemplified are polymers containing an aromatic amine such as polyvinyl carbazole and derivatives thereof, polyarylene derivatives having an aromatic amine on a side chain or main chain, arylamine derivatives, and phenylenediamine derivatives.

The film formation method of such an interlayer is not limited, but exemplified in the case of using a polymeric material is, for example, a film formation method from a solution.

A solvent used in the film formation method from a solution as described above preferably one that can dissolve or uniformly disperse the hole transporting material. Examples of such a solvent include: chlorine-based solvents such as chloroform, methylene chloride, 1,2-dichloroethane, 1,1,2-trichloroethane, chlorobenzene, and o-dichlorobenzene; ether-based solvents such as tetrahydrofuran and dioxane; aromatic hydrocarbon-based solvents such as toluene and xylene; aliphatic hydrocarbon-based solvents such as cyclohexane, methylcyclohexane, n-pentane, n-hexane, n-heptane, n-octane, n-nonane, and n-decane; ketone-based solvents such as acetone, methyl ethyl ketone, and cyclohexanone; ester-based solvents such as ethyl acetate, butyl acetate, and ethyl cellosolve acetate; polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, and 1,2-hexanediol, and derivatives thereof; alcohol-based solvents such as methanol, ethanol, propanol, isopropanol, and cyclohexanol; sulfoxide-based solvent such as dimethyl sulfoxide; and amide-based solvents such as N-methyl-2-pyrrolidone and N, N-dimethylformamide. Meanwhile, these solvents can be used one kind alone or in combination of two or more kinds.

As the film formation method from a solution, it is possible to use application methods from a solution such as a spin coating method, casting method, micro gravure coating method, gravure coating method, bar coating method, roll coating method, wire bar coating method, dip coating method, spray coating method, screen printing method, flexo printing method, offset printing method, and inkjet printing method.

The optimum value of the film thickness of the interlayer varies depending on the material to be used. The film thickness should be selected so as to give the driving voltage and luminous efficiency appropriate values. The film thickness of the interlayer is, for example, from 1 nm to 1 μm, preferably 2 nm to 500 nm, and further preferably 5 nm to 200 nm.

When such an interlayer is placed adjacent to the light-emitting layer, particularly when both of the layers are formed by the application method, the materials of the two layers may be mixed, so that the device properties and the like are unpreferably influenced in some cases. Examples of a method for reducing the mixing of the materials of the two layers, when the interlayer is formed by the application method and then the light-emitting layer is formed by the application method, include: (i) a method in which an interlayer is formed by the application method, then this interlayer is insolubilized in an organic solvent by a treatment such as heating or a light irradiation, and subsequently a light-emitting layer is formed; (ii) a method in which a solvent used at the time of applying a light-emitting layer is a solvent having a low solubility to an interlayer; and the like. When the interlayer is insolubilized by heating, the heating temperature is usually from about 150°C to 300°C, and the heating time is usually from about 1 minute to 1 hour. In such a case, for removal of a component not insolubilized to a solvent by heating, it is preferable to rinse the interlayer to remove the component with a solvent to be used for forming the light-emitting layer after the heating and before the formation of the light-emitting layer. When the insolubilization in the solvent by heating is sufficiently performed, the rinsing with a solvent may be omitted. For sufficient insolubilization in the solvent by heating, it is preferable to use a compound containing at least one polymerizable group in the molecule, as a polymer compound to be used in the interlayer. Furthermore, the number of polymerizable groups is more preferably 5% or more based on the number of repeating units in the molecule. Examples of the polymerizable group include a group having a double bond, a cyclic ether group, and the like. Examples of the group having a double bond include a vinyl group, 1,3-butedienyl group, acrylate group, methacrylate group, and the like. Examples of the cyclic ether group include an epoxy group, oxetane group, and the like.

Additionally, among charge transporting layers placed adjacent to the electrodes, those having a function of improving the charge injecting efficiency from the electrode and having an effect of lowering the driving voltage of the device are, in particular, generally called charge injecting layers (hole injecting layer, electron injecting layer).

Furthermore, for improving close adherence to an electrode and improving charge injection from an electrode,

the aforementioned charge injecting layer or an insulating layer may be placed adjacent to the electrode. Moreover, for improving close adherence at an interface, preventing mixing, and the like, a thin buffer layer may be inserted into the interface of a charge transporting layer and a light-emitting layer. Furthermore, the order and the number of layers to be laminated and the thickness of each layer can be appropriately set in consideration of the luminous efficiency and the lifetime of the device.

In the present invention, examples of the organic electroluminescence device having the charge injecting layers (electron injecting layer, hole injecting layer) include an organic electroluminescence device having a charge injecting layer placed adjacent to a cathode, and an organic electroluminescence device having a charge injecting layer placed adjacent to an anode.

Examples of the structure of such an organic electroluminescence device include the following structures e) to p).

e) anode/charge injecting layer/light-emitting layer/cathode
f) anode/light-emitting layer/charge injecting layer/cathode
g) anode/charge injecting layer/light-emitting layer/charge injecting layer/cathode
h) anode/charge injecting layer/hole transporting layer /light-emitting layer/cathode
i) anode/hole transporting layer/light-emitting layer/charge injecting layer/cathode
j) anode/charge injecting layer/hole transporting layer/light-emitting layer/charge injecting layer/cathode
k) anode/charge injecting layer/light-emitting layer/electron transporting layer/cathode
1) anode/light-emitting layer/electron transporting layer/charge injecting layer/cathode
m) anode/charge injecting layer/light-emitting layer/electron transporting layer/charge injecting layer/cathode
n) anode/charge injecting layer/hole transporting layer/light-emitting layer/electron transporting layer/cathode
o) anode/hole transporting layer/light-emitting layer/electron transporting layer/charge injecting layer/cathode
p) anode/charge injecting layer/hole transporting layer/light-emitting layer/electron transporting layer/charge injecting layer/cathode

Moreover, these structures are also exemplified as structures having an interlayer being placed between a light-emitting layer and an anode and being adjacent to the light-emitting layer.

As the charge injecting layer, exemplified are a layer containing an electric conductive polymer, a layer located between an anode and a hole transporting layer and containing a material having an ionization potential of an intermediate value between an anode material and a hole transporting material contained in the hole transporting layer, a layer located between a cathode and an electron transporting layer and containing a material having an electron affinity of an intermediate value between a cathode material and an electron transporting material contained in the electron transporting layer, and the like.

When such a charge injecting layer is a layer containing an electric conductive polymer, the electric conductivity of this electric conductive polymer is preferably from $10^{-5}$ S/cm to $10^{3}$ S/cm both inclusive, more preferably from $10^{-5}$ S/cm to $10^{2}$ S/cm both inclusive, and particularly preferably $10^{-5}$ S/cm to $10^{1}$ S/cm both inclusive for decreasing the leak current between light emitting picture elements. Meanwhile, for controlling the electric conductivity of the electric conductive polymer to be from $10^{-5}$ S/cm to $10^{3}$ S/cm both inclusive, usually the electric conductive polymer is doped with an appropriate amount of ions.

The kind of the ions to be doped with is an anion in the case of the hole injecting layer, and is a cation in the case of the electron injecting layer. As examples of the anion, exemplified are a polystyrenesulfonic acid ion, alkylbenzenesulfonic acid ion, camphorsulfonic acid ion, and the like. As examples of the cation, exemplified are a lithium ion, sodium ion, pottasium ion, tetrabutylammonium ion, and the like.

The film thickness of such a charge injecting layer is, for example, from 1 nm to 100 nm, and preferably 2 nm to 50 nm.

A material used in the charge injecting layer should be appropriately selected according to a relation with materials of an electrode and adjacent layer. Examples thereof include polyaniline and derivatives thereof, polythiophene and derivatives thereof, polypyrrole and derivatives thereof, polyphenylene vinylene and derivatives thereof, polythienylene vinylene and derivatives thereof, polyquinoline and derivatives thereof, polyquinoxaline and derivatives thereof, electric conductive polymer such as a polymer having an aromatic amine structure on a main chain or side chain, metal phthalocyanines (copper phthalocyanine and the like), and carbon.

Moreover, the organic electroluminescence device of the present invention may further comprise an insulating layer to make charge injection easier. The film thickness of such an insulating layer is usually 2 nm or smaller. Examples of a material of such an insulating layer include a metal fluoride, metal oxide, organic insulating material, and the like. Examples of the organic electroluminescence device comprising such an insulating layer include an organic electroluminescence device in which the insulating layer is placed adjacent to a cathode, and an organic electroluminescence device in which the insulating layer is placed adjacent to an anode.

Examples of the structure of such an organic electroluminescence device include the following structures q)

to ab).

q) anode/insulating layer/light-emitting layer/cathode
r) anode/light-emitting layer/insulating layer/cathode
s) anode/insulating layer/light-emitting layer/insulating layer/cathode
t) anode/insulating layer/hole transporting layer/light-emitting layer/cathode
u) anode/hole transporting layer/light-emitting layer/insulating layer/cathode
v) anode/insulating layer/hole transporting layer/light-emitting layer/insulating layer/cathode
w) anode/insulating layer/light-emitting layer/electron transporting layer/cathode
x) anode/light-emitting layer/electron transporting layer/insulating layer/cathode
y) anode/insulating layer/light-emitting layer/electron transporting layer/insulating layer/cathode
z) anode/insulating layer/hole transporting layer/light-emitting layer/electron transporting layer/cathode
aa) anode/hole transporting layer/light-emitting layer/electron transporting layer/insulating layer/cathode
ab) anode/insulating layer/hole transporting layer/light-emitting layer/electron transporting layer/insulating layer/cathode

Moreover, these structures are also exemplified as structures having an interlayer being located between a light-emitting layer and an anode and being adjacent to the light-emitting layer.

A substrate for forming the organic electroluminescence device of the present invention should be a substrate which does not change, when an electrode and layers of organic substances are formed. Examples of such a substrate include substrates made of glass, plastic, polymer film, silicon, and the like. When the substrate is not transparent, it is preferable that the electrode opposite thereto be transparent or semi-transparent.

Moreover, usually at least one of the anode and the cathode that the organic electroluminescence device of the present invention comprises is transparent or semi-transparent. The anode side is preferably transparent or semi-transparent.

As a material for such an anode, an electric conductive metal oxide film, semi-transparent metal thin film, or the like is used. As the material for such an anode, specifically, used are: films (NESA or the like) formed using an electric conductive glass made of indium oxide, zinc oxide, tin oxide, and their composites, i . e. , indium·tin·oxide (ITO), indium·zinc·oxide, and the like; gold, platinum, silver, and copper; and the like. Preferable are ITO, indium·zinc·oxide, and tin oxide. Examples of the forming method include a vacuum vapor-deposition method, sputtering method, ion plating method, plating method, and the like. Moreover, as such an anode, an organic transparent electric conductive film such as polyaniline, derivatives thereof, polythiophene, or derivatives thereof may be used.

The film thickness of the anode can be appropriately selected in consideration of the light transmittance and electric conductivity. Nevertheless, the thicknes is, for example, from 10 nm to 10μm, preferably 20 nm to 1 μm, and further preferably 50 nm to 500 nm.

Moreover, for making charge injection easier, a layer made of a phthalocyanine derivative, electric conductive polymer, carbon, or the like, or a layer made of a metal oxide, metal fluoride, organic insulating material, or the like having an average film thickness of 2 nm or smaller may be formed on the anode.

A material for the cathode used in the organic electroluminescence device of the present invention is preferably a material having a small work function. Examples of such a material used for the cathode are: metals such as lithium, sodium, pottasium, rubidium, cesium, beryllium, magnesium, calcium, strontium, barium, aluminium, scandium, vanadium, zinc, yttrium, indium, cerium, samarium, europium, terbium, and ytterbium; alloys of two or more of them; alloys of at least one of them and at least one of gold, silver, platinum, copper, manganese, titanium, cobalt, nickel, tungsten, and tin; and graphite or graphite intercalation compounds. Examples of the alloy include a magnesium-silver alloy, magnesium-indium alloy, magnesium-aluminium alloy, indium-silver alloy, lithium-aluminium alloy, lithium-magnesium alloy, lithium-indium alloy, calcium-aluminium alloy, and the like. The cathode may have a laminated structure of two or more layers.

The film thickness of the cathode can be appropriately selected in consideration of the electric conductivity and durability. Nevertheless, the thickness is, for example, from 10 nm to 10 μm, preferably 20 nm to 1 μm, and further preferably 50 nm to 500 nm.

As the forming method of the cathode, used are a vacuum vapor-deposition method, sputtering method, laminating method in which a metal thin film is adhered by heat and pressure, and the like. Moreover, a layer made of an electric conductive polymer or a layer made of a metal oxide, metal fluoride, organic insulating material, or the like having an average film thickness of 2 nm or smaller may be located between the cathode and the organic substance layer. After the cathode is formed, a protective layer for protecting the organic electroluminescence device of the present invention may be mounted. Additionally, for long-term stable use of the organic electroluminescence device of the present invention, a protective layer and/or protective cover are preferably mounted to protect the device from the outside.

As such a protective layer, a polymer compound, metal oxide, metal fluoride, metal boride, or the like can be

used. Meanwhile, as the protective cover, a glass plate, plastic plate having a surface subjected to a low-water-permeation treatment, or the like can be used. A method in which the cover is pasted to a device substrate with a thermosetting resin or photo curable resin for hermetical sealing is favorably used. When a spacer is used to retain a space, it is easy to prevent the device from damage. Moreover, when this space is filled with an inert gas such as nitrogen or argon, the cathode can be prevented from oxidization. Furthermore, when a desiccant such as barium oxide is placed in this space, a damage on the device by moisture adsorbed in the production process is easily suppressed. Among these, at least one of the measures is preferably adopted.

The organic electroluminescence device of the present invention can be used as surface light sources, display devices such as a segment display device and a dot-matrix display device, and backlights of liquid crystal display devices.

In order to obtain light emission in a planar form using the organic electroluminescence device of the present invention, an anode and cathode in a planar form should be disposed so as to be superposed on each other. Moreover, to obtain light emission in a pattern form, there are: a method in which a mask having a window in a pattern form is disposed on the surface of the aforementioned planar light-emitting device; a method in which a non-light-emitting part of an organic substance layer is formed considerably thick so that substantially no light can be emitted; and a method in which any one of an anode and a cathode or both of the electrodes are formed in a pattern form. By forming a pattern by any of these methods and disposing some electrodes in a way that the electrodes can be turned On/OFF independently, a display device of segment type is obtained which can display numbers, letters, simple marks, and the like. Furthermore, to produce a dot-matrix device, both an anode and a cathode should be formed in a stripe form and disposed so as to cross each other. Partial color display and multi-color display are made possible by a method in which multiple types of polymeric fluorescent substances that emit different light colors are separately applied, or a method in which a color filter or fluorescent conversion filter is used. The dot-matrix device may be passively driven, or actively driven in combination with TFT and the like. These display devices can be used as display devices of a computer, television, portable terminal, cell phone, car navigation, viewfinder of a video camera, and the like.

Furthermore, the planar light-emitting device is of thin self light-emitting type, and can be favorably used as surface light sources for backlights of liquid crystal display devices, or surface light sources for illumination. In addition, when a flexible substrate is used, the device can be used as curved surface light sources or curved display devices, also.

Examples

Hereinafter, the present invention will be described more specifically on the basis of Examples and Comparative Examples. However, the present invention is not limited to the following Examples. Note that the number average molecular weight, the weight average molecular weight, and the fluorescent spectrum were measured by the following methods.

(i) Number average molecular weight and weight average molecular weight

As to the number average molecular weight and weight average molecular weight, GPC (manufactured by Shimadzu Corporation: LC-10Avp) were used to obtain the polystyrene-equivalent number average molecular weight and polystyrene-equivalent weight average molecular weight. A polymer to be measured was dissolved in tetrahydrofuran, so that the concentration was about 0.5% by weight. Then, 50 μL of the solution was injected into GPC. Tetrahydrofuran was used as the mobile phase of GPC, and allowed to flow at a flow rate of 0.6 mL/min. As a column, two TSKgel SuperHM-H (manufactured by Tosoh Corporation) and one TSKgel SuperH2000 (manufactured by Tosoh Corporation) were connected in series. As a detector, a differential refractive index detector (manufactured by Shimadzu Corporation: RID-10A) was used.

(ii) Fluorescent spectrum

The fluorescent spectrum was measured by the following method. That is to say, a 0.8%-by-weight toluene solution of a polymer compound to be measured was spin-coated on silica glass to form a thin film of this polymer compound. A fluorescent spectrum of the thin film was measured by using a spectrofluorometer (manufactured by Horiba Ltd., product name "Fluorolog") under a condition of an excitation wavelength of 350 nm. In order to obtain relative fluorescence intensity of the thin film, a fluorescent spectrum plotted against wavenumber with the intensity of the Raman spectrum of water taken as the standard was integrated within the measurement range of the spectrum. Then, a value obtained by dividing this integrated value by the absorbance determined by using a spectrophotometer (manufactured by Varian, Inc., product name "Cary5E") at the excitation wavelength was determined as the fluorescence intensity of the corresponding thin film.

(Examples 1 to 5)

A compound A represented by the following structural formula (A) was synthesized as described below.

[Chemical formula 77]

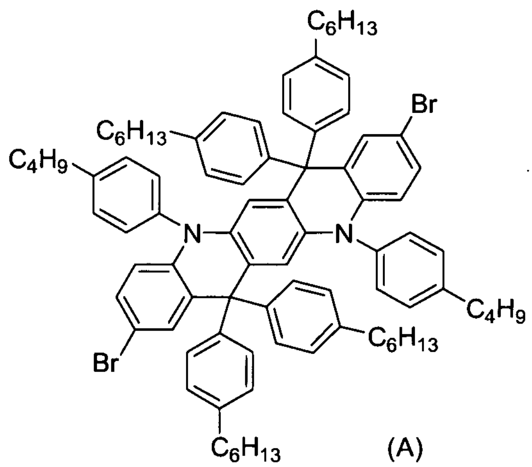

(A)

<Example 1: Synthesis of compound A-1>

First, a compound represented by the following structural formula (A-1) was synthesized.

[Chemical formula 78]

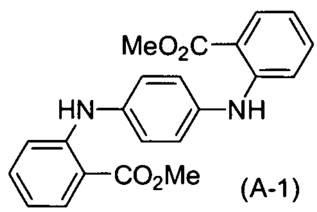

(A-1)

That is to say, 5.00 g of 1,4-dibromobenzene and 7.05 g of methyl anthranilate were charged into a 300-ml four-necked flask, followed by adding 100 ml of dehydrated toluene and nitrogen-bubbling for 1 hour. Then, 0.19 g of tris(dibenzylideneacetone)dipalladium, 0.24 g of tri(t-butyl)phosphine tetrafluoroborate, and 10.36 g of cesium carbonate were added, followed by heating at 70°C for 5 hours and subsequently refluxing for 20 hours. Hot filtration was performed through a glass filter covered with 20 g of celite, followed by washing with ethyl acetate. Next, the solvent was distilled off, followed by washing with a saturated aqueous solution of sodium hydrogencarbonate, deionized water and saturated aqueous solution of sodium chloride and drying with sodium sulfate. Then, the solvent was distilled off, and thereby 5.49 g of a crude product was obtained. Moreover, the aqueous phase was extracted with 100 ml of chloroform three times, followed by washing with water and saturated aqueous solution of sodium chloride and drying with sodium sulfate. Thereafter, the solvent was distilled off, and 4.00 g of a crude product was further obtained. The obtained crude products were combined and recrystallized from 30 ml of toluene. Thus, 6.28 g of a compound A-1 was obtained.

<Analysis data>

*LC-MS
APPI-MS, positive 377 ($[M+H]^+$, exact mass=376)
*$^1$H-NMR (300 MHz, $CDCl_3$)

δ3.91 (6H, s), 6.72 (2H,t), 7.17-7.26 (6H, m), 7.31 (2H, t), 7.96 (2H, d), 9.42 (2H, s)
*$^{13}$C-NMR (300 MHz, $CDCl_3$)
δ52.1, 111.8, 114.1, 117.1, 124.4, 131.9, 134.5, 136.9, 148.7, 169.3.

<Example 2: Synthesis of compound A-2>

Next, a compound represented by the following structural formula (A-2) was synthesized.

[Chemical formula 79]

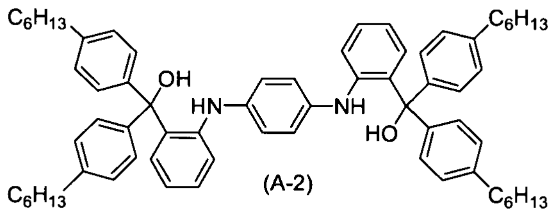

(A-2)

Specifically, 8.98 g of 1-bromo-4-n-hexylbenzene was charged into a 300-ml four-neck flask, which was purged with nitrogen. Then, it was dissolved in 90 ml of dehydrated THF, followed by cooling to -78°C. Subsequently, n-butyllithium (1.6 M hexane solution) was added dropwise in 10 minutes. Next, after the temperature was retained for 2 hours, 2.00 g of the compound A-1 dissolved in 20 ml of dehydrated THF was added dropwise. Subsequently, after the temperature was gradually increased to room temperature followed by stirring for 5 hours, 100 ml of water was added dropwise at 0°C. After that, the resultant solution was separated, and the aqueous phase was extracted with 100 ml of ethyl acetate. Moreover, after the organic phase was washed with water and saturated salt solution, the solvent was distilled off. Thereby, 8.86 g of a crude product (red-orange solid) was obtained. The obtained crude product was recrystallized from 50 ml of hexane. Thus, 4.14 g of a compound A-2 was obtained.

<Analysis data>

*LC-MS
ESI, positive 999 ($[M+K]^+$, exact mass=960)
*$^{1}$H-NMR (300 MHz, $CDCl_3$)
δ0.88 (12H, t), 1.30 (24H, m), 1.60 (8H, m), 2.60 (8H, t), 4.74 (2H, brs), 5.68 (2H, brs), 6.55-6.63 (6H, m), 6.75 (2H, m), 7.03-7.26 (20H, m)
*$^{13}$C-NMR (300 MHz, $CDCl_3$)
δ14.4, 22.9, 29.3, 31.6, 32.0, 35.8, 82.4, 118.8, 120.2, 122.0, 127.9, 128.4, 130.3, 136.1, 137.7, 142.3, 143.3, 144.0.

<Example 3: Synthesis of compound A-3>

Next, a compound represented by the following structural formula (A-3) was synthesized.

[Chemical formula 80]

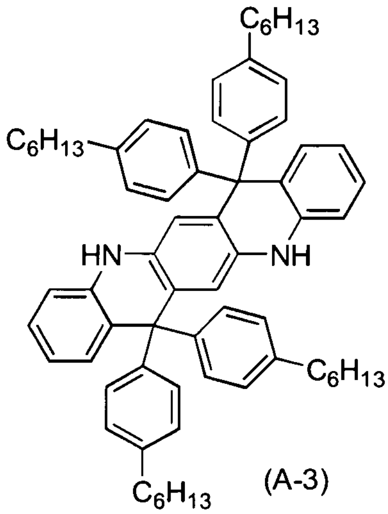

(A-3)

Specifically, 8.00 g of the compound A-2 was charged into a 300-ml Kjeldahl flask, which was purged with nitrogen. Then, the compound was dissolved in 80 ml of acetic acid, followed by cooling to 0°C. After 2.8 ml of concentrated hydrochloric acid was added dropwise, the temperature was increased to room temperature, followed by stirring for 5 hours, cooling to 0°C again, filtrating, and washing with water. Next, the resultant was dissolved in 250 ml of toluene, and was alkalized by an aqueous solution of sodium hydroxide. After washed with a saturated aqueous solution of sodium hydrogencarbonate, water and saturated salt solution, the resultant was dried with sodium sulfate. Thereafter, the solvent was distilled off, and 13.75 g of a crude product was obtained. The obtained crude product was recrystallized from 50 ml of toluene. Thus, 6.78 g of a compound A-3 was obtained.

<Analysis data>

*LC-MS
ESI, positive 963 ($[M+K]^+$, exact mass=924)
*$^1$H-NMR (300 MHz, THF-$d_8$)
δ0.90 (12H, t), 1.34 (24H, m), 1.55-1.62 (8H, m), 2.56 (8H, t), 6.37 (2H, s), 6.63-6.70 (6H, m), 6.87 (8H, d), 6.98-7.01 (10H, m), 7.87 (2H, s)
*$^{13}$C-NMR (300 MHz, THF-$d_8$)
δ13.7, 22.8, 29.5, 31.8, 32.0, 35.7, 56.2, 113.5, 115.2, 118.2, 126.8, 127.1, 127.3, 130.1, 130.4, 134.5, 140.4, 141.8, 144.6.

<Example 4: Synthesis of compound A-4>

Next, a compound represented by the following structural formula (A-4) was synthesized.

[Chemical formula 81]

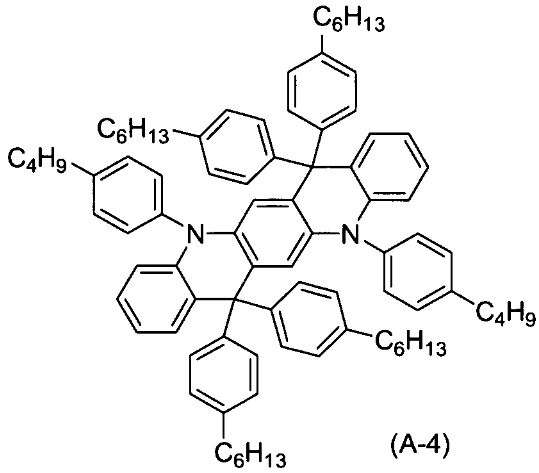

(A-4)

Specifically, 9.90 g of the compound A-3 and 4.94 g of 1-bromo-4-n-butylbenzene were charged into a 300-ml four-neck flask having been purged with nitrogen, and those were dissolved in 150 ml of dehydrated toluene. After 20 minutes of nitrogen bubbling, 0.05 g of tris(dibenzylideneacetone)dipalladium, 0.03 g of tri(t-butyl)phosphine tetrafluoroborate, and 0.30 g of sodium-t-butoxide were added, followed by refluxing for 10 hours. Then, after the temperature was cooled to 0°C, 100 ml of water was added. The resultant solution was separated, and the aqueous phase was extracted with 100 ml of toluene twice. Subsequently, the organic phase was collected, washed with water and saturated salt solution, and filtrated through a glass filter covered with 60 g of silica gel. Then, after washing with toluene, the solvent was distilled off. Thereby, 16.52 g of a crude product was obtained. To the obtained crude product, 50 ml of hexane was added for the crystallization, to which 50 ml of methanol was added, followed by filtration. The product thus obtained was recrystallized from 50 ml of hexane. Thus, 10.09 g of a compound A-4 was obtained.

<Analysis data>

*LC-MS
ESI, positive 1218 ($[M+H]^+$, exact mass=1217)
*$^1$H-NMR (300 MHz, THF-$d_8$)
δ0.62 (12H, t), 0.69 (6H, t), 1.00-1.34--17 (28H, m), 1.27-1.37 (12H, m), 2.26-2.35 (12H, m), 5.67 (2H, s), 5.93 (2H, d), 6.38-6.47 (8H, d), 6.56-6.61 (10H, m), 6.64 (8H, d), 6.79 (6H, d)
*$^{13}$C-NMR (300 MHz, THF-$d_8$)
δ15.5, 15.6, 24.6, 24.7, 31.3, 33.7, 33.9, 35.8, 37.6, 58.3, 116.0, 117.8, 121.2, 128.0, 129.1, 130.8, 138.5, 140.9, 142.2, 144.1, 145.0, 146.0.

<Example 5: Synthesis of compound A>

Next, a compound A was synthesized. Specifically, 10.09 g of the compound A-4 was charged into a 300-ml Kj eldahl flask, which was purged with nitrogen. Then, the compound was dissolved in 100 ml of chloroform, followed by cooling to 0°C. Next, a solution in which 2.87 g of NBS was dissolved in 6 ml of DMF was added dropwise in 20 minutes. Subsequently, the cold bath was removed, and after stirring for 7 hours, the temperature was cooled to 0°C. 0.5 ml of DMF in which 0.29 g of NBS was dissolved was added dropwise. Furthermore, after stirring for 2.5 hours, 100 ml of water was added dropwise. The resultant solution was separated, and the aqueous phase was extractd with chloroform. Moreover, the organic phase was washed with water and saturated salt solution, then filtrated through a glass filter covered with 50 g of silica gel, and washed with toluene. Thereafter, the solvent was distilled off. Thereby, 12.48 g of a crude product was obtained. The crude products obtained from the aqueous phase and the organic phase were recrystallized respectively from 180 ml and 200 ml of hexane. Thus, 9.55 g of a compound A was obtained.

<Analysis data>

*LC-MS
APCI, positive 1346 ($[M+H]^+$, exact mass=1345)

*$^1$H-NMR (300 MHz, THF-$d_8$)
δ0.90 (12H, t), 0.97 (6H, t), 1.30-1.45 (28H, m), 1.57-1.69 (12H, m), 2.56-2.61 (12H, m), 5.95 (2H, s), 6.16 (2H, brs), 6.70 (4H, d), 6.76 (8H, d), 7.01 (8H, d), 7.02 (2H, m), 7.05 (2H, m), 7.12 (4H, d),
*$^{13}$C-NMR (300 MHz, THF-$d_8$)
515.5, 15.6, 24.6, 24.7, 31.3, 33.6, 33.9, 35.7, 37.4, 37.6, 117.7, 129.4, 130.3, 132.0, 138.5, 142.7, 144.5.

(Example 6: Synthesis of polymer compound 1)

Under a nitrogen atmosphere, 1.01 g of the compound A, 0.40 g of 2,7-bis(1,3,2-dioxaborolane-2-yl)-9,9-di-n-octylfluore ne, 0.5 mg of dichlorobis(triphenylphosphine)palladium, 0.10 g of trioctylmethylammonium chloride (manufactured by Aldrich Co., product name "Aliquat336"), and 15 ml of toluene were mixed and heated to 90°C. To this reaction solution, 4.1 ml of an aqueous solution of 17. 5%-by-weight sodium carbonate was added dropwise, followed by refluxing for 2 hours. After the reaction, 10 mg of phenylboronic acid was added, further followed by refluxing for 4.5 hours. Then, an aqueous solution of sodium diethyldithiocarbamate was added, followed by stirring at 80°C for 3 hours. After cooling, the resultant was washed with 10 ml of water twice, with 10 ml of an aqueous solution of 3%-by-weight acetic acid twice, and with 10 ml of water twice. The resultant solution was added dropwise to 120 mL of methanol, and filtration was performed to obtain a precipitate. The precipitate was dissolved in 25 mL of toluene, followed by purification by passing through a column in which silica gel was covered with active alumina. The obtained toluene solution was added dropwise to 120 ml of methanol. After stirring, the resultant precipitate was filtrated and dried. Thus, a polymer compound 1 was obtained. The yield of the obtained polymer compound 1 was 0.89 g. Additionally, the polystyrene-equivalent number average molecular weight of the obtained polymer compound 1 was $1.0\times10^5$, and the polystyrene-equivalent weight average molecular weight was $3.0\times10^5$.

(Synthesis Example 1: Synthesis of polymer compound 2)

A polymer compound 2 which is obtained by polymerizing N,N'-di(p-bromophenyl)-N,N'-di(p-butylphenyl)-1,4-phen ylenediamine as a monomer was synthesized as follows. Note that, N,N'-di(p-bromophenyl)-N,N'-di(p-butylphenyl)-1,4-phen ylenediamine can be synthesized according to the method described in Polymer Preprints, 2001, 42(2), 587.
Specifically, under a nitrogen atmosphere, 2.73 g of N,N'-di(p-bromophenyl)-N,N'-di(p-butylphenyl)-1,4-phen ylenediamine, 2.11 g of 2,7-bis(1,3,2-dioxaborolane-2-yl)-9,9-di-n-octylfluore ne, 1.8 mg of palladium acetate, 11.3 mg of tri(o-tolyl)phosphine, 0.52 g of trioctylmethylammonium chloride (manufactured by Aldrich Co., product name "Aliquat336"), and 15 ml of toluene were mixed and heated to 90°C. To this reaction solution, 10.9 ml of an aqueous solution of 17.5%-by-weight sodium carbonate was added dropwise, followed by refluxing for 6 hours. After the reaction, 49 mg of phenylboronic acid was added, further followed by refluxing for 2 hours. Then, an aqueous solution of sodium diethyldithiocarbamate was added, followed by stirring at 80°C for 2 hours. After cooling, the resultant was washed with 50 ml of water twice, with 50 ml of an aqueous solution of 3%-by-weight acetic acid twice, and with 50 ml of water twice. The resultant solution was added dropwise to 620 ml of methanol, and filtration was performed to obtain a precipitate. The precipitate was dissolved in 120 ml of toluene, followed by purification by passing through a column in which silica gel was covered with active alumina. The obtained toluene solution was added dropwise to 620 ml of methanol. After stirring, the resultant precipitate was filtrated and dried. Thus, a polymer compound 2 was obtained. The yield of the obtained polymer compound 2 was 3.19 g. Additionally, the polystyrene-equivalent number average molecular weight of the polymer compound 2 was $4.2\times10^4$, and the polystyrene-equivalent weight average molecular weight was $1.7\times10^5$

<Evaluation of fluorescent spectrum and chromaticity of polymer compounds>

Table 1 shows the fluorescence peak wavelength and CIE chromaticity coordinate value of each of the polymer compounds obtained in Example 6 and Synthesis Example 1.

[Table 1]

| | Polymer composition | Fluorescence peak wavelength (nm) | CIE chromaticity coordinate value |
|---|---|---|---|
| Example 6 | Polymer compound 1 | 443 | (0.15, 0.10) |
| Synthesis Example 1 | Polymer compound 2 | 452 | (0.15, 0.14) |

As apparent from the result described in Table 1, it was found out that the polymer compound of the present invention (Example 6) gave shorter wavelength peak and better blue color fluorescence.

(Example 7: Synthesis of polymer compound 3) Under a nitrogen atmosphere, 2.13 g of

2,7-bis(1,3,2-dioxaborolane-2-yl)-9,9-di-n-octylfluore ne, 4.31 g of the compound A, 0.38 g of 3,7-dibromo-10-(4-butylphenyl)-10H-phenoxazine (synthesized according to the method described in Japanese Unexamined Patent Application Publication No. 2004-35221 (JP 2004-35221 A)), 2.7 mg of dichlorobis(triphenylphosphine)palladium, 0.52 g of trioctylmethylammonium chloride (manufactured by Aldrich Co. , product name "Aliquat336"), and 40 ml of toluene were mixed and heated to 90°C. To this reaction solution, 20 ml of an aqueous solution of 17.5%-by-weight sodium carbonate was added dropwise, followed by refluxing for 7 hours. After the reaction, 50 mg of phenylboronic acid was added, further followed by refluxing for 12 hours. Then, an aqueous solution of sodium diethyldithiocarbamate was added, followed by stirring at 85°C for 3 hours. After cooling, the resultant was washed with 50 ml of water twice, with 50 ml of an aqueous solution of 3%-by-weight acetic acid twice, and with 50 ml of water twice. The resultant solution was added dropwise to 600 mL of methanol, and filtration was performed to obtain a precipitate. The precipitate was dissolved in 120 mL of toluene, followed by purification by passing through a column in which silica gel was covered with active alumina. The obtained toluene solution was added dropwise to 600 ml of methanol. After stirring, the resultant precipitate was filtrated and dried. Thus, a polymer compound 3 was obtained. The yield of the obtained polymer compound 3 was 4.76 g. Additionally, the polystyrene-equivalent number average molecular weight of the obtained polymer compound 3 was $7.2\times10^4$, and the polystyrene-equivalent weight average molecular weight was $1.7\times10^5$.

(Synthesis Example 2: Synthesis of polymer compound 4)

Under a nitrogen atmosphere, 4.87 g of 2,7-bis(1,3,2-dioxaborolane-2-yl)-9,9-di(n-octyl)fluor ene, 2.96 g of 2,7-dibromo-9,9-di(n-octyl)fluorene, 1.67 g of 2,7-dibromo-9,9-di(3-methylbutyl)fluorene, 6 mg of palladium acetate, 38 mg of tri (2-methoxyphenyl) phosphine , 0.5 g of trioctylmethylammonium chloride (manufactured by Aldrich Co., product name "Aliquat336"), and 40 ml of toluene were mixed and heated to 45°C. To this reaction solution, 15 ml of an aqueous solution of 2M sodium carbonate was added dropwise, followed by heating to a reflux temperature and refluxing for 8 hours. Subsequently, 1.55 g of bromobenzene was added, followed by refluxing for 4 hours. Moreover, 1.21 g of phenylboronic acid was added, further followed by refluxing for 4 hours. Thereafter, an aqueous solution of sodium diethyldithiacarbamate was added, followed by stirring at 65°C for 4 hours. After cooling, the solution was separated and washed with 200 ml of water six times. The resultant was diluted with 5 L of toluene, and filtrated through a glass filter covered with 200 g of celite 545. The filtrate thus obtained was semi-concentrated to 150 ml. This solution was added dropwise into 1.6 L of methanol, and filtration was performed to obtain a precipitate. The precipitate was dissolved in 150 mL of toluene, and the solution was added dropwise into 1.6 L of methanol. After stirring, the resultant precipitate was filtrated and dried. Thus, a polymer compound 4 was obtained. The yield of the obtained polymer compound 4 was 5.92 g. Additionally, the polystyrene-equivalent number average molecular weight of the obtained polymer compound 4 was $8.4\times10^4$, and the polystyrene-equivalent weight average molecular weight was $2.1\times10^5$.

(Synthesis Example 3: Synthesis of polymer compound 5)

Under a nitrogen atmosphere, 10.4954 g of 2,7-bis(1,3,2-dioxaborolane-2-yl)-9,9-di-n-octylfluore ne, 9.3364 g of 3,7-dibromo-10-(4-butylphenyl)-10H-phenoxazine (synthesized according to JP 2004-35221 A), and 1.867 g of trioctylmethylammonium chloride (manufactured by Aldrich Co. , product name "Aliquat336") were dissolved in 120 g of toluene and heated to 90°C. A solution in which 4.4 mg of palladium acetate and 42.0 mg of tri(2-methylphenyl)phosphine were dissolved in 6.5 g of toluene was added. Then, 38.2 g of an aqueous solution of 17.5%-by-weight sodium carbonate was added dropwise. After refluxing for 4 hours, the molecular weight was measured. The polystyrene-equivalent number average molecular weight was $3.4\times10^4$, and the polystyrene-equivalent weight average molecular weight was $1.0\times10^5$. 0.1381 g of 2,7-bis(1,3,2-dioxaborolane-2-yl)-9,9-di-n-octylfluore ne was added, further followed by refluxing for 4 hours. As a result, the polystyrene-equivalent number average molecular weight became $5.5\times10^4$, and the polystyrene-equivalent weight average molecular weight became $2.9\times10^5$. 0.24 g of phenylboronic acid was added, further followed by refluxing for 14 hours. Then, an aqueous solution of sodium diethyldithiacarbamate was added, followed by stirring at 85°C for 3 hours. After cooling, the resultant was washed with 100 ml of water twice, with 100 ml of an aqueous solution of 3%-by-weight acetic acid twice, and with 100 ml of water twice. The resultant solution was added dropwise to 1.2 L of methanol, and filtration was performed to obtain a precipitate. The precipitate was dissolved in 250 mL of toluene, followed by purification by passing through a column in which silica gel was covered with active alumina. The obtained toluene solution was added dropwise to 1.2 L of methanol. After stirring, the resultant precipitate was filtrated and dried. Thus, a polymer compound 5 was obtained. The yield of the obtained polymer compound 5 was 12.2 g. Additionally, the polystyrene-equivalent number average molecular weight of the obtained polymer compound 5 was $5.5\times10^4$, and the polystyrene-equivalent weight average molecular weight was $2.4\times10^5$.

(Examples 8 to 10, Comparative Examples 1 and 2: Fabrication of organic electroluminescence devices 1 to 5)

<Example 8: Fabrication of organic electroluminescence device 1>

On a glass substrate with an ITO film having a thickness of 150 nm being attached thereon by a sputtering method, a film having a thickness of 65 nm was formed by spin-coating using a solution of poly(ethylenedioxythiophene) /polystyrenesulfonic acid (manufactured by Bayer AG, product name "AI4083"). The film was dried on a hot plate at 200°C for 10 minutes.

Next, a mixture of the polymer compounds [the molar ratio of the polymer compound 1 to the polymer compound 4 (polymer compound 1:polymer compound 4) was 1:1], which were dissolved in a concentration of 1.5% by weight in a xylene solvent, was spin-coated at a rotational speed of 3000 rpm to thereby form a film as a light-emitting layer. The film thickness was about 80 nm. This was dried under a nitrogen gas atmosphere at 130°C for 10 minutes. Then, as a cathode, barium was vapor-deposited with about 5 nm, and subsequently aluminium was vapor-deposited with about 80 nm. Thus, an organic electroluminescence device was fabricated. Note that, after the degree of vacuum reached $1 \times 10^{-4}$ Pa or less, the vapor-deposition of the metals was started.

<Example 9, Comparative Example 1: Fabrication of organic electroluminescence devices 2 and 3>

Organic electroluminescence devices were fabricated in the same manner as in Example 8 except that polymer compounds described in Table 2 were used as polymer compounds for forming light-emitting layers.

<Meaurement of emitted light color, driving voltage and half luminance lifetime of organic electroluminescence devices>

The emitted light color (CIE chromaticity coordinate value), driving voltage and half luminance lifetime of the organic electroluminescence devices obtained in each of Examples 8 and 9 and Comparative Example 1 were measured. The CIE chromaticity coordinate value, driving voltage, and driving current were measured using an organic EL test system (ST-P series) manufactured by Tokyo Systems Development Co., Ltd. The half luminance lifetime was measured using a PEL-100T series manufactured by EHC Co. , Ltd. Table 2 shows the obtained results. Note that, as the driving voltage, measured was a driving voltage when the organic electroluminescence device was driven in the condition that the luminance was 1000 cd/m$^2$. Moreover, as the half luminance lifetime, measured was time until the luminance becomes half of the initial luminance, with the organic electroluminescence device driven in the condition that the driving current was 3 mA. The half luminance lifetime was shown in the relative value with the value of Comparative Example 1 being set to 1.

[Table 2]

| | Light-emitting layer [composition (molar ratio) of mixture of polymer compounds] | CIE chromaticity coordinate value (x, y) | Driving voltage at 1000 cd/m$^2$ (V) | Half luminance lifetime (relative value) |
|---|---|---|---|---|
| Example 8 | Polymer compound 1:polymer compound 4=1:1 | (0.15, 0.11) | 5.8 | 5.1 |
| Example 9 | Polymer compound 3:polymer compound 4=1:1 | (0.14, 0.13) | 6.1 | 26.0 |
| Comparative Example 1 | Polymer compound 5:polymer compound 4=1:1 | (0.14, 0.17) | 6.5 | 1.0 |

<Example 10: Fabrication of organic electroluminescence device 4>

On a glass substrate with an ITO film having a thickness of 150 nm being attached thereon by a sputtering method, a film having a thickness of 65 nm was formed by spin-coating using a solution of poly(ethylenedioxythiophene) /polystyrenesulfonic acid (manufactured by Bayer AG, product name "AI4083"). The film was dried on a hot plate at

200°C for 10 minutes.

Next, the polymer compound 1 obtained in Example 6, which was 0.8% by weight in a xylene solution, was spin-coated to form a film with about 20 nm thick. Then, the film was dried under a nitrogen gas atmosphere at 180°C on a hot plate at 180°C for 60 minutes. Thereby, an interlayer was formed.

Next, a polymer compound (manufactured by Sumation Co., Ltd., product name "Lumation BP105"), which was dissolved in a concentration of 1.2% by weight in a xylene solvent, was spin-coated at a rotational speed of 2000 rpm to thereby form a film, and thus a light-emitting layer was formed. The film thickness was about 60 nm. This was dried under a nitrogen gas atmosphere at 130°C for 10 minutes. Then, as a cathode, barium was vapor-deposited with about 5 nm, and subsequently aluminium was vapor-deposited with about 80 nm. Thus, an organic electroluminescence device was fabricated. Note that, after the degree of vacuum reached $1\times10^{-4}$ Pa or less, the vapor-deposition of the metals was started.

<Comparative Example 2: Fabrication of organic electroluminescence device 5>

Organic electroluminescence devices were fabricated in the same manner as in Example 10 except that the polymer compound 2 obtained in Synthesis Example 1 was used as a polymer compound for forming an interlayer.

<Meaurement of emitted light color, driving voltage and half luminance lifetime of organic electroluminescence devices>

The emitted light color (CIE chromaticity coordinate value), driving voltage and half luminance lifetime of the organic electroluminescence devices obtained in each of Example 10 and Comparative Example 2 were measured. Table 3 shows the obtained results. Note that, as the driving voltage, measured was a driving voltage when the organic electroluminescence device was driven in the condition that the luminance was 1000 cd/$m^2$. Moreover, as the half luminance lifetime, measured was time until the luminance becomes half of the initial luminance, with each device being caused to emit light at the initial luminance of 2400 cd/$m^2$ and being driven with a constant current amount required at that time. The half luminance lifetime was shown in the relative value with the value of Comparative Example 2 being set to 1.

[Table 3]

| | Interlayer | Light-emitting layer | CIE chromaticity coordinate value (x,y) | Driving voltage at 1000 cd/$m^2$ (V) | Half luminance lifetime (relative value) |
|---|---|---|---|---|---|
| Example 10 | Polymer compound 1 | Polymer compound (Lumation BP105) | (0.14, 0.17) | 5.0 | 1.5 |
| Comparative Example 2 | Polymer compound 2 | Polymer compound (Lumation BP105) | (0.15, 0.19) | 6.5 | 1.0 |

**Industrial Applicability**

As has been described above, the present invention makes it possible to provide a compound excellent in chromaticity when used as a blue-light-emitting material for an organic electroluminescence device. Moreover, the driving voltage can be lowered when the compound of the present invention is used for the organic electroluminescence device.

**Claims**

1. A compound comprising a structure represented by the following general formula (1) (including a structure of a residue obtained by removing at least one hydrogen atom from the structure represented by the following general formula (1))

[Chemical formula 1]

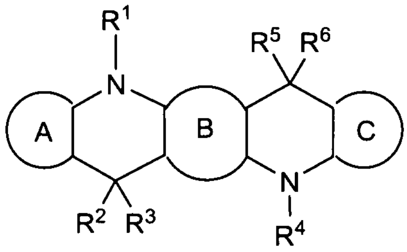

(1)

(in the formula (1), a ring A, a ring B and a ring C each independently represent any one of a monocyclic aromatic ring and fused aromatic ring; $R^1$ and $R^4$ each independently represent a monovalent group; and $R^2$, $R^3$, $R^5$ and $R^6$ each independently represent any one of a hydrogen atom, alkyl group, aryl group, arylalkyl group, alkenyl group, and alkynyl group).

**2.** The compound according to claim 1, wherein $R^2$, $R^3$, $R^5$ and $R^6$ in the general formula (1) are each independently any one of a hydrogen atom and monovalent hydrocarbon group.

**3.** The compound according to claim 1, wherein $R^2$, $R^3$, $R^5$ and $R^6$ in the general formula (1) are each independently a group represented by the following general formula (2)

[Chemical formula 2]

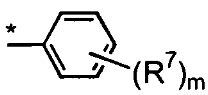

(2)

(in the formula (2), * represents a bond to a carbon atom; $R^7$ represents any one of a halogen atom, alkyl group, alkoxy group, alkylthio group, aryl group, aryloxy group, arylthio group, arylalkyl group, arylalkyloxy group, arylalkylthio group, alkenyl group, alkynyl group, disubstituted amino group, and trisubstituted silyl group; m represents an integer of 0 to 5; and when m is 2 or more, $R^7$'s may be the same or different).

**4.** The compound according to claim 1, having a polystyrene-equivalent number average molecular weight of 2000 or more.

**5.** The compound according to claim 1, comprising a repeating unit represented by the following general formula (3)

[Chemical formula 3]

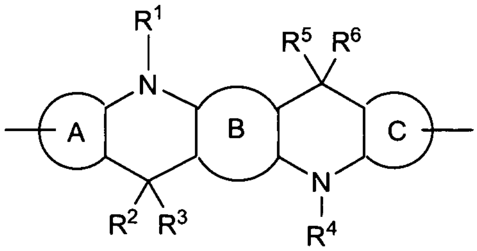

(3)

(in the formula (3), a ring A, a ring B and a ring C each independently represent any one of a monocyclic aromatic ring and fused aromatic ring; $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; and also the ring A and the ring C each have a bond thereon).

6. The compound according to claim 5, comprising a repeating unit represented by the following general formula (4)

[Chemical formula 4]

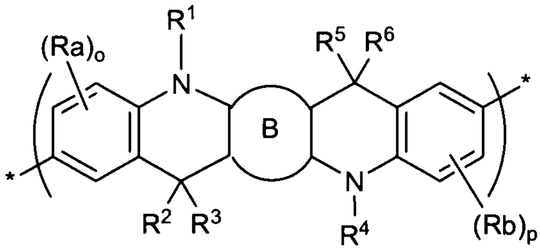

(4)

(in the formula (4), * represents a bond; a ring B represents any one of a monocyclic aromatic ring and fused aromatic ring; $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group; o and p each independently represent an integer of 0 to 3; when o is 2 or 3, a plurality of Ra' s may be the same or different; and when p is 2 or 3, a plurality of Rb's may be the same or different).

7. The compound according to claim 1, further comprising a repeating unit represented by the following general formula (5)

[Chemical formula 5]

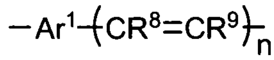

(5)

(in the formula (5) , $Ar^1$ represents any one of arylene group and divalent heterocyclic group; $R^8$ and $R^9$ each

independently represent any one of a hydrogen atom, alkyl group, aryl group, monovalent heterocyclic group, and cyano group; and n represents 0 or 1).

**8.** The compound according to claim 7, further compirising at least one repeating unit selected from the group consisting of repeating units represented respectively by the following general formulae (6-1), (6-2) and (6-3)

[Chemical formula 6]

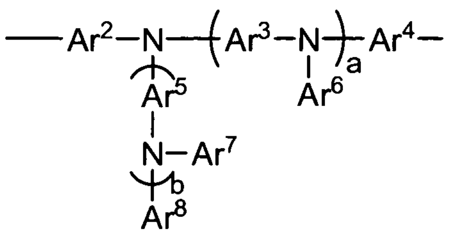

(6-1)

(in the formula (6-1), $Ar^2$, $Ar^3$, $Ar^4$ and $Ar^5$ each independently represent any one of arylene group and divalent heterocyclic group; $Ar^6$, $Ar^7$ and $Ar^8$ each independently represent any one of aryl group and monovalent heterocyclic group; and a and b each independently represent 0 or a positive integer)

[Chemical formula 7]

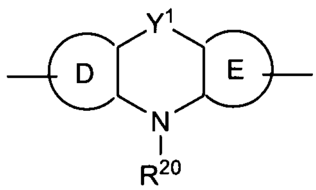

(6-2)

(in the formula (6-2), a ring D and a ring E each independently represent an aromatic ring having a bond on the ring; $Y^1$ represents any one of -O-, -S-, and -C(=O)-; and $R^{20}$ represents a monovalent group)

[Chemical formula 8]

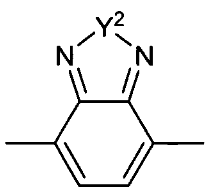

(6-3)

(in the formula (6-3), $Y^2$ represents any one of -O- and -S-; and also a 6-membered ring has two bonds thereon).

**9.** A method for producing the compound according to claim 1, comprising a step of polymerizing a compound represented by the following general formula (7) as a raw material

[Chemical formula 9]

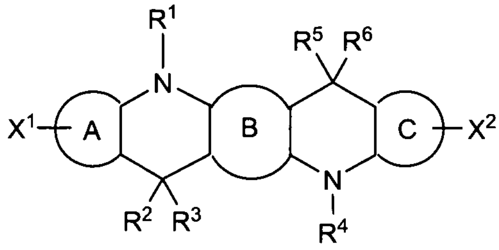

(7)

(in the formula (7), a ring A, a ring B and a ring C each independently represent any one of a monocyclic aromatic ring and fused aromatic ring; $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; and $X^1$ and $X^2$ each independently represent a substituent capable of participating in polymerization).

**10.** A compound represented by the general formula (7).

**11.** The compound according to claim 10, represented by the following general formula (8)

[Chemical formula 10]

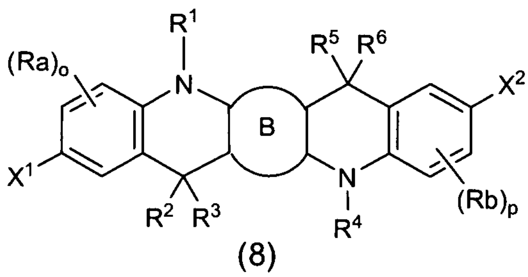

(8)

(in the formula (8), a ring B represents any one of a monocyclic aromatic ring and fused aromatic ring; $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; $X^1$ and $X^2$ each independently represent a substituent capable of participating in polymerization; Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group; o and p each independently represent an integer of 0 to 3; when o is 2 or 3, a plurality of Ra's may be the same or different; and when p is 2 or 3, a plurality of Rb's may be the same or different).

**12.** The compound according to any one of claims 10 and 11, wherein $X^1$ and $X^2$ in the general formulae (7) and (8) are each independently at least one substituent selected from the group consisting of $-B(OH)_2$, a boronic acid ester residue, halogenated magnesium, stannyl group, halogen atom, alkyl sulfonate group, aryl sulfonate group, and arylalkyl sulfonate group.

**13.** The compound according to claim 10, represented by the following general formula (9)

[Chemical formula 11]

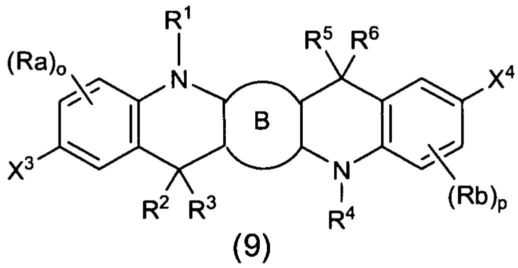

(9)

(in the formula (9), a ring B represents any one of a monocyclic aromatic ring and fused aromatic ring; $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; $X^3$ and $X^4$ each independently represent any one of a chlorine atom, bromine atom, and iodine atom; Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group; o and p each independently represent an integer of 0 to 3; when o is 2 or 3, a plurality of Ra's may be the same or different; and when p is 2 or 3, a plurality of Rb's may be the same or different).

**14.** A method for producing the compound according to claim 11, wherein the compound represented by the general formula (8) is produced by performing a functional group transformation of $X^3$ and $X^4$ in the compound represented by the general formula (9).

**15.** A method for producing the compound according to claim 13, wherein the compound represented by the general formula (9) is produced by halogenating a compound represented by the following general formula (10) in the presence of a halogenating agent

[Chemical formula 12]

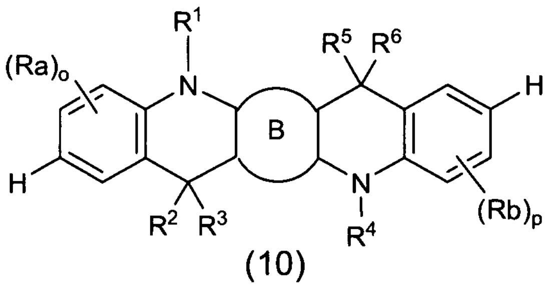

(10)

(in the formula (10), a ring B represents any one of a monocyclic aromatic ring and fused aromatic ring; $R^1$ and $R^4$ each independently represent a monovalent group; $R^2$, $R^3$, $R^5$ and $R^6$ each independently represent any one of a hydrogen atom, alkyl group, aryl group, arylalkyl group, alkenyl group, and alkynyl group; Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group; o and p each independently represent an integer of 0 to 3; when o is 2 or 3, a plurality of Ra's may be the same or different; and when p is 2 or 3, a plurality of Rb's may be the same or different).

**16.** A compound represented by the general formula (10).

**17.** A method for producing the compound according to claim 16, wherein the compound represented by the general formula (10) is produced by performing a substitution reaction on a nitrogen atom in a compound represented by the following general formula (11) in the presence of a base

[Chemical formula 13]

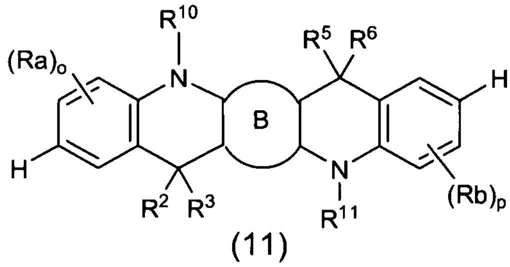

(11)

(in the formula (11), a ring B represents any one of a monocyclic aromatic ring and fused aromatic ring; $R^2$, $R^3$, $R^5$ and $R^6$ each independently represent any one of a hydrogen atom, alkyl group, aryl group, arylalkyl group, alkenyl group, and alkynyl group; $R^{10}$ and $R^{11}$ each independently represent any one of a hydrogen atom and monovalent group; at least one of $R^{10}$ and $R^{11}$ is a hydrogen atom; Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group; o and p each independently represent an integer of 0 to 3; when o is 2 or 3, a plurality of Ra's may be the same or different; and when p is 2 or 3, a plurality of Rb's may be the same or different).

**18.** A compound represented by the general formula (11).

**19.** A method for producing the compound according to any one of claims 16 and 18, wherein the compound represented by any one of the general formulae (10) and (11) is produced by cyclizing a compound represented by the following general formula (12) in the presence of an acid

[Chemical formula 14]

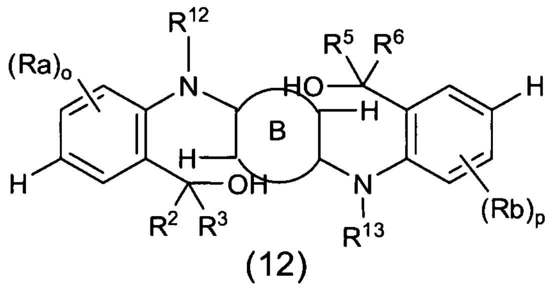

(12)

(in the formula (12), a ring B represents any one of a monocyclic aromatic ring and fused aromatic ring; $R^2$, $R^3$, $R^5$ and $R^6$ each independently represent any one of a hydrogen atom, alkyl group, aryl group, arylalkyl group, alkenyl group, and alkynyl group; $R^{12}$ and $R^{13}$ each independently represent any one of a hydrogen atom and monovalent group; Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group; o and p each independently represent an integer of 0 to 3; when o is 2 or 3, a plurality of Ra's may be the same or different; and when p is 2 or 3, a plurality of Rb's may be the same or different).

**20.** A compound represented by the following general formula (12-1) among the compounds represented by the general formula (12)

[Chemical formula 15]

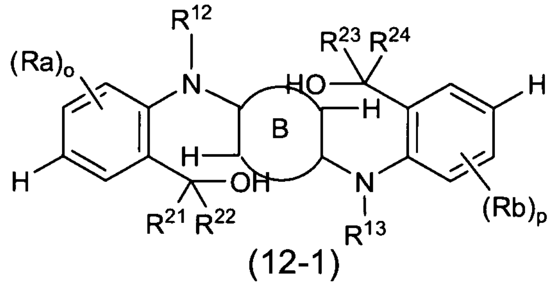

(12-1)

(in the formula (12-1), a ring B represents any one of a monocyclic aromatic ring and fused aromatic ring; $R^{12}$ and $R^{13}$ each independently represent any one of a hydrogen atom and monovalent group; $R^{21}$, $R^{22}$, $R^{23}$ and $R^{24}$ each independently represent any one of a hydrogen atom, alkyl group, aryl group, arylalkyl group, alkenyl group, and alkynyl group; at least one of $R^{21}$, $R^{22}$, $R^{23}$ and $R^{24}$ represents an aryl group; Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group; o and p each independently represent an integer of 0 to 3; when o is 2 or 3, a plurality of Ra's may be the same or different; and when p is 2 or 3, a plurality of Rb's may be the same or different).

**21.** A method for producing the compound according to claim 20, wherein the compound represented by the general formula (12-1) is produced by performing a nucleophilic reaction on a compound represented by the following general formula (13) with a compound represented by a general formula: $R^{14}$-M
(in the formula, $R^{14}$ represents any one of alkyl group, aryl group, arylalkyl group, alkenyl group, and alkynyl group; and M represents any one of lithium and halogenated magnesium)

[Chemical formula 16]

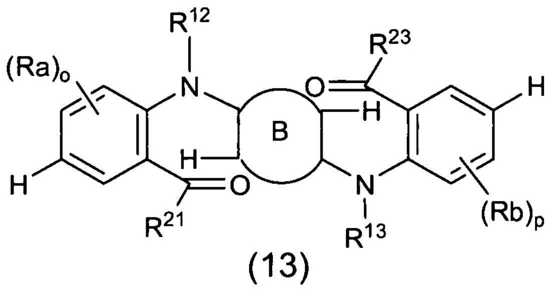

(13)

(in the formula (13), a ring B represents any one of a monocyclic aromatic ring and fused aromatic ring; $R^{21}$ and $R^{23}$ each independently represent any one of a hydrogen atom, alkyl group, aryl group, arylalkyl group, alkenyl group, and alkynyl group; $R^{12}$ and $R^{13}$ each independently represent any one of a hydrogen atom and monovalent group; Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group; o and p each independently represent an integer of 0 to 3; when o is 2 or 3, a plurality of Ra's may be the same or different; when p is 2 or 3, a plurality of Rb's may be the same or different; and at least one of $R^{21}$, $R^{23}$ and $R^{14}$ represents an aryl group).

**22.** A method for producing the compound according to claim 20, wherein the compound represented by the general formula (12-1) is produced by performing a nucleophilic reaction on a compound represented by the following general formula (14) with a compound represented by a general formula: $R^{15}$-M
(in the formula, $R^{15}$ represents any one of alkyl group, aryl group, arylalkyl group, alkenyl group, and alkynyl group; and M represents any one of lithium and halogenated magnesium)

[Chemical formula 17]

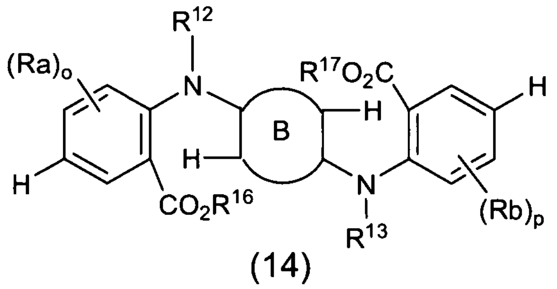

(14)

(in the formula (14), a ring B represents any one of a monocyclic aromatic ring and fused aromatic ring; $R^{12}$ and $R^{13}$ each independently represent any one of a hydrogen atom and monovalent group; $R^{16}$ and $R^{17}$ each independently represent any one of alkyl group, aryl group, and arylalkyl group; Ra and Rb each independently represent any one of alkyl group, alkyloxy group, aryl group, aryloxy group, arylalkyl group, arylalkyloxy group, disubstituted amino group, trisubstituted silyl group, acyl group, acyloxy group, substituted carboxyl group, monovalent heterocyclic group, and heteroaryloxy group; o and p each independently represent an integer of 0 to 3; when o is 2 or 3, a plurality of Ra' s may be the same or different; and when p is 2 or 3, a plurality of Rb's may be the same or different).

**23.** A compound represented by the following general formula (14-1) among the compounds represented by the general formula (14)

[Chemical formula 18]

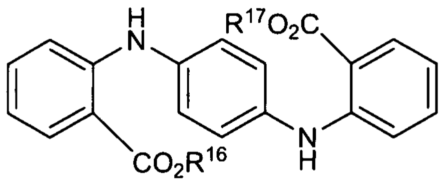

(14-1)

(in the formula (14-1), $R^{16}$ and $R^{17}$ each independently represent any one of alkyl group, aryl group, and arylalkyl group).

**24.** A method for producing the compound according to claim 23, wherein the compound represented by the general formula (14-1) is produced by performing a condensation reaction on a compound represented by the following general formula (15), a compound represented by the following general formula (16), and a compound represented by the following general formula (17) in the presence of a catalyst including at least one metal selected from the group consisting of palladium, nickel, and copper

[Chemical formula 19]

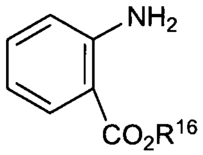

(15)

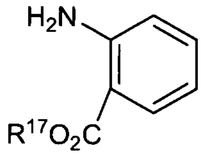

(16)

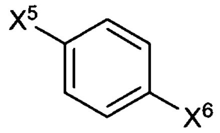

(17)

(in the formulae (15) to (17), $R^{16}$ and $R^{17}$ each independently represent any one of alkyl group, aryl group, and arylalkyl group; and $X^5$ and $X^6$ each independently represent any one of a chlorine atom, bromine atom, iodine atom, alkyl sulfonate group, aryl sulfonate group, and arylalkyl sulfonate group).

**25.** A method for producing the compound according to claim 23, wherein the compound represented by the general formula (14-1) is produced by performing a condensation reaction on a compound represented by the following general formula (18), a compound represented by the following general formula (19), and a compound represented by the following general formula (20) in the presence of a catalyst including at least one metal selected from the group consisting of palladium, nickel, and copper

[Chemical formula 20]

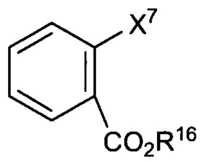

(18)

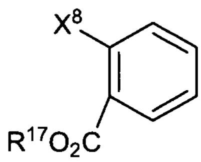

(19)

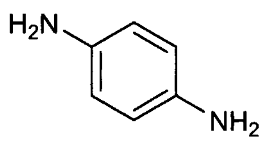

(20)

(in the formulae (18) to (20), $R^{16}$ and $R^{17}$ each independently represent any one of alkyl group, aryl group, and arylalkyl group; and $X^7$ and $X^8$ each independently represent any one of a chlorine atom, bromine atom, iodine atom, alkyl sulfonate group, aryl sulfonate group, and arylalkyl sulfonate group).

**26.** A composition comprising:

the compound according to claim 1; and
at least one material selected from the group consisting of a hole transporting material, an electron transporting material, and a light-emitting material.

**27.** An ink composition comprising any one of the compound according to claim 1 and the composition according to claim 26.

**28.** A thin film comprising any one of the compound according to claim 1 and the composition according to claim 26.

**29.** An organic transistor comprising the thin film according to claim 28.

**30.** An organic electroluminescence device comprising an organic layer between electrodes of an anode and a cathode, the organic layer including any one of the compound according to claim 1 and the composition according to claim 26.

**31.** The organic electroluminescence device according to claim 30, wherein the organic layer is a light-emitting layer.

**32.** The organic electroluminescence device according to claim 30, comprising a light-emitting layer and a hole transporting layer being located between the electrodes of the anode and the cathode, the hole transporting layer including

the compound according to claim 1.

**33.** The organic electroluminescence device according to claim 30, comprising;
a light-emitting layer and a hole transporting layer being located between the electrodes of the anode and the cathode; and
an interlayer being located between the light-emitting layer and the hole transporting layer, the interlayer including the compound according to claim 1.

**34.** A surface light source comprising the organic electroluminescence device according to claim 30.

**35.** A display device comprising the organic electroluminescence device according to claim 30.

## INTERNATIONAL SEARCH REPORT

International application No.
PCT/JP2008/063405

A. CLASSIFICATION OF SUBJECT MATTER
*C08G61/12*(2006.01)i, *C07C213/00*(2006.01)i, *C07C215/68*(2006.01)i, *C07C227/14*(2006.01)i, *C07C229/58*(2006.01)i, *C09D11/00*(2006.01)i, *C09K11/06*(2006.01)i, *H01L51/50*(2006.01)i

According to International Patent Classification (IPC) or to both national classification and IPC

B. FIELDS SEARCHED

Minimum documentation searched (classification system followed by classification symbols)
C08G61/00-61/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2007/064104 A1 (LG CHEM. LTD.), 07 June, 2007 (07.06.07), Full text & KR 10-2007-0056829 A | 1-3,10-18, 26-35 |
| A | JP 6-330031 A (Mitsubishi Kasei Corp.), 29 November, 1994 (29.11.94), Claims (Family: none) | 1-3,10-18, 26-35 |
| A | Danny Bartholome et al., Macromolecules, 2006, Vol.39, No.17, pages 5646 to 5651 | 1-3,10-18, 26-35 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 October, 2008 (09.10.08) | 21 October, 2008 (21.10.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

INTERNATIONAL SEARCH REPORT

International application No.
PCT/JP2008/063405

C (Continuation). DOCUMENTS CONSIDERED TO BE RELEVANT

| Category* | Citation of document, with indication, where appropriate, of the relevant passages | Relevant to claim No. |
|---|---|---|
| A | JP 2004-31353 A (Eastman Kodak Co.), 29 January, 2004 (29.01.04), Claims & US 2004/0001969 A1 & US 2004/0265634 A1 & EP 1375624 A1 & KR 10-2004-0002747 A & CN 1469496 A | 1-3,10-18, 26-35 |

Form PCT/ISA/210 (continuation of second sheet) (April 2007)

**INTERNATIONAL SEARCH REPORT**

International application No.
PCT/JP2008/063405

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

This international application contains the following three inventions.

1. Invention of claims 1-3, 10-18, 26-35
2. Invention of claims 4-9
3. Invention of claims 19-25

The invention of group 1 is disclosed or mentioned in WO 2007/064104 A1, and thus does not have a special technical feature.

The invention of group 2 is related to a polymer having a specific structure.

(Continued to extra sheet)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1-3, 10-18, 26-35

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

INTERNATIONAL SEARCH REPORT

International application No.
PCT/JP2008/063405

Continuation of Box No.III of continuation of first sheet(2)

The invention of group 3 is related to a method for producing the compound of the invention of group 1 or raw materials used for production of the compound.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5202355 A
- WO 9709394 A
- WO 9827136 A
- WO 9954385 A
- WO 0022027 A
- WO 0119834 A
- GB 2340304 A
- GB 2348316 A
- US 573636 A
- US 5741921 A
- US 5777070 A
- EP 0707020 A
- JP 9111233 A
- JP 10324870 A
- JP 2000080167 A
- JP 2001123156 A
- JP 2004168999 A
- JP 2007162009 A
- JP 57051781 A
- JP 63070257 A
- JP 63175860 A
- JP 2135359 A
- JP 2135361 A
- JP 2209988 A
- JP 3037992 A
- JP 3152184 A
- GB 2300196 A
- JP 2004035221 A


### Non-patent literature cited in the description


- *Polymer preprints,* 2001, vol. 42 (2), 587
- Organic Reactions. John Wiley & Sons, Inc, 1965, vol. 14, 270-490
- Organic Reactions. John Wiley & Sons, Inc, 1982, vol. 27, 345-390
- Organic Syntheses. Collective. John Wiley & Sons, Inc, 1988, vol. VI, 407-411
- Chemical Review. *Chem. Rev.,* 1995, vol. 95, 2457
- *Journal of Organometallic Chemistry,* 1999, vol. 576, 147
- *Journal of Practical Chemistry,* 1994, vol. 336, 247
- *Macromolecular Chemistry, Macromolecular Symposium,* 1987, vol. 12, 229
- *J. Org. Chem.,* 1995, vol. 60 (23), 7508
- *Angewandte Chemie, International Edition in English,* 1995, vol. 34 (12), 1348
- Development of Organic EL Device & Their Materials. CMC Publishing Co., Ltd, 2006
- Data book on work function of organic thin films. CMC Publishing Co., Ltd, 2006
- *Chemical Review,* 1989, vol. 89, 1359